# EUROPEAN PATENT SPECIFICATION

(11) **EP 3 345 995 B1**
(45) Date of publication and mention of the grant of the patent: **06.01.2021**
(21) Application number: 15903017.0
(22) Date of filing: 02.09.2015
(51) Int. Cl.: C12N 1/21, C12P 7/18, C12P 7/54, C12N 15/09

(54) **RECOMBINANT CELLS, METHOD FOR PRODUCING RECOMBINANT CELLS, AND METHOD FOR PRODUCING 1,4-BUTANEDIOL**
REKOMBINANTE ZELLEN, VERFAHREN ZUR HERSTELLUNG VON REKOMBINANTEN ZELLEN UND VERFAHREN ZUR HERSTELLUNG VON 1,4-BUTANDIOL
CELLULES RECOMBINANTES, PROCÉDÉ DE PRODUCTION DE CELLULES RECOMBINANTES ET PROCÉDÉ DE PRODUCTION DE 1,4-BUTANEDIOL

(43) Date of publication of application: 11.07.2018
(73) Proprietor: Sekisui Chemical Co., Ltd., Osaka-shi, Osaka 530-8565 (JP); Fraunhofer Gesellschaft zur Förderung der angewandten Forschung E.V., 80686 München (DE)
(72) Inventor: FURUTANI, Masahiro, Tsukuba-shi Ibaraki 300-4292 (JP); JENNEWEIN, Stefan, 53604 Bad Honnef (DE); FISCHER, Rainer, Indianapolis, Indiana 46234 (US); MCELROY, Christopher, 52064 Aachen (DE); GAIDA, Stefan, 14467 Potsdam (DE)
(74) Representative: Ter Meer Steinmeister & Partner
(86) International application number: PCT/JP2015/074979
(87) International publication number: WO 2017/037897

(56) References cited:
- WO-A1-2010/071697
- WO-A1-2014/080687
- WO-A1-2014/089436
- WO-A1-2014/112627
- WO-A1-2015/084633
- WO-A2-2014/210535
- WO-A2-2015/051298
- JP-A- 2012 511 928
- JP-A- 2012 529 267
- JP-A- 2013 504 326
- JP-A- 2013 530 724
- JP-A- 2015 156 826

## Description

### TECHNICAL FIELD

The present invention relates to a recombinant cell, a method for producing a recombinant cell, and a method for producing 1,4-butanediol.

### BACKGROUND ART

1,4-Butanediol is an organic compound that can be a raw material of butadiene which is important as a monomer of synthetic rubber, and is an important material, in particular, in the tire industry. In recent years, the technique for conversion from a production process of basic chemicals relying on petroleum to a production process from renewable resources such as plant resources has been developed and practical realization thereof is steadily progressing. Also regarding 1,4-butanediol, for example, a production technique from saccharides as a raw material by recombinant Escherichia coli is known (Patent Document 1).

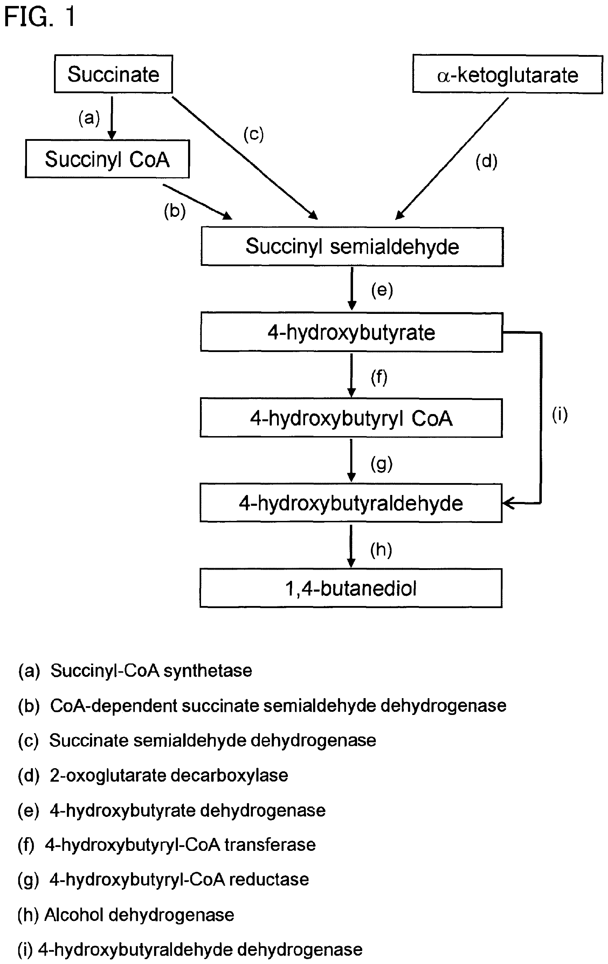

Fig. 1 shows an example of the biosynthesis pathway of 1,4-butanediol. Specifically, 1,4-butanediol can be biosynthesized, for example, from succinate or α-ketoglutarate as a starting material.

In the pathway starting from succinate, succinate is converted into 1,4-butanediol via succinyl CoA, succinyl semialdehyde, 4-hydroxybutyrate, 4-hydroxybutyryl CoA, and 4-hydroxybutyraldehyde. Enzymes that catalyze these reactions are (a) succinyl-CoA synthase, (b) CoA-dependent succinate semialdehyde dehydrogenase, (e) 4-hydroxybutyrate dehydrogenase, (f) 4-hydroxybutyryl-CoA transferase, (g) 4-hydroxybutyryl-CoA reductase, and (h) alcohol dehydrogenase, respectively (Fig. 1). Every organism has (a) succinyl-CoA synthase.

Also, there is a pathway that directly converts succinate into succinyl semialdehyde. In that case, succinate is converted into 1,4-butanediol via succinyl semialdehyde, 4-hydroxybutyrate, 4-hydroxybutyryl CoA, and 4-hydroxybutyraldehyde. The enzyme that catalyzes the reaction of converting succinate into succinyl semialdehyde is (c) succinate semialdehyde dehydrogenase (Fig. 1).

On the other hand, in the pathway starting from α-ketoglutarate, α-ketoglutarate is converted into 1,4-butanediol via succinyl semialdehyde, 4-hydroxybutyrate, 4-hydroxybutyryl CoA, and 4-hydroxybutyraldehyde. The enzyme that catalyzes the reaction of converting α-ketoglutarate into succinyl semialdehyde is (d) 2-oxoglutarate decarboxylase (Fig. 1).

Further, there is a pathway that generates 4-hydroxybutyraldehyde directly from 4-hydroxybutyrate by (i) 4-hydroxybutyraldehyde dehydrogenase (Fig. 1).

Regarding the production process from renewable resources, most of the conventional techniques including the aforementioned 1,4-butanediol production technique are production methods by microorganisms relying on organic substances, in particular, saccharides, glycerol or oil components. However, for covering the global production quantity of a large number of basic chemicals derived from petroleum, the amounts of currently available saccharides, glycerin and oil components derived from plant resources and the like will be necessarily insufficient for carbon sources of microorganisms. In other words, the production amounts of basic chemicals by microorganisms relying on saccharides or oil components is limited also in the future. These processes also have a fear of competition with foods.

Syngas (synthesis gas) is a mixed gas mainly containing carbon monoxide, carbon dioxide, and hydrogen, which is efficiently obtained by burning waste, natural gas and coal. In the field of C1 chemistry by metal catalysts starting from syngas, a process for mass production of liquid chemicals such as methanol, formic acid and formaldehyde at low costs has been developed.

Carbon monoxide, carbon dioxide and hydrogen are contained in syngas derived from waste, industrial exhaust gas, natural gas or syngas derived from coal, and are available almost permanently. However, at present, examples of producing chemicals by microorganisms from C1 carbon sources represented by syngas are very limited. Only production of ethanol, 2,3-butanediol or the like from syngas is currently under development. In particular, there is little report about utilization of a syngas utilizing substance by a recombinant. Patent Document 2 discloses a production technique of isopropanol by a recombinant of *Escherichia coli.* In this technique, a plurality of CO metabolic enzyme genes are introduced into *Escherichia coli* to impart a syngas utilizing ability, and isopropanol is produced from syngas. However, this technique does not relate to production of 1,4-butanediol. In addition, high productivity of an intended substance cannot be expected from a system using recombinant *Escherichia coli* having CO metabolism, due to reasons such as the difficultly of functionally and efficiently expressing in *Escherichia coli* a group of CO metabolic enzyme genes held originally by a syngas assimilating microorganism, and CO tolerance of *Escherichia coli* being low.

Lignocellulose is the most abundantly available non-food-based biomass. Lignocellulose can break down into cellulose, hemicellulose and lignin by conducting a physical or chemical treatment. Specifically, cellulose is expected to be a carbon source available for culturing a microorganism. However, to utilize cellulose efficiently, it is necessary to saccharify cellulose in advance by physical, chemical and enzymatic reactions, and the cost is high. Therefore, technology to cheaply saccharify cellulose and to further convert it into energies or useful materials is desired. Technology to effectively produce 1,4-butanediol from cellulose has not been found until now.

### PRIOR ART DOCUMENTS

### PATENT DOCUMENT

Patent Document 1: JP 2012-529267 A
Patent Document 2: JP 2012-509691 A

### DISCLOSURE OF INVENTION

### TECHNICAL PROBLEM

In light of the above, it is an object of the present invention to provide a series of techniques capable of producing 1,4-butanediol from cellulose or syngas which does not either rely on petroleum or compete with foods.

### SOLUTION TO PROBLEM

One aspect of the present invention for solving the aforementioned problem is a recombinant cell that is acetogenic and obligatory anaerobic, wherein the recombinant cell comprises first genes or second genes, the first genes comprise: a gene encoding CoA-dependent succinate semialdehyde dehydrogenase; a gene encoding 4-hydroxybutyrate dehydrogenase; a gene encoding 4-hydroxybutyryl-CoA transferase; a gene encoding 4-hydroxybutyryl-CoA reductase; and a gene encoding alcohol dehydrogenase, the second genes comprise: a gene encoding 2-oxoglutarate decarboxylase; a gene encoding 4-hydroxybutyrate dehydrogenase; a gene encoding 4-hydroxybutyryl-CoA transferase; a gene encoding 4-hydroxybutyryl-CoA reductase; and a gene encoding alcohol dehydrogenase, the first or second genes are expressed in the recombinant cell, and the recombinant cell produces 1,4-butanediol from at least one Cl compound selected from the group consisting of carbon monoxide and carbon dioxide.

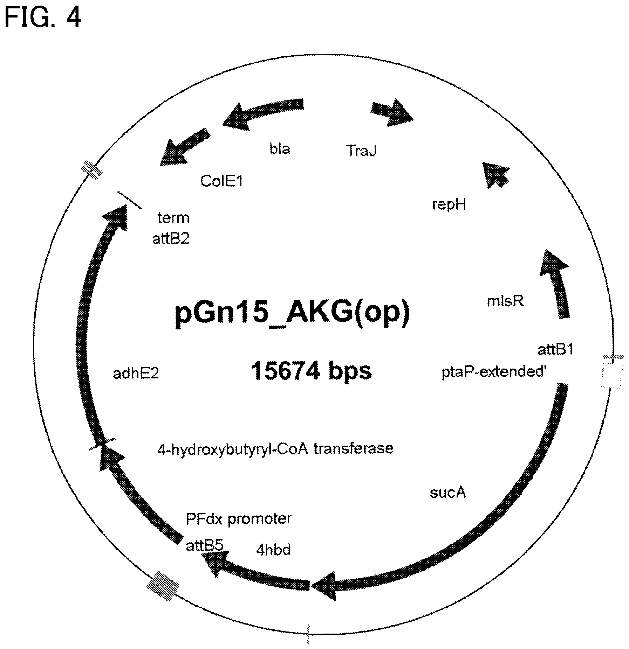

As illustrated in Fig. 1, 1,4-butanediol can be biosynthesized from succinate.

As illustrated in Fig. 1, 1,4-butanediol can be biosynthesized also from α-ketoglutarate. The gene is expressed in the recombinant cell, and the recombinant cell produces 1,4-butandiol from at least one C1 compound selected from the group consisting of carbon monoxide, carbon dioxide, formic acid, and methanol. According to the recombinant cell of the present invention, it is possible to produce 1,4-butanediol from, for example, cellulose or syngas that contains carbon monoxide and/or carbon dioxide.

Preferably, the recombinant cell has a function of synthesizing acetyl-CoA from methyltetrahydrofolate, carbon monoxide, and CoA, and the recombinant cell produces 1,4-butandiol from at least one C1 compound selected from the group consisting of carbon monoxide, carbon dioxide, formic acid, and methanol.

With such a constitution, 1,4-butanediol can be produced from the C1 compound via succinate or α-ketoglutarate based on the "function of synthesizing acetyl-CoA from methyltetrahydrofolate, carbon monoxide, and CoA", which is included in the recombinant cell.

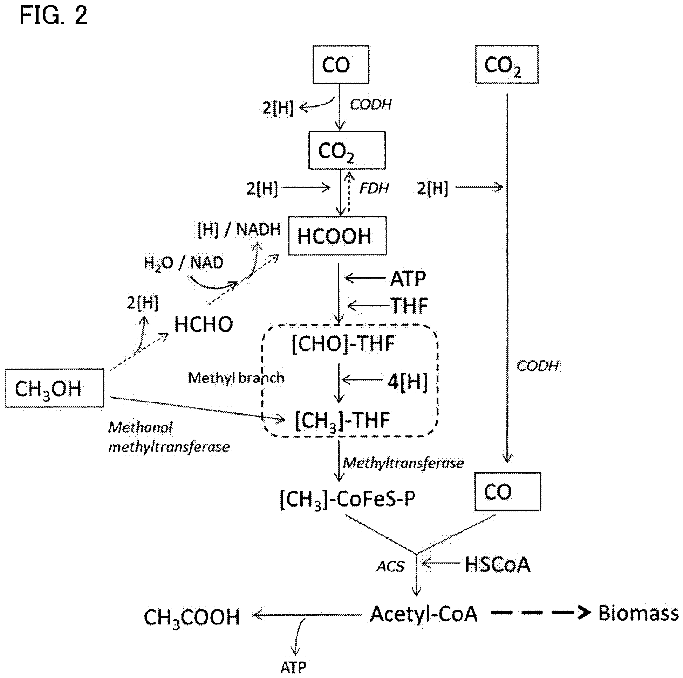

Examples of the cell having a "function of synthesizing acetyl-CoA from methyltetrahydrofolate, carbon monoxide, and CoA" include anaerobic microorganisms having an acetyl-CoA pathway (Wood-Ljungdahl pathway) and a methanol pathway shown in Fig. 2.

Preferably, the recombinant cell has carbon monoxide dehydrogenase.

Carbon monoxide dehydrogenase (EC 1.2.99.2/1.2.7.4) (CO dehydrogenase, CODH) has an activity of generating carbon dioxide and proton from carbon monoxide and water, and an activity of generating carbon monoxide and water from carbon dioxide and proton, which is a reverse reaction. The carbon monoxide dehydrogenase is one of enzymes that act in the acetyl-CoA pathway (Fig. 2).

Preferably, the recombinant cell is a *Clostridium* bacterium,

Preferably, the recombinant cell has cellulose assimilating ability, and the recombinant cell produces 1,4-butandiol from lignocellulose, cellulose, or soluble carbohydrate.

With such a constitution, 1,4-butanediol can be produced from lignocellulose, cellulose, or soluble carbohydrate via succinate or α-ketoglutarate based on the cellulose assimilating ability included in the recombinant cell, specifically on the function of synthesizing acetyl-CoA from cellulose. The pathway of synthesizing acetyl-CoA from cellulose mainly consists of Embden-Meyerhof-Parnas pathway.

Preferably, the recombinant cell produces cellulosome.

Preferably, the recombinant cell is a *Clostridium* bacterium.

Preferably, the recombinant cell is *Clostridium thermocellum, Clostridium cellulolyticum,* or *Clostridium cellulovorans.*

Preferably, at least one of the genes is derived from a bacterium.

Preferably, at least one of the genes is derived from its original host cell.

Preferably, the gene is incorporated in a genome of the host cell.

With such a configuration, the gene is retained in the recombinant cell more stably.

Preferably, the recombinant cell is tolerant to at least 400 mM 1,4-butanediol.

With such a configuration, a larger amount of 1,4-butanediol can be produced.

Preferably, ethanol generating ability of the recombinant cell is down-regulated compared to that of its original host cell.

Preferably, 2,3-butanediol generating ability of the recombinant cell is down-regulated compared to that of its original host cell.

Another aspect of the present invention is a method for producing a recombinant cell, including: a first step of providing a host cell that is acetogenic and obligatory anaerobic; and a second step of introducing a first gene set or second gene set, the first genes comprise: a gene encoding CoA-dependent succinate semialdehyde dehydrogenase; a gene encoding 4-hydroxybutyrate dehydrogenase; a gene encoding 4-hydroxybutyryl-CoA transferase; a gene encoding 4-hydroxybutyryl-CoA reductase; and a gene encoding alcohol dehydrogenase, the second genes comprise: a gene encoding 2-oxoglutarate decarboxylase; a gene encoding 4-hydroxybutyrate dehydrogenase; a gene encoding 4-hydroxybutyryl-CoA transferase; a gene encoding 4-hydroxybutyryl-CoA reductase; and a gene encoding alcohol dehydrogenase, the first or second genes are expressed in the recombinant cell, and the recombinant cell produces 1,4-butanediol from at least one Cl compound selected from the group consisting of carbon monoxide and carbon dioxide.

Preferably, the host cell has a function of synthesizing acetyl-CoA from methyltetrahydrofolate, carbon monoxide, and CoA, and the recombinant cell produces 1,4-butandiol from at least one C1 compound selected from the group consisting of carbon monoxide, carbon dioxide, formic acid, and methanol.

Preferably, the host cell has carbon monoxide dehydrogenase.

Preferably, the host cell is a *Clostridium* bacterium,

Preferably, the host cell has cellulose assimilating ability, and the recombinant cell produces 1,4-butandiol from lignocellulose, cellulose, or soluble carbohydrate.

Preferably, the host cell produces cellulosome.

Preferably, the host cell is a *Clostridium* bacterium.

Preferably, the host cell is *Clostridium thermocellum, Clostridium cellulolyticum,* or *Clostridium cellulovorans.*

Preferably, at least one of the genes is derived from a bacterium.

Preferably, at least one of the genes is derived from the host cell.

Preferably, wherein the gene introduced in the second step is incorporated in a genome.

Preferably, the recombinant cell is tolerant to at least 400 mM 1,4-butanediol.

Preferably, ethanol generating ability of the recombinant cell is down-regulated compared to that of the host cell.

Preferably, 2,3-butanediol generating ability of the recombinant cell is down-regulated compared to that of the host cell.

Another aspect of the present invention is a method for producing 1,4-butanediol, including: bringing at least one C1 compound selected from the group consisting of carbon monoxide, carbon dioxide, formic acid, and methanol into contact with the aforementioned recombinant cell or the recombinant cell produced by the aforementioned method, to cause the recombinant cell to produce 1,4-butanediol from the C1 compound.

The aspect relates to a method for producing 1,4-butanediol. In the aspect, by bringing at least one C1 compound selected from the group consisting of carbon monoxide, carbon dioxide, formic acid, and methanol into contact with the aforementioned recombinant cell, the recombinant cell is caused to produce 1,4-butanediol from the C1 compound. According to the aspect, it is possible to produce 1,4-butanediol from syngas containing carbon monoxide and/or carbon dioxide, formic acid, methanol, or the like.

Preferably, the method includes: culturing the recombinant cell by using at least one C1 compound selected from the group consisting of carbon monoxide, carbon dioxide, formic acid, and methanol as a carbon source; and obtaining 1,4-butanediol from a cultured product of the recombinant cell.

Preferably, the method includes providing a gas mainly containing carbon monoxide and hydrogen, a gas mainly containing carbon dioxide and hydrogen, or a gas mainly containing carbon monoxide, carbon dioxide and hydrogen with the recombinant cell.

The wording "provide the recombinant cell with a gas" means giving to the recombinant cell a gas as a carbon source or the like, or bringing the gas into contact with the recombinant cell.

Preferably, the recombinant cell is a *Clostridium* bacterium,

Another aspect of the present invention is a method for producing 1,4-butanediol, including: bringing lignocellulose, cellulose, or soluble carbohydrate in contact with the aforementioned recombinant cell or the recombinant cell produced by the aforementioned method, to cause the recombinant cell to produce 1,4-butanediol from the lignocellulose, cellulose, or soluble carbohydrate.

In the aspect, by bringing lignocellulose, cellulose, or soluble carbohydrate into contact with the aforementioned recombinant cell, the recombinant cell is caused to produce 1,4-butanediol. According to the aspect, it is possible to produce 1,4-butanediol from lignocellulose, cellulose, or soluble carbohydrate.

Preferably, the method includes: culturing the recombinant cell by using lignocellulose, cellulose, or soluble carbohydrate as a carbon source; and obtaining 1,4-butanediol from a cultured product of the recombinant cell.

Preferably, the recombinant cell is *Clostridium thermocellum, Clostridium cellulolyticum,* or *Clostridium cellulovorans.*

### ADVANTAGEOUS EFFECT OF INVENTION

According to the recombinant cell of the present invention, it is possible to produce 1,4-butanediol from carbon monoxide, carbon dioxide, formic acid, or methanol. For example, it is possible to produce 1,4-butanediol from syngas containing carbon monoxide and/or carbon dioxide. Furthermore, it is possible to produce 1,4-butanediol from cellulose.

According to the method for producing a recombinant cell of the present invention, it is possible to produce a recombinant cell that produces 1,4-butanediol from carbon monoxide, carbon dioxide, formic acid, or methanol. Furthermore, it is possible to produce a recombinant cell that produces 1,4-butanediol from cellulose.

Similarly, according to the method for producing 1,4-butanediol of the present invention, it is possible to produce 1,4-butanediol from carbon monoxide, carbon dioxide, formic acid, or methanol. Furthermore, it is possible to produce 1,4-butanediol from cellulose.

### BRIEF DESCRIPTION OF DRAWINGS

Fig. 1 is an explanatory view showing a metabolic pathway from succinate or α-ketoglutarate to 1,4-butanediol.
Fig. 2 is an explanatory view showing an acetyl-CoA pathway and a methanol pathway.
Fig. 3 is an explanatory view showing a structure of vector pGn15_SUC(op).
Fig. 4 is an explanatory view showing a structure of vector pGn15_AKG(op).
Fig. 5 is an explanatory view showing a structure of vector pM9_SUC(op).
Fig. 6 is an explanatory view showing a structure of vector pM9_AKG.

### DESCRIPTION OF EMBODIMENT

Hereinafter, embodiments of the present invention will be described. In the present invention, the term "gene" can be replaced by the term "nucleic acid" or "DNA".

The recombinant cell of the present invention is a recombinant cell that is acetogenic and obligatory anaerobic, wherein the recombinant cell includes a gene encoding the group of enzyme acting in a biosynthesis pathway from succinate or α-ketoglutarate to 1,4-butanediol. For example, the recombinant cell of the present invention is prepared by introducing the gene into a host cell that is acetogenic and obligatory anaerobic.

In an embodiment (first embodiment), the recombinant cell has a "function of synthesizing acetyl-CoA from methyltetrahydrofolate, carbon monoxide, and CoA". For example, the recombinant cell is prepared by introducing the gene into a host cell having the function. Then, the recombinant cell produces 1,4-butandiol from at least one C1 compound selected from the group consisting of carbon monoxide, carbon dioxide, formic acid, and methanol. The pathway of synthesizing acetyl-CoA from methyltetrahydrofolate ([CH₃]-THF), carbon monoxide (CO), and CoA is included in, for example, acetyl-CoA pathway (Wood-Ljungdahl pathway) and methanol pathway shown in Fig. 2.

As shown in Fig. 2, in the acetyl-CoA pathway, carbon dioxide (CO₂) is reduced to carbon monoxide (CO) and a source of methyl cation in two pathways, separately. Then, by using these two carbon sources as substrate, thiol group of CoA (represented as HSCoA in Fig. 2) is acetylated to synthesize a single molecule of acetyl-CoA. Enzymes that act in the acetyl-CoA pathway include enzymes such as acetyl-CoA synthase (ACS), methyltransferase, carbon monoxide dehydrogenase (CODH), and formate dehydrogenase (FDH). It should be noted that the pathway to [CH₃]-THF from formyltetrahydrofolate ([CHO]-THF) is referred to as methyl branch.

On the other hand, the methanol pathway includes a pathway of converting methanol to formaldehyde (HCHO), and then to formic acid (HCOOH), and a pathway of deriving [CH₃]-THF from methanol.

Therefore, the pathway of synthesizing acetyl-CoA from methyltetrahydrofolate ([CH₃]-THF), carbon monoxide (CO), and CoA is common for acetyl-CoA pathway and methanol pathway.

In this embodiment, it is preferable that the recombinant cell has carbon monoxide dehydrogenase (CODH). Specifically, a cell that grows by a function of generating carbon dioxide and proton from carbon monoxide and water mainly by carbon monoxide metabolism, namely, by the action of carbon monoxide dehydrogenase is preferred. Anaerobic microorganisms having acetyl-CoA pathway and methanol pathway have carbon monoxide dehydrogenase (CODH).

Representative examples of the anaerobic microorganisms include *Clostridium* bacteria, *Moorella* bacteria, or *Acetobacterium* bacteria such as *Clostridium ljungdahlii, Clostridium autoethanogenum, Clostridium carboxidivorans, Clostridium ragsdalei* (Kopke M. et al., Appl. Environ. Microbiol. 2011, 77(15), 5467-5475), *Moorella thermoacetica* (same as *Clostridium thermoaceticum*) (Pierce EG. et al., Environ. Microbiol. 2008, 10, 2550-2573), and *Acetobacterium woodii* (Dilling S. et al., Appl. Environ. Microbiol. 2007, 73(11), 3630-3636). In particular, *Clostridium* bacteria are preferred as the host cell in the present invention because their host-vector systems and culture methods have been established.

The five species of *Clostridium* bacteria, *Moorella* bacteria, or *Acetobacterium* bacteria recited above are known as representative examples of syngas utilizing microorganisms.

Besides *Clostridium* bacteria, *Moorella* bacteria and *Acetobacterium* bacteria, *Carboxydocella sporoducens* sp. Nov. (Slepova TV. et al., Inter. J. Sys. Evol. Microbiol. 2006, 56, 797-800), *Rhodopseudomonas gelatinosa* (Uffen RL, J. Bacteriol. 1983, 155(3), 956-965), *Eubacterium limosum* (Roh H. et al., J. Bacteriol. 2011, 193(1), 307-308), *Butyribacterium methylotrophicum* (Lynd, LH. et al., J. Bacteriol. 1983, 153(3), 1415-1423) and the like may be used as a host cell on which the recombinant cell of the embodiment is based.

All of proliferation and CODH activity of the bacteria as described above are oxygen sensitive. However, oxygen insensitive CODH is also known. For example, oxygen insensitive CODH exists in other bacterial species represented by *Oligotropha carboxidovorans* (Schubel, U. et al., J. Bacteriol., 1995, 2197-2203), and *Bradyrhizobium japonicum* (Lorite MJ. et al., Appl. Environ. Microbiol., 2000, 66(5), 1871-1876) (King GM et al., Appl. Environ. Microbiol. 2003, 69 (12), 7257-7265). Also in *Ralsotonia* bacteria which are aerobic hydrogen oxidizing bacteria, oxygen insensitive CODH exists (NCBI Gene ID: 4249199, 8019399).

As described above, there widely exist bacteria having CODH. The host cell for use in the present invention can be appropriately selected therefrom. For example, using a selective medium containing CO, CO/H₂ (gas mainly containing CO and H₂), or CO/CO₂/H₂ (gas mainly containing CO, CO₂ and H₂) as the sole carbon source and energy source, a bacterium having CODH that is usable as the host cell can be isolated in anaerobic, microaerobic or aerobic conditions.

In another embodiment (second embodiment), the recombinant cell has cellulose assimilating ability. Then, the recombinant cell produces 1,4-butandiol from lignocellulose, cellulose, or soluble carbohydrate.

Some obligatory anaerobic, acetogenic microbes can use cellulose as a carbon source. Specifically, *Clostridium* bacteria can effectively digest cellulose and assimilating it (Giallo J., *et al., Applied Environ. Microbiol.* 1985, 49(5), 1216). These microbes have cellulosome which is a protein complex with activity to effectively digest cellulose, and can effectively assimilate cellulose. Cellulosome has a structure in which many kinds of polysaccharide-degrading enzymes assemble on a scaffold protein. These polysaccharide-degrading enzymes cooperatively digest cellulose which has structural diversity. *Clostridium* bacteria are known as a cell producing cellulosome. Specifically, there are *Clostridium cellulolyticum, Clostridium cellulosolvens, Clostridium acetobutylicum, Clostridium cellulovorans, Clostridium clariflavum, Clostridium josui, Clostridium thermocellum, Clostridium papyrosolvens,* and the like. *Acetivibrio cellulolyticus, Bacteroides cellulosolvens, Ruminococcus albus, Ruminococcus flavefaciens,* and the like also produce cellulosome other than *Clostridium* bacteria.

Cellulose is converted into acetyl-CoA mainly in Embden-Meyerhof-Parnas pathway. Specifically, the pathway consists of: 1) a pathway in which cellulose is decomposed into a saccharide such as cellobiose being a disaccharide, 2) a pathway in which a cell takes saccharide and decomposes it into glucose to synthesize pyruvate in Embden-Meyerhof-Parnas pathway, and 3) a pathway in which acetyl-CoA is synthesized from pyruvate. Then, acetyl-CoA enters citric acid cycle to synthesize succinate and α-ketoglutarate which lead to biosynthesis of 1,4-butanediol.

A recombinant cell of the present invention has a gene encoding a group of enzymes acting in the biosynthesis pathway from succinate or α-ketoglutarate to 1,4-butanediol (hereinafter, collectively called "1,4-butanediol biosynthesis related enzyme(s)"). For example, the gene is introduced into a host cell. The enzymes represented by (a) to (i) of Fig. 1 correspond to the 1,4-butanediol biosynthesis related enzymes.

In one aspect, a recombinant cell comprises first genes or second genes, the first genes comprise: a gene encoding CoA-dependent succinate semialdehyde dehydrogenase; a gene encoding 4-hydroxybutyrate dehydrogenase; a gene encoding 4-hydroxybutyryl-CoA transferase; a gene encoding 4-hydroxybutyryl-CoA reductase; and a gene encoding alcohol dehydrogenase, the second genes comprise: a gene encoding 2-oxoglutarate decarboxylase; a gene encoding 4-hydroxybutyrate dehydrogenase; a gene encoding 4-hydroxybutyryl-CoA transferase; a gene encoding 4-hydroxybutyryl-CoA reductase; and a gene encoding alcohol dehydrogenase, the first or second genes are expressed in the recombinant cell, and the recombinant cell produces 1,4-butanediol from at least one Cl compound selected from the group consisting of carbon monoxide and carbon dioxide.

These enzymes (1,4-butanediol biosynthesis related enzymes) are not particularly limited as far as they can exert the enzymatic activity in the recombinant cell. Similarly, the genes encoding these enzymes are not particularly limited as far as they are normally transcribed and translated in the recombinant cell.

In a preferable embodiment, the 1,4-butanediol biosynthesis related enzymes are derived from bacteria. In a more preferable embodiment, the 1,4-butanediol biosynthesis related enzymes are derived from the host cell. With such a configuration, when the host cell is a bacterium, since the composition of codons used in the host cell becomes close to that of bacteria, the introduced enzyme gene can be transcribed and translated in the host cell efficiently.

Concrete examples of 1,4-butanediol biosynthesis related enzymes and genes thereof include those disclosed in Patent Document 1. For example, the following enzymes can be recited. When the host cell originally has the enzyme shown below, a gene of enzyme having higher substrate specificity, molecular activity and stability, namely having a higher value of Kcat/Km can be introduced. In this case, the enzyme gene includes a gene encoding a modified enzyme of the enzyme that is inherent in the host cell.

### (c) Succinate semialdehyde dehydrogenase (e.g., EC 1.2.1.16, EC 1.2.1.24)

Examples of genes include (each indicated by UniProtKB No.) P76149 *(E. coli);* P25526 (*E. coli*); P94428 (*Bacillus subtilis*); Q55585 (*Synechocystis* sp.); and P38067 (*Saccharomyces cerevisiae*).

### (a) Succinyl-CoA synthase (Succinyl-CoA synthetase, Succinyl-CoA ligase: e.g., EC 6.2.1.4, EC 6.2.1.5 etc.)

Examples of genes include (each indicated by UniProtKB/Swiss-Prot No.) P0AGE9 (*E. coli*); P0A836 (*E. coli*); P53598 (*Saccharomyces cerevisiae*); P53312 (*Saccharomyces cerevisiae*); P09143 (*Thermus thermophilus*); and 082662 (*Arabidopsis thaliana*). The present enzymatic activity is possessed by every organism, however, it is also effective to introduce the enzymatic activity as a foreign gene as is necessary.

### (b) CoA-dependent succinate semialdehyde dehydrogenase (e.g., EC 1.2.1.79)

Examples of genes include (each indicated by UniProtKB/Swiss-Prot No.) P38947 (*Clostridium kluyveri*); A4YGN0 (*Metallosphaera sedula*) etc.

### (e) 4-hydroxybutyrate dehydrogenase (e.g., EC 1.1.1.61)

Examples of genes include (each indicated by UniProtKB/Swiss-Prot No.) D8GUP1 (*Clostridium ljungdahlii*); C9YNR6 (*Clostridium difficile*); Q97IR6 *(Clostridium acetobutylicum*); Q8XYI7 (*Ralstonia solanacearum*); Q7MWD4 (*Porphyromonas gingivalis*) etc.

### (f) 4-hydroxybutyryl-CoA transferase (e.g., EC2.8.3.a)

Examples of genes include (each indicated by UniProtKB/Swiss-Prot No.) Q9RM86 (*Clostridium aminobutyricum*); P38942 (*Clostridium kluyveri*); Q185L2 (*Clostridium difficile*); Q3ACH6 (*Carboxydothermus hydrogenoformans*); C4Z8H6 (*Eubacterium rectale*); I8UF15 (*Porphyromonas gingivalis*) etc.

### (g) 4-hydroxybutyryl-CoA reductase (e.g., EC1.2.1.10 etc., 4-hydroxybutyryl-CoA reductase activity shows the catalytic activity of the reverse reaction of CoA-acylating aldehyde dehydrogenase)

Examples of genes include Q716S8 (*Clostridium beijerinckii*); Q7X4B7 (*Clostridium saccharoperbutylacetonicum*); A5HYN9 (*Clostridium botulinum*); P0A9Q7 (*E. coli*) (these are indicated by UniProtKB/Swiss-Prot No.); GenBank CAQ57983 *(Clostridium saccharobutylicum*); NCBI ZP_03705305 (*Clostridium methylpentosum*); NCBI ZP_08533507 (*Caldalkalibacillus thermarum*) etc.

### (h) Alcohol dehydrogenase (e.g., EC 1.1.1.1, EC 1.1.1.2 etc.)

Examples of genes include (each indicated by UniProtKB/Swiss-Prot No.) P0A4X1 (*Mycobacterium bovis*); P00331 (*Saccharomyces cerevisiae*); P00330 (*Saccharomyces cerevisiae*); Q9HIM3 (*Thermoplasma acidophilum*); B9WPR7 (*Arthrobacter* sp.); P00334 (*Drosophila melanogaster*) etc.

The enzymatic reaction step of the two stages of (g) and (h) can be catalyzed also by the action of aldehyde/alcohol dehydrogenase (adhE: EC1.1.1.1, 1.1.1.10 etc.). That is, adhE corresponds to both the above (g) and (h). Examples of adhE include D8GU53 *(Clostridium ljungdahlii*), D8GU52 (*Clostridium ljungdahlii*), Q9ANR5 *(Clostridium acetobutylicum*), P0A9Q7 (E. coli), and F7TVB7 (*Brevibacillus laterosporus*) (each indicated by UniProtKB/Swiss-Prot No.)

### (i) 4-hydroxybutyraldehyde dehydrogenase

This enzyme is able to catalyze conversion from 4-hydroxybutyrate to 4-hydroxybutyraldehyde reversibly, and belongs to aldehyde dehydrogenase according to the enzymatic classification (e.g., EC 1.2.1.3, EC 1.2.1.4, EC 1.2.1.5 etc.)

Examples of genes of aldehyde dehydrogenase showing 4-hydroxybutyraldehyde dehydrogenase activity include (each indicated by UniProtKB/Swiss-Prot No.) E4R8S4 (*Pseudomonas putida*); P23883 (*E. coli);* P12693 (*Pseudomonas putida*); P40047 (*Saccharomyces cerevisiae*); P25553 (*E. coli);* P0C6D7 (*Vibrio* sp.); P47771 (*Saccharomyces cerevisiae*); G3XYI2 (*Aspergillus niger*) etc.

### (d) 2-oxoglutarate decarboxylase (e.g., EC 4.1.1.71)

Examples of genes include (each indicated by UniProtKB/Swiss-Prot No.) A0R2B1 (*Mycobacterium smegmatis*); I0WZ48 (*Rhodococcus imtechensis*); G2EJR8 (*Corynebacterium glutamicum*); J1S9U2 (*Streptomyces auratus*); J7LQH4 (*Arthrobacter* sp.) etc.

The kind (number) of genes of "1,4-butanediol biosynthesis related enzymes" to be introduced may be at least one as far as the recombinant cell is "capable of producing 1,4-butanediol from at least one C1 compound selected from the group consisting of carbon monoxide, carbon dioxide, formic acid, and methanol". However, when the activity of each enzyme is enhanced by introducing two or more kinds of genes, improvement in productivity of 1,4-butanediol is expected. Basically, regarding a 1,4-butanediol biosynthesis related enzyme not possessed by the host cell, a gene encoding the enzyme is introduced externally. When the enzyme is possessed by the host cell, but its molecular activity is low, it is preferred to introduce a gene encoding an enzyme having higher molecular activity.

The 1,4-butanediol biosynthesis related enzymes may be naturally occurring enzymes or enzymes modified therefrom. For example, amino acid substitution variants of each enzyme, and polypeptides that are partial fragments of each enzyme and have equivalent enzyme activity are also applicable.

In a recombinant cell of the present invention, it is preferable that ethanol generating ability of the recombinant cell is down-regulated compared to its original host cell. Furthermore, in the recombinant cell of the present invention, it is preferable that 2, 3-butanediol generating ability is down-regulated comparing to its original host cell. Many of host cells described above originally produce alcohols such as ethanol and 2, 3-butanediol. Therefore, it became able to enhance 1, 4-butandiol production by down-regulating the expression of these synthesis genes. The down-regulation includes lack of targeted gene, mutation of promoter, and mutation of enzyme genes.

In the recombinant cell of the present invention, other gene may further be introduced in addition to the gene encoding 1,4-butanediol biosynthesis related enzyme or the like. In the first embodiment, examples of the genes that can be introduced include a gene encoding an enzyme acting in acetyl-CoA pathway and methanol pathway such as acetyl-CoA synthase (ACS), methyltransferase, carbon monoxide dehydrogenase (CODH), and formate dehydrogenase (FDH). By introducing these genes, enhancement of acetyl-CoA synthesis can be expected.

In the second embodiment, examples of the genes include a gene encoding proteins which are components of cellulosome.

Similar effects are also obtained by up-regulating the expression of the genes. Herein, the up-regulation means an increase in activity by increasing the copy number of the target DNA, mutation in promoter, mutation of enzyme genes, and the like.

The technique for introducing a gene into the host cell is not particularly limited, and can be appropriately selected depending on the kind of the host cell and the like. For example, a vector that can be introduced into the host cell and can allow expression of the gene incorporated therein may be used.

For example, when the host cell is a prokaryote such as a bacterium, a vector that can self duplicate or can be incorporated in chromosome in the host cell, and contains a promoter at the position allowing transcription of the inserted gene may be used. For example, it is preferred to construct in the host cell a series of structures including a promoter, a ribosome binding sequence, the above gene and a transcription termination sequence by using the vector.

In the case where the host cell is a *Clostridium* bacterium (including related species such as Moorella bacteria), a shuttle vector pIMP1 between *Clostridium* bacterium and *Escherichia coli* (Mermelstein LD et al., Bio/technology 1992, 10, 190-195) may be used. The shuttle vector is a fusion vector of pUC9 (ATCC 37252) and pIM13 isolated from *Bacillus subtilis* (Projan SJ et al., J. Bacteriol. 1987, 169 (11), 5131-5139) and is retained stably in the *Clostridium* bacterium. Other examples of a shuttle vector between *Clostridium* bacterium and *Escherichia coli* include pSOS95 (GenBank: AY187686.1).

For gene introduction into the *Clostridium* bacterium, an electroporation method is generally used. However, the introduced exogenous plasmid immediately after gene introduction is liable to be decomposed by a restriction enzyme Cac824I and the like, and is therefore very instable. For this reason, it is preferred to once amplify the vector from pIMP1 in *Escherichia coli,* for example, strain ER2275 having pAN1 (Mermelstein LD et al., Apply. Environ. Microbiol. 1993, 59(4), 1077-1081) carrying a methyl transferase gene from *Bacillus subtilis* phage Φ3T1, followed by a methylation treatment, and to recover the resultant vector from *Escherichia coli* for use in transformation by electroporation. Recently, Cac824I gene-deficient *Clostridium acetobutylicum* has been developed, and make it possible to stably carry a vector which is not subjected to a methylation treatment (Dong H. et al., PLoS ONE 2010, 5 (2), e9038). Furthermore, it should be noted that an unmethylated vector is efficiently introduced by amplifying the vector with NEB express which is an improved version of *E. coli* BL21 strain (Leang C. et al., Appl Environ Microbiol. 2013 79(4), 1102-9).

Examples of the promoter for heterologous gene expression in *Clostridium* bacteria include thl (thiolase) promoter (Perret S et al., J. Bacteriol. 2004, 186(1), 253-257), Dha (glycerol dehydratase) promoter (Raynaud C. et al., PNAS 2003, 100(9), 5010-5015), ptb (phosphotransbutyrylase) promoter (Desai RP et al., Appl. Environ. Microbiol. 1999, 65(3), 936-945), and adc (acetoacetate decarboxylase) promoter (Lee J et al., Appl. Environ. Microbiol. 2012, 78 (5), 1416-1423). However, in the present invention, sequences of promoter regions used in operons of various metabolic systems found in the host cell or the like may be used without limited to the above examples.

For introducing plural kinds of genes into the host cell by using a vector, the genes may be incorporated in one vector, or incorporated in individual vectors. When plural kinds of genes are incorporated in one vector, these genes may be expressed under a common promoter for these genes, or expressed under individual promoters. As an exemplary form of introducing plural kinds of genes, a mode of introducing a gene encoding an enzyme acting in acetyl-CoA pathway or methanol pathway in addition to "gene encoding 1,4-butanediol biosynthesis related enzymes" can be recited.

As described above, while the known vectors that can be used in the present invention have been shown, the region involved in transcription control and replication regions such as promoter and terminator can be modified depending on the purpose. The modification includes change to other natural gene sequence in each host cell or its related species, and change to an artificial gene sequence.

By increasing the expression amount of the introduced gene in the host cell, the 1,4-butanediol discharging function and the 1,4-butanediol tolerance of the host cell by combining a variation technique such as mutation or genome shuffling in addition to the modification by the gene introduction as described above, it is possible to further improve the productivity of 1,4-butanediol.

That is, in the present invention, an exogenous gene may be incorporated in a genome of the host cell or incorporated in a plasmid.

In one preferred embodiment, the recombinant cell is tolerant to at least 400 mM 1,4-butanediol. With such a configuration, it is possible to mass-produce 1,4-butanediol. For example, the recombinant cell having such characteristics can be obtained by subjecting host cells to an appropriate variation treatment to select a host cell having the intended characteristics, and using the host cell.

One aspect of the method for producing 1,4-butanediol of the present invention includes bringing at least one C1 compound selected from the group consisting of carbon monoxide, carbon dioxide, formic acid, and methanol into contact with the aforementioned recombinant cell to cause the recombinant cell to produce 1,4-butanediol from the C1 compound.

Preferably, the method includes: culturing the recombinant cell by using at least one C1 compound selected from the group consisting of carbon monoxide, carbon dioxide, formic acid, and methanol as a carbon source; and obtaining 1,4-butanediol from a cultured product of the recombinant cell. The C1 compound used as a carbon source may be used singly or in combination of two or more. The C1 compound is preferably used as a main carbon source, and more preferably as the sole carbon source.

Also, it is preferred to provide hydrogen (H2) concurrently as an energy source.

The method for culturing the recombinant cell of the present invention is not particularly limited, and may be appropriately conducted depending on the kind of the host cell and the like. When the recombinant cell is a *Clostridium,* bacterium (obligatory anaerobic, strictly anaerobic), it is cultured, for example, in a nutrient condition including inorganic salts required for growth, and syngas. Preferably, it is cultured under a pressurized condition at about 0.2 to 0.3MPa (absolute pressure). Further, for improving initial proliferation and attained cell density, small amounts of organic substances such as vitamin, yeast extract, corn steep liquor, and Bacto Tryptone may be added.

When the recombinant cell is aerobic or facultative anaerobic, for example, it may be cultured in a liquid medium under aeration and stirring.

It is also possible to produce 1,4-butanediol without culturing the recombinant cell. That is, regardless of whether cell division (cell proliferation) is involved or not, it is possible to produce 1,4-butanediol by bringing the C1 compound into contact with the recombinant cell. For example, it is possible to continuously produce 1,4-butanediol by continuously supplying a fixed recombinant cell with the C1 compound. Also in the present aspect, regarding these C1 compounds, only one C1 compound may be used, or a combination of two or more C1 compounds may be used. Also, it is preferred to provide hydrogen (H₂) concurrently as an energy source.

In a preferred embodiment, the recombinant cell is provided with a gas mainly containing carbon monoxide and hydrogen (CO/H2), a gas mainly containing carbon dioxide and hydrogen (CO₂/H₂), or a gas mainly containing carbon monoxide, carbon dioxide and hydrogen (CO/CO₂/H₂). In other words, 1,4-butanediol is produced from carbon monoxide or carbon dioxide in such a gas by culturing the recombinant cell by using the gas as a carbon source, or by bringing the gas into contact with the recombinant cell. Also in this case, hydrogen is used as an energy source.

In another preferred embodiment, the recombinant cell is provided with methanol/carbon monoxide, methanol/carbon dioxide, or methanol/carbon monoxide/carbon dioxide. In other words, 1,4-butanediol is produced from methanol, carbon monoxide, or carbon dioxide by culturing the recombinant cell by using methanol and the gases as a carbon source, or by bringing methanol and the gases into contact with the recombinant cell. Modes for providing the recombinant cell with methanol and the gases include providing methanol and the gases concurrently.

Furthermore, 1,4-butanediol may be produced from formic acid and/or methanol by providing the recombinant cell with formic acid and/or methanol. In other words, 1,4-butanediol can also be produced from formic acid and/or methanol by culturing the recombinant cell using, as a carbon source, formic acid or methanol solely or in addition to carbon monoxide and/or carbon dioxide, or by bringing formic acid and/or methanol into contact with the recombinant cell.

A method for producing 1,4-butanediol of the present invention includes bringing lignocellulose, cellulose, or soluble carbohydrate in contact with the aforementioned recombinant cell to cause the recombinant cell to produce 1,4-butanediol from the lignocellulose, cellulose, or soluble carbohydrate. Preferably, the method includes culturing the recombinant cell by using lignocellulose, cellulose, or soluble carbohydrate as a carbon source, and obtaining 1,4-butanediol from a cultured product of the recombinant cell.

Cellulose substrates include, for example cellulose biomass. Examples of the cellulose biomass includes rice straw, vegetable scraps, bagasse, wood pulp, wood chips, sawdust, used papers, rayon, cotton, hemp, thread, corrugated cardboards, and crystalline cellulose. While it is possible to make these cellulose substrates directly contact with the recombinant cell of this invention, it is preferable that these substrates are treated physically, chemically, and/or enzymatically, in advance of contact with the cells, or in the same time as culture of the cell. As a physical treatment, for example, ball mill, vibrating mill, pressurized hot water, cooking blasting, and the like are available. As a chemically treatment, acids, alkalis, and the like are available. As an enzymatic treatment, hemicellulases, cellulases, liquids of these enzymes, and the like are available.

The produced 1,4-butanediol is accumulated in the cell or released outside the cell. For example, purified 1,4-butanediol can be obtained by using the recombinant cell prepared from a host cell of a *Clostridium* bacterium or a *Moorella* bacterium, and recovering 1,4-butanediol released outside the cell, followed by isolation and purification.

Herein, combinations of carbon monoxide, carbon dioxide, formic acid, and methanol that can be provided to the recombinant cell in the case where the host cell has the acetyl-CoA pathway and the methanol pathway (Fig. 2) are described.

In acetyl-CoA synthesis by the acetyl-CoA pathway, a synthesis process of acetyl-CoA from CoA, methyltetrahydrofolate ([CH₃]-THF), and CO by the actions of methyltransferase, Corrinoid iron-sulfur protein (CoFeS-P), acetyl-CoA synthase (ACS), and CODH is essential (Ragsdale SW et al., B.B.R.C. 2008, 1784(12), 1873-1898).

On the other hand, it is known that adding formic acid and/or methanol besides CO and/or CO₂ as a carbon source in culturing of *Butyribacterium methylotrophicum* increases the content of tetrahydrofolate (THF) in CO metabolism, namely, methyl branch in the acetyl-CoA pathway, and activities of CODH, formate dehydrogenase (FDH) and hydrogenase required in CO metabolism (Kerby R. et al., J. Bacteriol. 1987, 169(12), 5605-5609). Also in *Eubacterium. limosum* or the like, it is demonstrated that high proliferation is achieved by using CO₂ and methanol as a carbon source in an anaerobic condition (Genthner BRS. et al., Appl. Environ. Microbiol., 1987, 53(3), 471-476).

The influence of methanol on syngas utilizing microorganisms, and the results of genome analysis of *Moorella thermoacetica* (*Clostridium thermoaceticum*), *Clostridium ljungdahlii* and the like (Pierce E. et al., Environ. Microbiol. 2008, 10(10), 2550-2573; Durre P. et al., PNAS 2010, 107(29), 13087-13092) can give an explanation for involvement of the methanol pathway as shown in Fig. 2 as a donor of a methyl group in the acetyl-CoA pathway (Wood-Ljungdahl pathway) in these microorganism species.

Actually in some *Clostridium* bacteria, the forward activity of formate dehydrogenase (FDH) (EC 1.2.1.2/1.2.1.43: Formate + NAD(P)⁺ ⇔ CO₂ + NAD(P)H) (formation of CO₂ from formate) is confirmed (Liu CL et al., J. Bacteriol. 1984, 159(1), 375-380; Keamy JJ et al., J. Bacteriol. 1972, 109(1), 152-161). Therefore, in these strains, a reaction in the direction of generating CO₂ from methanol (CH₃OH) and/or formic acid (HCOOH) can partly proceed when CO₂ and/or CO is deficient (Fig. 2). This can also be explained by the phenomenon that formate dehydrogenase activity and CODH activity increase by addition of CH₃OH (Kerby R et al., J. Bateriol. 1987, 169(12), 5605-5609) as described above. In other words, these can be proliferated with formic acid (HCOOH) or methanol (CH₃OH) as the sole carbon source.

Even if the host cell strain inherently lacks the forward activity of formate dehydrogenase, it may be provided with the forward activity by gene modification such as introduction of mutation, introduction of foreign gene, or genome shuffling.

For these reasons, it is possible to produce 1,4-butanediol using the following gas or liquid when the host cell has the acetyl-CoA pathway and the methanol pathway.

CO

CO₂

CO/H₂

CO₂/H₂

CO/CO₂/H₂

CO/HCOOH

CO₂/HCOOH

CO/CH₃OH

CO₂/CH₃OH

CO/H₂/HCOOH

CO₂/H₂/HCOOH

CO/H₂/CH₃OH

CO₂/H₂/CH₃OH

CO/CO₂/H₂/HCOOH

CO/CO₂/H₂/CH₃OH

CH₃OH/H₂

HCOOH/H₂

CH₃OH

HCOOH

When the recombinant cell of the present invention is cultured exclusively for cell proliferation, rather than for production of 1,4-butanediol, it is not necessary to use carbon monoxide and/or carbon dioxide as a carbon source. For example, the recombinant cell may be cultured using other carbon sources such as saccharides or glycerin which is easily assimilated by the recombinant cell of the present invention

In the following, the present invention will be described more specifically by way of examples. However, the present invention is not limited to these examples.

### EXAMPLE 1

### 1,4-BDO production by syngas utilizing bacterium C. ljungdahlii (succinate pathway)

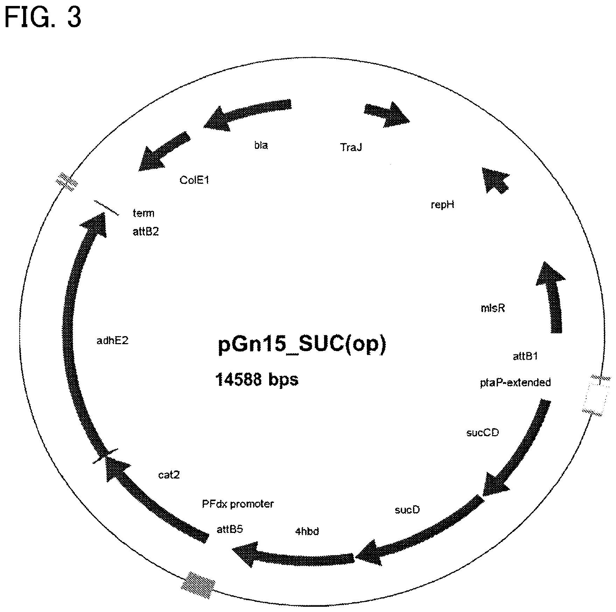

*Clostridium*/*E. coli* shuttle vector pGn15_SUC(op) (SEQ ID NO: 1, Fig 3.) was introduced into *C*. *ljungdahlii* by electroporation (performed with a BioRad Micropulser (BioRad, Hercules, CA, USA) at 0.63 kV) and the transformants were collected on YTF agar plates (YTF-medium containing per liter, 16 g tryptone, 10 g yeast extract, 4 g NaCl, 2 mM L-cysteine supplemented with 1% Xylose plus 1.5% agar).

For the electroporation the following protocol was used. All work was always performed under strictly anaerobic conditions and, except the centrifugation steps and the storage of the cryocultures, inside the Whitley Anaerobic Workstation A55 (Don Whitley Scientific Limited, United Kingdom) with the gas composition 10% CO₂, 5% H₂ and 85% N₂. *Clostridium ljungdahlii* strain (DSM No.: 13528) was ordered from DSMZ (Deutsche Sammlung von Mikroorganismen und Zellkulturen) and received as freeze dried culture. The general recommendation by DSMZ for handling strictly anaerobic microorganisms and for strain specific cultivation conditions was employed. The pellet was dissolved in 20 mL YTF-medium (1 L contains 16 g tryptone, 10 g yeast extract, 4 g NaCl, 2 mM L-cysteine supplemented with 1% Xylose) in a 50 mL centrifuge tube. The culture was taken at OD660 of around 2.0 to inoculate 20 mL fresh medium with 1% of the preculture. This was repeated three times and then 100 mL medium were inoculated the same way in a 200 mL flask for preparation of competent cells. For the destabilization of the cell wall 40 mL DL-threonine were added. The cells were harvested at OD600 of around 0.3 and then washed three times with a volume of 100 mL of ice cold washing buffer (270 mM sucrose, 1 mM MgCl₂, 20 mM BisTris, pH 5.9). The supernatant was discarded and after each washing step the samples were centrifuged at 4 °C and 4000 g for 5 min in 50 mL centrifuge tubes to spin down the cells. Finally, the washed cell pellet was dissolved in 2 mL washing buffer supplemented with 10% DMSO and 25 µL aliquots of cells were prepared in 1.5 mL tubes. The competent cells were then stored up to three months at -80°C.

The methylation of DNA is generally an important issue for the transformation of clostridia. For *Clostridium ljungdahlii E. coli* B-strains with Dcm⁻ Dam⁺ methylation have descripted as more efficient for plasmid preparation with subsequent electroporation (Leang *et al.* 2013). For the transformation of the 1,4 BDO cluster plasmid DNA was isolated from the K-strain NEB10B (New England Biolabs, Frankfurt am Main, Germany), because the plasmid stability is considered to be higher especially when pathways are driven by constitutive promoters. The successful transformation of *Clostridium ljungdahlii* with DNA from the *E. coli* Dcm⁺ Dam⁻ strain NEB10B suggests that a specific methylation is not a fundamental step for the DNA transfer into *Clostridium ljungdahlii.* For the DNA transfer the cells were thawed on ice and were mixed with 3 µg of the plasmid

The DNA/cell mix was then transferred to a prechilled 0.1 mm gap cuvette and electroporation was performed with a BioRad Micropulser (BioRad, Hercules, CA, USA) at 0.63 kV. Next the cells were regenerated at 37°C in 20 mL YTF-medium for at least 12 hours. Then the antibiotic clarithromycin was added to the culture (dissolved in 70% ethanol, final concentration 5 µg/ mL) and the cells were further incubated for 6 hours. Then different amounts of the cell culture were mixed with YTF-agar medium. Around 30 mL of YTF-agar medium (containing 5 µg/mL clarithromycin) at 37°C were mixed with 1 µL, 10 µL, 100 µL, 1 mL, 2 mL, 3 mL and the rest of the 20 mL regeneration culture in petri dishes (9 cm diameter) and incubated for at least 5 days at 37°C. The obtained colonies were picked from plates, where 1-100 µL culture had been added, and only when they showed the morphology of *Clostridium ljungdahlii.* Fifteen milliliter centrifuge tubes were used for further cultivation and precultures. Clarithromycin was always added to final concentration of 5 µg/mL.

The vector pGn15_SUC(op) includes 1,4-BDO synthesizing gene cluster (Fig. 3) encoding *sucCD* from *Escherichia coli, sucD* from *Clostridium kluyveri, 4hbd* from *Porphyromonas gingivalis, cat2* from *Porphyromonas gingivalis,* and *adhE2* from *Clostridium acetobutylicum.* The collected transformants were named CLJU_pGn15_SUC(op).

The transformant named CLJU_pGn15_SUC(op) described above and *C*. *ljungdahlii* wildtype were anaerobically cultured in the YTF medium (containing per liter, 16 g tryptone, 10 g yeast extract, 4 g NaCl, 2 mM L-cysteine supplemented with 1% Xylose or Fructose). The culture was conducted in the 50 mL of sealed bottle with 200 mL of the above medium at 37°C and the culture bottles were shaken. When the OD600 of the culture medium was reached 2.0, the medium was centrifuged and the supernatant was collected. The supernatant was filtered by 0.22 µm filter and analyzed by GCMS (QP2010S GC-MS system (Shimadzu), InterCap FFAP (GL Sciences), mobile phase: 100% Methanol, carrier gas: helium, split ratio: 1:10, injection temperature: 220°C).

**GC-MS Analysis Parameters for Operated Columns:**

| | |
|---|---|
| Column: | InterCap FFAP (GL Sciences) |
| Dimension: | 30 m x 0.25 mm, 0.25 µm |
| Mobile phase: | 100 % Methanol |
| Carrier gas: | Helium |
| Split ratio: | 1:10 |
| Total flow: | 14.4 mL/min |
| Injection Temperature: | 220°C |

**GC-MS Analysis using InterCap FFAP for detecting 1,4-BDO**

| Rate [°C/min] | Final Temperature [°C] | Hold Time [min] |
|---|---|---|
| - | 60 | 1.00 |
| 5.0 | 70 | 0.00 |
| 35.0 | 220 | 2.00 |

As a result, while the CLJU_pGn15_SUC(op) introduced with 1,4-BDO synthesizing gene cluster produced significant amount of 1,4-BDO with the yield of 20-40 µM, the wildtype did not produce 1,4-BDO. It is shown that the syngas utilizing bacterium *C*. *ljungdahlii* introduced with 1,4-BDO synthesizing gene can produce 1,4-BDO.

### EXAMPLE 2

### 1,4-BDO production by syngas utilizing bacterium Clostridium ljungdahlii (a-ketoglutarate pathway)

*Clostridium*/*E. coli* shuttle vector pGn15_AKG (op) (SEQ ID NO: 2, Fig. 4) was introduced into *C*. *ljungdahlii* by electroporation (performed with a BioRad Micropulser (BioRad, Hercules, CA, USA) at 0.63 kV) and the transformants were collected on YTF agar plates (YTF-medium containing per liter, 16 g tryptone, 10 g yeast extract, 4 g NaCl, 2 mM L-cysteine supplemented with 1% Xylose plus 1.5% agar). The vector pGn15_AKG(op) includes 1,4-BDO synthesizing gene cluster (Fig. 4) encoding α-ketoglutarate decarboxylase (*sucA* from *Mycobacterium bovis*), 4-hydroxybutyrate dehydrogenase (*4hbd* from *Porphyromonas gingivalis*), 4-hydroxybutyryl CoA transferase (*cat2* from *Porphyromonas gingivalis*), and bifunctional 4-hydroxybutyryl CoA reductase and alcohol dehydrogenase (*adhE2* from *Clostridium acetobutylicum*). The collected transformants were named CLJU_pGn15_AKG(op).

The transformant CLJU_pGn15AKG(op) described above and *C*. *ljungdahlii* wildtype were anaerobically cultured in the ATCC medium (containing 1 g/L Yeast extract, 2 mM cysteine, pH 6.0, adapted from Koepke Dissertation, Ulm 2009). The culture was conducted in the 50 mL of sealed bottle with 200 mL of the above medium at 37°C and the culture bottles were shaken. The head-space of the bottle was filled up with a mix Gas of CO/CO₂/H₂ = (1:1:1) at the pressure of 1 MPa (absolute pressure), and then sealed with butyl gum caps and aluminum caps.

When OD600 of the culture medium was reached 2.0, the medium was centrifuged and the supernatant was collected. The supernatant was filtered by 0.22 µm filter and analyzed by GCMS (QP2010S GC-MS system (Shimadzu), InterCap FFAP (GL Sciences), mobile phase: 100% Methanol, carrier gas: helium, split ratio: 1:10, injection temperature: 220°C).

This transformant was also used as a Pre-culture for syngas fermentation. Pre-cultures were prepared in flasks containing 50 mL ATCC media (pH 6.0). Either Syngas (1/1/1; 1 bar overpressure) or Syngas (1/1/1; 1 bar overpressure) plus 1% fructose was used as carbon source in precultures. Selection was maintained with clarithromycin (10 µg/mL). Pre-cultures required at least 48 hours at 37°C to grow before being inoculated into the fermenter. Pre-cultures growing on syngas required more than 72 hours before inoculation. For the fermentation 2 L of ATCC media containing an additional 50 mL of 0.8 M BIS-TRIS pH 6.0 and 1.5 mL antifoam (Struktol J673) was prepared. For each fermentation-unit 500 mL of 1M NaOH as base was prepared and autoclaved separately, furthermore 200 µL of resazurin (20 mg/mL) were added before autoclaving the media. After autoclaving, the trace elements, vitamin-solution, L-Cystein and antibiotics were added and the pH control was activated so that pH 6.0 could be maintained. To deoxygenate the vessels a continuous nitrogen gasflow (0,8 L/min; 10-15 min) was used. Afterwards the media was saturated with syngas (1/1/1; 0.25 L/min) for 15 min. Samples were taken two times a day for analysis of the supernatant via GC/MS (6 mL) and every 24 h for targeted proteomics analysis via LC/MS-MS (50-100 mL depending on the OD600 which was also measured twice daily and recorded. After 5-6 days the fermentation broth was harvested and 1,4-BDO was extracted using solvent extraction. The method employed was via a dichloromethane and salt extraction which enabled the reduction and concentration of the 2 L fermentation broth down to less than 100 mL. Five hundred (500) µL of the extract was pipetted into a GCMS analysis tube, and then 1 µL thereof was injected.

As a result, while the CLJU_pGn15_AKG(op) introduced with 1,4-BDO synthesizing gene cluster produced significant amount of 1,4-BDO with the yield of 40-170 µM, the wildtype did not produce 1,4-BDO. It is shown that the syngas-utilizing bacterium C. *ljungdahlii* introduced with 1,4-BDO synthesizing gene can produce 1,4-BDO.

### EXAMPLE 3

### 1,4-BDO production by cellulolytic bacterium Clostridium cellulolyticum (succinate-pathway)

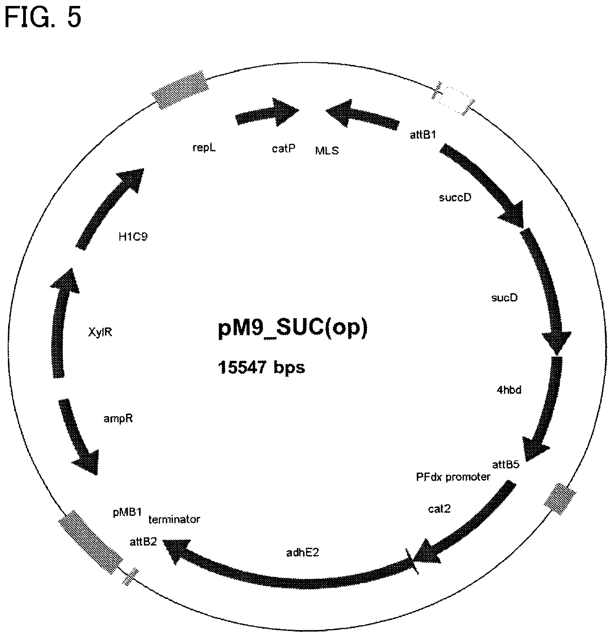

*Clostridium*/*E. coli* shuttle vector, pM9_SUC(op) (SEQ ID NO: 3, Fig. 5) was introduced into *C*. *cellulolyticum* by electroporation (at 1.6 kV) and transformants were collected on CM3 agar plates (1.3 g L⁻¹ (NH₄)₂ SO₄, 1.5 g L⁻¹ KH₂PO₄, 2.9 g L⁻¹ K₂HPO₄ x 3 H₂O, 0.2 g L⁻¹ MgCl₂ x 6 H₂O, 75.0 mg L⁻¹ CaCl₂ x 2 H₂O, 1.25 mg L⁻¹ FeSO₄ x 7 H₂O, 1.0 mL L⁻¹ trace element solution SL-10, 1.0 mg L⁻¹ Resazurin, 2.0 g L⁻¹ yeast extract, 2.5 g L⁻¹ Na₂CO₃, 0.5 g L⁻¹ L-cysteine-HCL x H₂O, 6.0 g L⁻¹ D-cellobiose and 15.0 g L⁻¹ agar). The vector pM9_SUC(op) includes 1,4-BDO synthesizing gene cluster (Fig. 5) encoding succinyl CoA synthetase (*sucCD* from *Escherichia coli*), CoA dependent succinate semialdedhyde dehydrogenase (*sucD* from *Clostridium kluyveri*), *4hbd* from *Porphyromonas gingivalis, cat2* from *Porphyromonas gingivalis,* and *adhE2* from *Clostridium acetobutylicum.* The collected transformants were named CCE_pM9_SUC(op).

The CCE_pM9_SUC(op) and CCE wildtype described above were anaerobically cultured in the CM3 medium (CM3 agar without agar). The culture was conducted in the 50 mL of sealed bottle with 200 mL of the above medium at 34°C. The culture bottles were sealed with butyl gum caps and aluminum caps.

When OD600 of the culture medium was reached 2.0, the medium was centrifuged and the supernatant was collected. The supernatant was filtered by 0.22 µm filter and analyzed by GCMS (QP2010S GC-MS system (Shimadzu), InterCap FFAP (GL Sciences), mobile phase: 100% Methanol, carrier gas: helium, split ratio: 1:10, injection temperature: 220°C).

As a result, while the pM9_SUC(op) introduced with 1,4-BDO synthesizing gene cluster produced significant amount of 1,4-BDO with the yield of 60-120 µM, the wildtype did not produce 1,4-BDO. It is shown that the cellulolytic bacterium *C*. *cellulolyticum* introduced with 1,4-BDO synthesizing gene can produce 1,4-BDO.

### EXAMPLE 4

### 1,4-BDO production by cellulolytic bacterium Clostridium cellulolyticum (α-ketoglutarate-pathway)

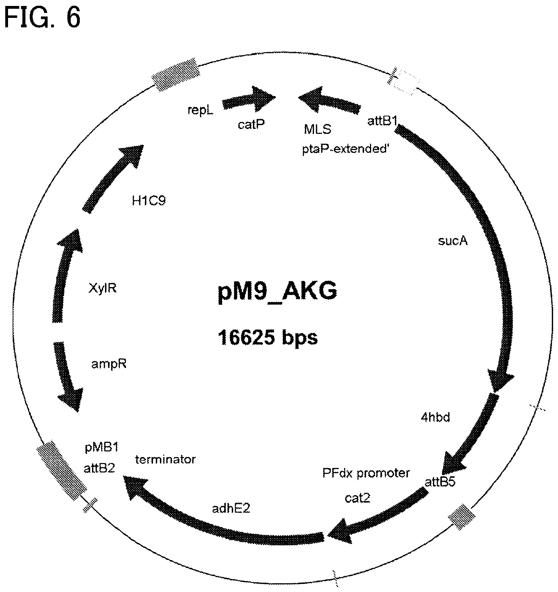

*Clostridium*/*E. coli* shuttle vector, pM9_AKG (SEQ ID NO: 4, Fig. 6) was introduced into C. *cellulolyticum* by electroporation (at 1.6 kV) and the transformants were collected on CM3 agar plates (containing 1.3 g L⁻¹ (NH₄)₂ SO₄, 1.5 g L⁻¹ KH₂PO₄, 2.9 g L⁻¹ K₂HPO₄ x 3 H₂O, 0.2 g L⁻¹ MgCl₂ x 6 H₂O, 75.0 mg L⁻¹ CaCl₂ x 2 H₂O, 1.25 mg L⁻¹ FeSO₄ x 7 H₂O, 1.0 mL L⁻¹ trace element solution SL-10, 1.0 mg L⁻¹ Resazurin, 2.0 g L⁻¹ yeast extract, 2.5 g L⁻¹ Na₂CO₃, 0.5 g L⁻¹ L-cysteine-HCL x H₂O, 6.0 g L⁻¹ D-cellobiose and 15.0 g L⁻¹ agar). The vector pM9_AKG includes 1,4-BDO synthesizing gene cluster (Fig. 6) encoding *sucA* from *Mycobacterium bovis, 4hbd* from *Porphyromonas gingivalis, cat2* from *Porphyromonas gingivalis,* and *adhE2* from *Clostridium acetobutylicum.* The collected transformants were named CCE_pM9_AKG.

The CCE_pM9_AKG and CCE wildtype described above were anaerobically cultured in the CM3 medium (CM3 agar without agar). The culture was conducted in the 50 mL of sealed bottle with 200 mL of the above medium at 34°C. The culture bottles were sealed with butyl gum caps and aluminum caps.

When the OD600 of the culture medium was reached 2.0, the medium was centrifuged and the supernatant was collected. The supernatant was filtered by 0.22 µm filter and analyzed by GCMS (QP2010S GC-MS system (Shimadzu), InterCap FFAP (GL Sciences), mobile phase: 100% Methanol, carrier gas: helium, split ratio: 1:10, injection temperature: 220°C).

As a result, while the pM9_AKG introduced with 1,4-BDO synthesizing gene cluster produced significant amount of 1,4-BDO with the yield of 60 µM, the wildtype did not produce 1,4-BDO. It is shown that the cellulolytic bacterium *C*. *cellulolyticum* introduced with 1,4-BDO synthesizing gene can produce 1,4-BDO.

### EXAMPLE 5

### 1,4-butanediol production from syngas by Clostridium ljungdahlii (succinate-pathway)

### (1) Construction of a vector that expresses 1,4-butanediol biosynthesis related enzymes

The artificial gene of SEQ ID NO: 5 (6774bp), which is a gene cluster encoding 1,4-butanediol biosynthesis related enzymes, was synthesized. The gene cluster includes sucD (CoA-deopendent succinate semialdehyde dehydrogenase; Gene ID: 5394466), 4Hb (4-hydroxybutyrate dehydrogenase; Gene ID: 2552693), abfT (4-hydroxybutyryl-CoA reductase, EMBL_CAB60036.2), adhE2 (alcohol dehydrogenase; EMBL_AAK09379.1) genes, and T7 terminator, and further includes a BamHI recognition site and a XhoI recognition site at a terminal and the other terminal of the cluster, respectively.

The artificial DNA of SEQ ID NO: 6 (120 bp) was introduced into the BamHI/NheI site of *Clostridium*/*E. coli* shuttle vector pSOS95 (Genbank: AY187686.1) to give pSOS-MS. A gene fragment obtained by digesting the artificial gene of SEQ ID NO: 5 with BglII/XhoI was introduced into the BamHI/NheI site ofpSOS-MS to give pSOS-BDO. NBL express was used for the cloning procedure. Like pSOS95, pSOS-BDO also functions as a *Clostridium*/*E. coli* shuttle vector.

### (2) Preparation of a recombinant cell that produces 1,4-butanediol

The pSOS-BDO prepared in item (1) was introduced by electroporation into *Clostridium ljungdahlii* (DSM13528/ATCC55383) to give a transformant. As a control, the pSOS-MS was also introduced into *Clostridium ljungdahlii* (DSM13528/ATCC55383) to give another transformant. The electroporation was conducted by the methods recommended in Appl. Environ. Microbiol. 2013, 79 (4): 1102-9.

### (3) 1,4-BDO production by the transformant

The two kinds of transformants obtained in item (2) were anaerobically cultured at 37°C. Fructose-free ATCC 1754 PETC medium containing 4 µg/mL clarithromycin was used for the culture. The culture was conducted in a 125 mL sealed bottle with 45 mL of the medium and a mixed gas of CO/CO₂/H₂=33/33/34 at the pressure of 0.25 MPa (absolute pressure). The bottle was sealed with butyl gum caps and aluminum caps, and then shaken. When the OD600 of the culture medium was reached 0.9, the medium was centrifuged and the supernatant was collected. The supernatant was analyzed by LCMS.

As a result, while the transformant introduced with pSOS-BDO produced 1,4-BDO, the transformant introduced with pSOS-MS did not produce 1,4-BDO. It is shown that recombinant *Clostridium ljungdahlii* introduced with 1,4-BDO synthesizing gene cluster can produce 1,4-BDO from syngas.

## Claims

1. A recombinant cell that is acetogenic and obligatory anaerobic, wherein
the recombinant cell is a *Clostridium* bacterium
the recombinant cell comprises first genes or second genes,
the first genes comprise:
a gene encoding CoA-dependent succinate semialdehyde dehydrogenase;
a gene encoding 4-hydroxybutyrate dehydrogenase;
a gene encoding 4-hydroxybutyryl-CoA transferase;
a gene encoding 4-hydroxybutyryl-CoA reductase; and
a gene encoding alcohol dehydrogenase,
the second genes comprise:
a gene encoding 2-oxoglutarate decarboxylase;
a gene encoding 4-hydroxybutyrate dehydrogenase;
a gene encoding 4-hydroxybutyryl-CoA transferase;
a gene encoding 4-hydroxybutyryl-CoA reductase; and
a gene encoding alcohol dehydrogenase,
the first or second genes are expressed in the recombinant cell, and
the recombinant cell produces 1,4-butanediol from at least one C1 compound selected from the group consisting of carbon monoxide and carbon dioxide.

2. The recombinant cell according to claim 1, wherein the recombinant cell has carbon monoxide dehydrogenase.

3. The recombinant cell according to claim 1 or 2, wherein the recombinant cell is *Clostridium ljungdahlii.*

4. A recombinant cell that is acetogenic and obligatory anaerobic, wherein
the recombinant cell is a *Clostridium* bacterium,
the recombinant cell comprises first genes or second genes,
the first genes comprise:
a gene encoding CoA-dependent succinate semialdehyde dehydrogenase;
a gene encoding 4-hydroxybutyrate dehydrogenase;
a gene encoding 4-hydroxybutyryl-CoA transferase;
a gene encoding 4-hydroxybutyryl-CoA reductase; and
a gene encoding alcohol dehydrogenase,
the second genes comprise:
a gene encoding 2-oxoglutarate decarboxylase;
a gene encoding 4-hydroxybutyrate dehydrogenase;
a gene encoding 4-hydroxybutyryl-CoA transferase;
a gene encoding 4-hydroxybutyryl-CoA reductase; and
a gene encoding alcohol dehydrogenase,
the first or second genes are expressed in the recombinant cell,
the recombinant cell has cellulose assimilating ability,
the recombinant cell produces cellulosome, and
the recombinant cell produces 1,4-butanediol from lignocellulose, cellulose, or soluble carbohydrate.

5. The recombinant cell according to claim 4, wherein the recombinant cell is *Clostridium thermocellum, Clostridium cellulolyticum,* or *Clostridium cellulovorans.*

6. The recombinant cell according to any one of claims 1 to 5, wherein ethanol generating ability of the recombinant cell is down-regulated compared to that of its original host cell.

7. The recombinant cell according to any one of claims 1 to 6, wherein 2,3-butanediol generating ability of the recombinant cell is down-regulated compared to that of its original host cell.

8. A method for producing a recombinant cell, comprising:
a first step of providing a host cell that is acetogenic and obligatory anaerobic, wherein the host cell is a *Clostridium* bacterium; and
a second step of introducing first genes or second genes into the host cell, wherein
the first genes comprise:
a gene encoding CoA-dependent succinate semialdehyde dehydrogenase;
a gene encoding 4-hydroxybutyrate dehydrogenase;
a gene encoding 4-hydroxybutyryl-CoA transferase;
a gene encoding 4-hydroxybutyryl-CoA reductase; and
a gene encoding alcohol dehydrogenase,
the second genes comprise:
a gene encoding 2-oxoglutarate decarboxylase;
a gene encoding 4-hydroxybutyrate dehydrogenase;
a gene encoding 4-hydroxybutyryl-CoA transferase;
a gene encoding 4-hydroxybutyryl-CoA reductase; and
a gene encoding alcohol dehydrogenase,
wherein the first or second genes are expressed in the recombinant cell, and the recombinant cell produces 1,4-butanediol from at least one C1 compound selected from the group consisting of carbon monoxide and carbon dioxide.

9. A method for producing a recombinant cell, comprising:
a first step of providing a host cell that is acetogenic and obligatory anaerobic, wherein the host cell is a *Clostridium* bacterium; and
a second step of introducing first genes or second genes into the host cell, wherein
the first genes comprise:
a gene encoding CoA-dependent succinate semialdehyde dehydrogenase;
a gene encoding 4-hydroxybutyrate dehydrogenase;
a gene encoding 4-hydroxybutyryl-CoA transferase;
a gene encoding 4-hydroxybutyryl-CoA reductase; and
a gene encoding alcohol dehydrogenase,
the second genes comprise:
a gene encoding 2-oxoglutarate decarboxylase;
a gene encoding 4-hydroxybutyrate dehydrogenase;
a gene encoding 4-hydroxybutyryl-CoA transferase;
a gene encoding 4-hydroxybutyryl-CoA reductase; and
a gene encoding alcohol dehydrogenase,
wherein the first or second genes are expressed in the recombinant cell,
wherein the recombinant cell has cellulose assimilating ability,
wherein the recombinant cell produces cellulosome, and
wherein the recombinant cell produces 1,4-butanediol from lignocellulose, cellulose, or soluble carbohydrate.

10. A method for producing 1,4-butanediol, comprising:
bringing at least one C1 compound selected from the group consisting of carbon monoxide and carbon dioxide into contact with the recombinant cell according to any one of claims 1 to 3 or the recombinant cell produced by the method according to claim 8, to cause the recombinant cell to produce 1,4-butanediol from the C1 compound.

11. The method according to claim 10, comprising:
culturing the recombinant cell by using at least one C1 compound selected from the group consisting of carbon monoxide and carbon dioxide as a carbon source; and
obtaining 1,4-butanediol from a cultured product of the recombinant cell.

12. The method according to claim 10 or 11, comprising:
providing a gas mainly containing carbon monoxide and hydrogen, a gas mainly containing carbon dioxide and hydrogen, or a gas mainly containing carbon monoxide, carbon dioxide and hydrogen with the recombinant cell.

13. A method for producing 1,4-butanediol, comprising:
bringing lignocellulose, cellulose, or soluble carbohydrate in contact with the recombinant cell according to claim 4 or 5 or the recombinant cell produced by the method according to claim 9, to cause the recombinant cell to produce 1,4-butanediol from the lignocellulose, cellulose, or soluble carbohydrate.

14. The method according to claim 13, comprising:
culturing the recombinant cell by using lignocellulose, cellulose, or soluble carbohydrate as a carbon source; and
obtaining 1,4-butanediol from a cultured product of the recombinant cell.

15. The method according to claim 13 or 14, wherein the recombinant cell is *Clostridium thermocellum, Clostridium cellulolyticum,* or *Clostridium cellulovorans.*

## Patentansprüche

1. Rekombinante Zelle, die acetogen und obligat anaerob ist, wobei die rekombinante Zelle ein *Clostridium-*Bakterium ist, wobei die rekombinante Zelle erste Gene oder zweite Gene umfasst, wobei die ersten Gene Folgendes umfassen:
ein Gen, das für CoA-abhängige Succinat-Semialdehyd-Dehydrogenase codiert;
ein Gen, das für 4-Hydroxybutyrat-Dehydrogenase codiert;
ein Gen, das für 4-Hydroxybutyryl-CoA-Transferase codiert;
ein Gen, das für 4-Hydroxybutyryl-CoA-Reduktase codiert; und
ein Gen, das für Alkoholdehydrogenase codiert, wobei die zweiten Gene Folgendes umfassen:
ein Gen, das für 2-Oxoglutarat-Decarboxylase codiert;
ein Gen, das für 4-Hydroxybutyrat-Dehydrogenase codiert;
ein Gen, das für 4-Hydroxybutyryl-CoA-Transferase codiert;
ein Gen, das für 4-Hydroxybutyryl-CoA-Reduktase codiert; und
ein Gen, das für Alkoholdehydrogenase codiert, wobei das erste oder zweite Gen in der rekombinanten Zelle exprimiert wird und die rekombinante Zelle 1,4-Butandiol aus mindestens einer C1-Verbindung, ausgewählt aus der Gruppe bestehend aus Kohlenmonoxid und Kohlendioxid, produziert.

2. Rekombinante Zelle nach Anspruch 1, wobei die rekombinante Zelle Kohlenmonoxid-Dehydrogenase aufweist.

3. Rekombinante Zelle nach Anspruch 1 oder 2, wobei die rekombinante Zelle *Clostridium ljungdahlii* ist.

4. Rekombinante Zelle, die acetogen und obligat anaerob ist, wobei die rekombinante Zelle ein *Clostridium-*Bakterium ist, wobei die rekombinante Zelle erste Gene oder zweite Gene umfasst, wobei die ersten Gene Folgendes umfassen:
ein Gen, das für CoA-abhängige Succinat-Semialdehyd-Dehydrogenase codiert;
ein Gen, das für 4-Hydroxybutyrat-Dehydrogenase codiert;
ein Gen, das für 4-Hydroxybutyryl-CoA-Transferase codiert;
ein Gen, das für 4-Hydroxybutyryl-CoA-Reduktase codiert; und
ein Gen, das für Alkoholdehydrogenase codiert, wobei die zweiten Gene Folgendes umfassen:
ein Gen, das für 2-Oxoglutarat-Decarboxylase codiert;
ein Gen, das für 4-Hydroxybutyrat-Dehydrogenase codiert;
ein Gen, das für 4-Hydroxybutyryl-CoA-Transferase codiert;
ein Gen, das für 4-Hydroxybutyryl-CoA-Reduktase codiert; und
ein Gen, das für Alkoholdehydrogenase codiert, wobei das erste oder zweite Gen in der rekombinanten Zelle exprimiert wird, wobei die rekombinante Zelle die Fähigkeit hat, Zellulose zu assimilieren, wobei die rekombinante Zelle Zellulosom produziert und die rekombinante Zelle 1,4-Butandiol aus Lignozellulose, Zellulose oder löslichem Kohlenhydrat produziert.

5. Rekombinante Zelle nach Anspruch 4, wobei die rekombinante Zelle *Clostridium thermocellum, Clostridium cellulolyticum* oder *Clostridium cellulovorans* ist.

6. Rekombinante Zelle nach einem der Ansprüche 1 bis 5, wobei die ethanolerzeugende Fähigkeit der rekombinanten Zelle im Vergleich zu der ihrer ursprünglichen Wirtszelle herunterreguliert ist.

7. Rekombinante Zelle nach einem der Ansprüche 1 bis 6, wobei die Fähigkeit der rekombinanten Zelle zur Erzeugung von 2,3-Butandiol im Vergleich zu der ihrer ursprünglichen Wirtszelle herunterreguliert ist.

8. Verfahren zum Produzieren einer rekombinanten Zelle, Folgendes umfassend:
einen ersten Schritt des Bereitstellens einer Wirtszelle, die acetogen und obligat anaerob ist, wobei die Wirtszelle ein *Clostridium-*Bakterium ist; und
einen zweiten Schritt des Einbringens erster Gene oder zweiter Gene in die Wirtszelle, wobei die ersten Gene Folgendes umfassen:
ein Gen, das für CoA-abhängige Succinat-Semialdehyd-Dehydrogenase codiert;
ein Gen, das für 4-Hydroxybutyrat-Dehydrogenase codiert;
ein Gen, das für 4-Hydroxybutyryl-CoA-Transferase codiert;
ein Gen, das für 4-Hydroxybutyryl-CoA-Reduktase codiert; und
ein Gen, das für Alkoholdehydrogenase codiert, wobei die zweiten Gene Folgendes umfassen:
ein Gen, das für 2-Oxoglutarat-Decarboxylase codiert;
ein Gen, das für 4-Hydroxybutyrat-Dehydrogenase codiert;
ein Gen, das für 4-Hydroxybutyryl-CoA-Transferase codiert;
ein Gen, das für 4-Hydroxybutyryl-CoA-Reduktase codiert; und
ein Gen, das für Alkoholdehydrogenase codiert, wobei das erste oder zweite Gen in der rekombinanten Zelle exprimiert wird und die rekombinante Zelle 1,4-Butandiol aus mindestens einer C1-Verbindung, ausgewählt aus der Gruppe bestehend aus Kohlenmonoxid und Kohlendioxid, produziert.

9. Verfahren zum Produzieren einer rekombinanten Zelle, Folgendes umfassend:
einen ersten Schritt des Bereitstellens einer Wirtszelle, die acetogen und obligat anaerob ist, wobei die Wirtszelle ein *Clostridium-*Bakterium ist; und
einen zweiten Schritt des Einbringens erster Gene oder zweiter Gene in die Wirtszelle, wobei die ersten Gene Folgendes umfassen:
ein Gen, das für CoA-abhängige Succinat-Semialdehyd-Dehydrogenase codiert;
ein Gen, das für 4-Hydroxybutyrat-Dehydrogenase codiert;
ein Gen, das für 4-Hydroxybutyryl-CoA-Transferase codiert;
ein Gen, das für 4-Hydroxybutyryl-CoA-Reduktase codiert; und
ein Gen, das für Alkoholdehydrogenase codiert, wobei die zweiten Gene Folgendes umfassen:
ein Gen, das für 2-Oxoglutarat-Decarboxylase codiert;
ein Gen, das für 4-Hydroxybutyrat-Dehydrogenase codiert;
ein Gen, das für 4-Hydroxybutyryl-CoA-Transferase codiert;
ein Gen, das für 4-Hydroxybutyryl-CoA-Reduktase codiert; und
ein Gen, das für Alkoholdehydrogenase codiert, wobei das erste oder zweite Gen in der rekombinanten Zelle exprimiert wird, wobei die rekombinante Zelle die Fähigkeit hat, Zellulose zu assimilieren, wobei die rekombinante Zelle Zellulosom produziert und wobei die rekombinante Zelle 1,4-Butandiol aus Lignozellulose, Zellulose oder löslichem Kohlenhydrat produziert.

10. Verfahren zum Produzieren von 1,4-Butandiol, Folgendes umfassend:
Inkontaktbringen mindestens einer C1-Verbindung, ausgewählt aus der Gruppe bestehend aus Kohlenmonoxid und Kohlendioxid, mit der rekombinanten Zelle nach einem der Ansprüche 1 bis 3 oder der nach dem Verfahren nach Anspruch 8 produzierten rekombinanten Zelle, um die rekombinante Zelle zu veranlassen, aus der C1-Verbindung 1,4-Butandiol zu produzieren.

11. Verfahren nach Anspruch 10, Folgendes umfassend:
Kultivieren der rekombinanten Zelle unter Verwendung mindestens einer C1-Verbindung, ausgewählt aus der Gruppe bestehend aus Kohlenmonoxid und Kohlendioxid, als Kohlenstoffquelle; und
Gewinnen von 1,4-Butandiol aus einem kultivierten Produkt der rekombinanten Zelle.

12. Verfahren nach Anspruch 10 oder 11, Folgendes umfassend:
Bereitstellen eines Gases, das hauptsächlich Kohlenmonoxid und Wasserstoff enthält, eines Gases, das hauptsächlich Kohlendioxid und Wasserstoff enthält, oder eines Gases, das hauptsächlich Kohlenmonoxid, Kohlendioxid und Wasserstoff enthält, mit der rekombinanten Zelle.

13. Verfahren zum Produzieren von 1,4-Butandiol, Folgendes umfassend:
Inkontaktbringen von Lignocellulose, Cellulose oder löslichem Kohlenhydrat mit der rekombinanten Zelle nach Anspruch 4 oder 5 oder der rekombinanten Zelle, die durch das Verfahren nach Anspruch 9 produziert wurde, um die rekombinante Zelle zu veranlassen, aus der Lignocellulose, Cellulose oder dem löslichen Kohlenhydrat 1,4-Butandiol zu produzieren.

14. Verfahren nach Anspruch 13, Folgendes umfassend:
Kultivieren der rekombinanten Zelle unter Verwendung von Lignozellulose, Zellulose oder löslichen Kohlenhydraten als Kohlenstoffquelle; und
Gewinnen von 1,4-Butandiol aus einem kultivierten Produkt der rekombinanten Zelle.

15. Verfahren nach Anspruch 13 oder 14, wobei die rekombinante Zelle *Clostridium thermocellum, Clostridium cellulolyticum* oder *Clostridium cellulovorans* ist.

## Revendications

1. Cellule recombinante acétogénique et obligatoirement anaérobique, la cellule recombinante étant une bactérie *Clostridium,* la cellule recombinante comprenant des premiers gènes ou des seconds gènes, les premiers gènes comprenant :
un gène codant pour la semi-aldéhyde succinique déshydrogénase dépendante de CoA;
un gène codant pour la 4-hydroxybutyrate déshydrogénase ;
un gène codant pour la 4-hydroxybutyryl-CoA transférase ;
un gène codant pour la 4-hydroxybutyryl-CoA réductase ; et
un gène codant pour l'alcool déshydrogénase, les seconds gènes comprenant :
un gène codant pour la 2-oxoglutarate décarboxylase ;
un gène codant pour la 4-hydroxybutyrate déshydrogénase ;
un gène codant pour la 4-hydroxybutyryl-CoA transférase ;
un gène codant pour la 4-hydroxybutyryl-CoA réductase ; et
un gène codant pour l'alcool déshydrogénase, les premier ou second gènes étant exprimés dans la cellule recombinante, et la cellule recombinante produisant du 1,4-butanediol à partir d'au moins un composé C1 choisi dans le groupe constitué de monoxyde de carbone et de dioxyde de carbone.

2. Cellule recombinante selon la revendication 1, la cellule recombinante ayant du monoxyde de carbone déshydrogénase.

3. Cellule recombinante selon la revendication 1 ou 2, la cellule recombinante est *Clostridium ljungdahlii.*

4. Cellule recombinante acétogénique et obligatoirement anaérobique, la cellule recombinante étant une bactérie *Clostridium,* la cellule recombinante comprend des premiers gènes ou des seconds gènes, les premiers gènes comprennent :
un gène codant pour la semi-aldéhyde succinique déshydrogénase dépendante de CoA;
un gène codant pour la 4-hydroxybutyrate déshydrogénase ;
un gène codant pour la 4-hydroxybutyryl-CoA transférase ;
un gène codant pour la 4-hydroxybutyryl-CoA réductase ; et
un gène codant pour l'alcool déshydrogénase, les seconds gènes comprenant :
un gène codant pour la 2-oxoglutarate décarboxylase ;
un gène codant pour la 4-hydroxybutyrate déshydrogénase ;
un gène codant pour la 4-hydroxybutyryl-CoA transférase ;
un gène codant pour la 4-hydroxybutyryl-CoA réductase ; et
un gène codant pour l'alcool déshydrogénase, les premier ou second gènes étant exprimés dans la cellule recombinante, la cellule recombinante ayant une capacité d'assimilation de la cellulose, la cellule recombinante produisant du cellulosome et la cellule recombinante produisant du 1,4-butanediol à partir de la lignocellulose, de la cellulose ou d'un glucide soluble.

5. Cellule recombinante selon la revendication 4, la cellule recombinante étant *Clostridium thermocellum, Clostridium cellulolyticum* ou *Clostridium cellulovorans.*

6. Cellule recombinante selon l'une quelconque des revendications 1 à 5, dans laquelle la capacité de génération d'éthanol de la cellule recombinante est rétro-régulée par rapport à celle de sa cellule hôte d'origine.

7. Cellule recombinante selon l'une quelconque des revendications 1 à 6, dans laquelle la capacité de génération de 2,3-butanediol de la cellule recombinante est rétro-régulée par rapport à celle de sa cellule hôte d'origine.

8. Procédé de production d'une cellule recombinante, comprenant :
une première étape de fourniture d'une cellule hôte qui est acétogénique et obligatoirement anaérobique, la cellule hôte étant une bactérie *Clostridium* ; et
une seconde étape d'introduction de premiers gènes ou de seconds gènes dans la cellule hôte, les premiers gènes comprenant :
un gène codant pour la semi-aldéhyde succinique déshydrogénase dépendante de CoA;
un gène codant pour la 4-hydroxybutyrate déshydrogénase ;
un gène codant pour la 4-hydroxybutyryl-CoA transférase ;
un gène codant pour la 4-hydroxybutyryl-CoA réductase ; et
un gène codant pour l'alcool déshydrogénase, les seconds gènes comprenant :
un gène codant pour la 2-oxoglutarate décarboxylase ;
un gène codant pour la 4-hydroxybutyrate déshydrogénase ;
un gène codant pour la 4-hydroxybutyryl-CoA transférase ;
un gène codant pour la 4-hydroxybutyryl-CoA réductase ; et
un gène codant pour l'alcool déshydrogénase, les premier ou second gènes étant exprimés dans la cellule recombinante, et la cellule recombinante produisant du 1,4-butanediol à partir d'au moins un composé C1 choisi dans le groupe constitué de monoxyde de carbone et de dioxyde de carbone.

9. Procédé de production d'une cellule recombinante, comprenant :
une première étape de fourniture d'une cellule hôte qui est acétogénique et obligatoirement anaérobique, la cellule hôte étant une bactérie *Clostridium* ; et
une seconde étape d'introduction de premiers gènes ou de seconds gènes dans la cellule hôte, les premiers gènes comprenant :
un gène codant pour la semi-aldéhyde succinique déshydrogénase dépendante de CoA;
un gène codant pour la 4-hydroxybutyrate déshydrogénase ;
un gène codant pour la 4-hydroxybutyryl-CoA transférase ;
un gène codant pour la 4-hydroxybutyryl-CoA réductase ; et
un gène codant pour l'alcool déshydrogénase, les seconds gènes comprenant :
un gène codant pour la 2-oxoglutarate décarboxylase ;
un gène codant pour la 4-hydroxybutyrate déshydrogénase ;
un gène codant pour la 4-hydroxybutyryl-CoA transférase ;
un gène codant pour la 4-hydroxybutyryl-CoA réductase ; et
un gène codant pour l'alcool déshydrogénase, les premier ou second gènes étant exprimés dans la cellule recombinante, la cellule recombinante ayant une capacité d'assimilation de la cellulose, la cellule recombinante produisant du cellulosome et la cellule recombinante produisant du 1,4-butanediol à partir de la lignocellulose, de la cellulose, ou d'un glucide soluble.

10. Procédé de production de 1,4-butanediol, comprenant :
la mise en contact d'au moins un composé C1 choisi dans le groupe constitué de monoxyde de carbone et de dioxyde de carbone avec la cellule recombinante selon l'une quelconque des revendications 1 à 3 ou la cellule recombinante produite par le procédé selon la revendication 8, pour amener la cellule recombinante à produire du 1,4-butanediol à partir du composé C1.

11. Procédé selon la revendication 10, comprenant :
la culture de la cellule recombinante au moyen d'au moins un composé C1 choisi dans le groupe constitué de monoxyde de carbone et de dioxyde de carbone comme source de carbone ; et
l'obtention du 1,4-butanediol à partir d'un produit de culture de la cellule recombinante.

12. Procédé selon la revendication 10 ou 11, comprenant :
la fourniture d'un gaz contenant principalement du monoxyde de carbone et de l'hydrogène, d'un gaz contenant principalement du dioxyde de carbone et de l'hydrogène, ou d'un gaz contenant principalement du monoxyde de carbone, du dioxyde de carbone et de l'hydrogène avec la cellule recombinante.

13. Procédé de production de 1,4-butanediol, comprenant :
la mise en contact de la lignocellulose, de la cellulose ou du carbohydrate soluble avec la cellule recombinante selon la revendication 4 ou 5 ou avec la cellule recombinante produite par le procédé selon la revendication 9, pour amener la cellule recombinante à produire du 1,4-butanediol à partir de la lignocellulose, de la cellulose, ou d'un glucide soluble.

14. Procédé selon la revendication 13, comprenant :
la culture de la cellule recombinante en utilisant la lignocellulose, la cellulose ou un glucide soluble comme source de carbone ; et
l'obtention du 1,4-butanediol à partir d'un produit de culture de la cellule recombinante.

15. Procédé selon la revendication 13 ou 14, selon lequel la cellule recombinante est *Clostridium thermocellum, Clostridium cellulolyticum* ou *Clostridium cellulovorans.*


### SEQUENCE LISTING

<110> Sekisui Chemical Co., Ltd. Fraunhofer-Gesellschaft zur Foerderung der angewandten Forschung e.V.
<120> RECOMBINANT CELLS, METHOD FOR PRODUCING RECOMBINANT CELLS, AND METHOD FOR PRODUCING 1,4-BUTANEDIOL
<130> P0003947-PCT
<160> 6
<170> PatentIn version 3.1
<210> 1
   <211> 14588
   <212> DNA
   <213> Artificial
<220>
   <223> Clostridium/E. coli shuttle vector pGn15_SUC(op)
<400> 1
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcg     240
   ccattcgcca ttcaggctgc gcaactgttg ggaagggcga tcggtgcggg cctcttcgct     300
   attacgccag tctagacagg gccccctgct tcggggtcat tatagcgatt ttttcggtat     360
   atccatcctt tttcgcacga tatacaggat tttgccaaag ggttcgtgta gactttcctt     420
   ggtgtatcca acggcgtcag ccgggcagga taggtgaagt aggcccaccc gcgagcgggt     480
   gttccttctt cactgtccct tattcgcacc tggcggtgct caacgggaat cctgctctgc     540
   gaggctggcc ggctaccgcc ggcgtaacag atgagggcaa gcggatggct gatgaaacca     600
   agccaaccag gaagggcagc ccacctatca aggtgtactg ccttccagac gaacgaagag     660
   cgattgagga aaaggcggcg gcggccggca tgagcctgtc ggcctacctg ctggccgtcg     720
   gccagggcta caaaatcacg ggcgtcgtgg actatgagca cgtccgcgag ctggcccgca     780
   tcaatggcga cctgggccgc ctgggcggcc tgctgaaact ctggctcacc gacgacccgc     840
   gcacggcgcg gttcggtgat gccacgatcc tcgccctgct ggcgaagatc gaagagaagc     900
   aggacgagct tggcaaggtc atgatgggcg tggtccgccc gagggcagag ccatgacttt     960
   tttagccgct aaaacggccg gggggtgcgc gtgattgcca agcacgtccc catgcgctcc    1020
   atcaagaaga gcgacttcgc ggagctggtg aagtacatca ccgacgagca aggcaagacc    1080
   gatcgggccc cctaggcctc tggcgaaagg gggatgtgct gcaaggcgat taagttgggt    1140
   aacgccaggg ttttcccagt cacgacgttg taaaacgacg gccagtgcca agcttgagct    1200
   taatcgcact ggccggccgc ttataatcca taacaatcat cctttctgtg acactgtcag 1260
   acacttatca cattaagtat atactattat taaactattc tatatactta atttatttta 1320
   atagaaaaac ataatatcat aataacttca aaattaaact ttatttatga tttcatactt 1380
   gactttgatt ttagaaagga tatacttttt agcagatttg gaaacggctt tggacgtagt 1440
   ttgcccatag atgaacaaac aaactacatc caaaaattat acttttccct tcattggtat 1500
   ccgtattttt acatcttaat agcgtatgta ttacaacaca cctaaacaac gaccttacgg 1560
   tctgctactg catatcctag cttgattgtt tagttgcctc aactatgctt aaccctaccc 1620
   cgaactcttt ttttattgtg ggttttcgtc gtgaagtccc accgacacat aatcataaca 1680
   taagatgtat tatgaaaatg cgagtgacta tccttttgta tcggctcact acaccacaga 1740
   tatatttttt agtgcatact gtgtcggcac tctcaatatt aaattaataa ataaattatt 1800
   ttttcttttt actcttcttt acatgagctt ttttaaagct ccttgcataa tatttaatgc 1860
   atgtacgttc ttttttctgt tcttcctctg taaaacatct catttttatg gcacaccatc 1920
   catatcggtt catctttgaa caattaatac attggacttt ccctttatgt aaacatcttg 1980
   actcattgta tttactgcaa taggttgcta ctgttttatc atgaatcatc aacgaatgta 2040
   ttttgcatat ttcagtatct ttaataaact ctttgcaatt tttacaagtt ttcatatacg 2100
   cccttctttt cataaattaa tttatgaatt ctatgtattc caaggagctt tttaaagctc 2160
   cccctttcgt actacttagc tacaagcact ataaaagtca taatgtttac tgctaaagtc 2220
   aataatgata ttgctaatac cttgttattt gataagatac tgctttcctc tgtcactttg 2280
   ctcaccccct ttcattttca taaattaatt atgaaaaaat aaatatactt ctaattttta 2340
   tcaaataaaa aagcctttgc gtactgcttc caatacacaa aggctataaa cttctaaatc 2400
   ttacttattg caatttacat ttatatctgt taagataatc tcataaattg aatatatata 2460
   gttaatgttt ctcttgtatg tcggtacatt tgaaatattg ctatagatag agttctctaa 2520
   cggcttgatg tgttggtagc acatttaagt tttggcttat atactaagtt ggtagcttaa 2580
   tatataagag ctgaggactt atttttttat taaatttttc aacttgtcta tattttaacc 2640
   cgtaattgaa tacataacaa gtattttttt ttgtattcaa ttaaacattc ataaatgagt 2700
   ataattaatc atactaaatt ctataatttt cttttctgta aatttctttc tattcagcac 2760
   tgttatgcct tttgactatc acttaataaa aaataagaaa tgaattgtca attgttcaaa 2820
   aaaataatgg cggcgcgcca tgcaaaagac ataatcgatt cacaaaaaat aggtacacga 2880
   aaaacaagtt aagggatgca gtttatgcat cccttaactt acttattaaa taatttatag 2940
   ctattgaaaa gagataagaa ttgttcaaag ctaatattgt ttaaatcgtc aattcctgca 3000
   tgttttaagg aattgttaaa ttgatttttt gtaaatattt tcttgtattc tttgttaacc 3060
   catttcataa cgaaataatt atacttctgt ttatctttgt gtgatattct tgattttttt 3120
   ctatttaatc tgataagtga gctattcact ttaggtttag gatgaaaata ttctcttgga 3180
   accatactta atatagaaat atcaacttct gccattaaaa ataatgccaa tgagcgtttt 3240
   gtatttaata atcttttagc aaacccgtat tccacgatta aataaatctc atcagctata 3300
   ctatcaaaaa caattttgcg tattatatcc gtacttatgt tataaggtat attaccaaat 3360
   attttatagg attggttttt aggaaattta aactgcaata tatccttgtt taaaacttgg 3420
   aaattatcgt gatcaacaag tttattttct gtagttttgc ataatttatg gtctatttca 3480
   atggcagtta cgaaattaca cctctgtact aattcaaggg taaaatgccc ttttcctgag 3540
   ccgatttcaa agatattatc atgttcattt aatcttatat ttgtcattat tttatctata 3600
   ttatgttttg aagtaataaa gttttgactg tgttttatat ttttctcgtt cattataacc 3660
   ctctttattt tttcctcctt ataaaattag tataattata gcacgagctc tgataaatat 3720
   gaacatgatg agtgatcgtt aaatttatac tgcaatctga tgcgattatt gaataaaaga 3780
   tatgagagat ttatctagtt tcttttttta caagaaaaaa gaaagttctt aaaggtttta 3840
   tacttttggt cgtagagcac acggtttaac gacttaatta cgaagtaaat aagtctagtg 3900
   tgttagactt taatgttttt ttaaggcatt agtgcattta agcgtcagag catggcttta 3960
   tgccgagaaa actattggtt ggaatggcgt gtgtgttagc caaagctcct gcagtaacta 4020
   gtatcacaag tttgtacaaa aaagcaggct taaggctcct aagtgaaata tatacatatt 4080
   ataacaataa aataagtatt agtgtaggat ttttaaatag agtatctatt ttcagattaa 4140
   atttttgctt atttgattta cattatataa tattgagtaa agtattgact agcaaaattt 4200
   tttgatactt taatttgtga aatttcttat caaaagttat atttttgaat gatttttatt 4260
   gaaaaataca actaaaaagg attatagtat aagtgtgtgt aattttgtgt taaatttaag 4320
   gcagcgttcc aggagggaca gaacaatgaa cttacatgaa tatcaggcaa aacaactttt 4380
   tgcccgctat ggcttaccag caccggtggg ttatgcctgt actactccgc gcgaagcaga 4440
   agaagccgct tcaaaaatcg gtgccggtcc gtgggtagtg aaatgtcagg ttcacgctgg 4500
   tggccgcggt aaagcgggcg gtgtgaaagt tgtaaacagc aaagaagaca tccgtgcttt 4560
   tgcagaaaac tggctgggca agcgtctggt aacgtatcaa acagatgcca atggccaacc 4620
   ggttaaccag attctggttg aagcagcgac cgatatcgct aaagagctgt atctcggtgc 4680
   cgttgttgac cgtagttccc gtcgtgtggt ctttatggcc tccaccgaag gcggcgtgga 4740
   aatcgaaaaa gtggcggaag aaactccgca cctgatccat aaagttgcgc ttgatccgct 4800
   gactggcccg atgccgtatc agggacgcga gctggcgttc aaactgggtc tggaaggtaa 4860
   actggttcag cagttcacca aaatcttcat gggcctggcg accattttcc tggagcgcga 4920
   cctggcgttg atcgaaatca acccgctggt catcaccaaa cagggcgatc tgatttgcct 4980
   cgacggcaaa ctgggcgctg acggcaacgc actgttccgc cagcctgatc tgcgcgaaat 5040
   gcgtgaccag tcgcaggaag atccgcgtga agcacaggct gcacagtggg aactgaacta 5100
   cgttgcgctg gacggtaaca tcggttgtat ggttaacggc gcaggtctgg cgatgggtac 5160
   gatggacatc gttaaactgc acggcggcga accggctaac ttccttgacg ttggcggcgg 5220
   cgcaaccaaa gaacgtgtaa ccgaagcgtt caaaatcatc ctctctgacg acaaagtgaa 5280
   agccgttctg gttaacatct tcggcggtat cgttcgttgc gacctgatcg ctgacggtat 5340
   catcggcgcg gtagcagaag tgggtgttaa cgtaccggtc gtggtacgtc tggaaggtaa 5400
   caacgccgaa ctcggcgcga agaaactggc tgacagcggc ctgaatatta ttgcagcaaa 5460
   aggtctgacg gatgcagctc agcaggttgt tgccgcagtg gaggggaaat aacaaggcaa 5520
   agtgaggagg taaggtatga gtaatgaagt atctataaaa gaattaattg aaaaggcaaa 5580
   ggtggcacaa aaaaaattgg aagcctatag tcaagaacaa gttgatgtac tagtaaaagc 5640
   actaggaaaa gtggtttatg ataatgcaga aatgtttgca aaagaagcag ttgaagaaac 5700
   agaaatgggt gtttatgaag ataaagtagc taaatgtcat ttgaaatcag gagctatttg 5760
   gaatcatata aaagacaaga aaactgtagg cataataaaa gaagaacctg aaagggcact 5820
   tgtttatgtt gctaagccaa agggagttgt ggcagctact acgcctataa ctaatccagt 5880
   ggtaactcct atgtgtaatg caatggctgc tataaagggc agaaatacaa taatagtagc 5940
   accacatcct aaagcaaaga aagtttcagc tcatactgta gaacttatga atgctgagct 6000
   taaaaaattg ggagcaccag aaaatatcat acagatagta gaagcaccat caagagaagc 6060
   tgctaaggaa cttatggaaa gtgctgatgt agttattgct acaggcggtg ctggaagagt 6120
   taaagctgct tactccagtg gaagaccagc ttatggcgtt ggacctggaa attcacaggt 6180
   aatagttgat aagggatacg attataacaa agctgcacag gatataataa caggaagaaa 6240
   atatgacaat ggaattatat gttcttcaga gcaatcagtt atagctcctg ctgaagatta 6300
   tgataaggta atagcagctt ttgtagaaaa tggggcattc tatgtagaag atgaggaaac 6360
   agtagaaaag tttagatcaa ctttatttaa agatggaaaa ataaacagca agattatagg 6420
   taaatccgtc caaattattg cggatcttgc aggagtaaaa gtaccagaag gtactaaggt 6480
   tatagtactt aagggtaaag gtgcaggaga aaaagatgta ctttgtaaag aaaaaatgtg 6540
   tccagtttta gtagcattga aatatgatac ttttgaagaa gcagttgaaa tagctatggc 6600
   taattatatg tatgaaggag ctggtcatac agcaggcata cattctgaca atgacgagaa 6660
   cataagatat gcaggaactg tattacctat aagcagatta gttgtaaatc agcctgcaac 6720
   tactgctgga ggaagtttca ataatggatt taaccctact actacactag gctgcggatc 6780
   atggggcaga aacagtattt cagaaaatct tacttacgag catcttataa atgtttcaag 6840
   aatagggtat ttcaataaag aagcaaaagt tcctagctat gaggaaatat ggggataaaa 6900
   gaggaggtta ggcatgcagc tgtttaaact caagagtgta acacatcatt tcgatacttt 6960
   tgcagaattt gctaaagagt tctgtttagg agaaagagat cttgtgatca ctaacgaatt 7020
   tatctacgaa ccatatatga aagcttgtca attaccttgt cactttgtta tgcaggaaaa 7080
   gtatggtcaa ggtgaaccta gtgatgaaat gatgaacaat atactcgcag acattcgtaa 7140
   tattcagttt gatagagtca taggaatagg aggaggtact gtgatagaca tcagtaagct 7200
   atttgttctt aaaggtctca atgacgtttt agatgcattt gatagaaaaa tccccttaat 7260
   caaagagaag gaactcatca tagttcctac aacatgtggt acaggaagtg aagtaacgaa 7320
   tataagcata gctgaaatca aatcaagaca cactaaaatg ggtttagctg atgatgcaat 7380
   agtagcagat catgcgatta taatacccga actacttaaa tctctccctt ttcattttta 7440
   tgcttgtagt gctattgatg cattgattca tgcaattgag tcttatgtaa gccctaaagc 7500
   aagtccatat agcagattat tcagtgaagc agcatgggat ataatactcg aggtttttaa 7560
   aaagattgct gaacatggac cagaatatag gtttgagaag ttaggagaga tgataatggc 7620
   ttcgaattat gcaggtattg catttggaaa tgctggtgtt ggtgctgtac atgcattatc 7680
   ttacccttta ggtggaaatt atcatgttcc acatggagaa gcaaactatc aattttttac 7740
   cgaagttttt aaagtttatc aaaagaaaaa tccgtttgga tatatcgttg agttaaactg 7800
   gaaactttcc aaaattctca attgtcaacc tgaatacgta tatcctaagc tagatgaact 7860
   tctcggttgt cttttgacca aaaaaccctt acatgaatat ggaatgaaag atgaagaagt 7920
   tagaggattt gctgaatctg tgttaaagac gcaacaaaga ttattggcta acaattatgt 7980
   cgaacttaca gtagatgaga tagaagggat ttataggaga ctttactaga gcttccgcgg 8040
   aggccggcgc ggccgattta aatcctcagc ctgagccccc gggcggtatc agctcactca 8100
   aaggcggtaa taacaacttt gtatacaaaa gttgtagtgt agtagcctgt gaaataagta 8160
   aggaaaaaaa agaagtaagt gttatatatg atgattattt tgtagatgta gataggataa 8220
   tagaatccat agaaaatata ggttatacag ttatataaaa attactttaa aaattaataa 8280
   aaacatggta aaatataaat cgtataaagt tgtgtaattt ttaaggaggt gtgttacata 8340
   tgcagtggca agaactgtat cgccagagag tgtgctcagc ggatgaagcc gtagtggata 8400
   gcctgaaacc gggaacgaaa gtggtgtttg gtcatgcagc tgcggcccct gtaagatttt 8460
   ctcaggctat gtatcgccaa agagaaaaac tggaaaatat tacagtgttt catatgcttt 8520
   attttggcga tgcccctcac cttgcaccgg aaatgcgtag ccatgttcac cctactctta 8580
   attttcttga gggtaattct cgtccggcca gccgcgatag acgtgtggat tttataccgt 8640
   gtcattttca cgaagttcca gaactgtttc gccagggttt ctttccatta gatgttgcag 8700
   ttgtacaagt atctacccct aatgaagaag gatattgcag ttttggcgtt tcatgcgatt 8760
   atacgaaagc agctgcggaa tgcgccccgg tggttgtagc agaagtaaat aaacagatgc 8820
   catttattgg tggagaaaat ctgattcata tttcaaaact tacccacata attgaagtag 8880
   atgaaccgat agctgaagtg cttccgccag cgattagtga tctggaatta cgtattggtc 8940
   agaattgtgc atcactgatt aaagatggcg atacacttca actgggtatt ggcggtatac 9000
   cagatgctgt gctgcgcgcg ttagaaggtc ataaagattt aggaattcac accgaaatgt 9060
   ttacggatgg agtaatgcgc atgataagaa aaggcataat taatggtaaa aagaaaacac 9120
   ttcatccaga aaaagtggtt acgtctctta tttttggcag caaagaactg tatgattttg 9180
   tgaataataa tccagttatt gaatgttatc ctgtggatta tataaataat cctgatgtta 9240
   taggaaaaaa tgatcgtatg gtatctatta attcatgcct tgaaatggat ctgatgggcc 9300
   aggccgcaag tgaatcaata ggatatgaac agttttctgg cagcggaggc caagttgatt 9360
   ttctgagagg tgctaaacgt agtaaaggtg gaatatcaat tatggctttt ccttctacag 9420
   caaagaaagg tactgaaagc cgcattgttc cgatattaaa agaaggtgct tgcgttacaa 9480
   ctggaagaaa tgaagtagat tatgtagtga ctgaatatgg cgtagcccgt ttacgcggtg 9540
   caacacttag acaacgtgct gaagcgttaa ctgctatagc acatccagat tttagaccag 9600
   cattagaaga ggaaatccgc cgacgcttcg aataaggcat gcacggtagc caggaggtta 9660
   atgaaagtta caaatcaaaa agaactaaaa caaaagctaa atgaattgag agaagcgcaa 9720
   aagaagtttg caacctatac tcaagagcaa gttgataaaa tttttaaaca atgtgccata 9780
   gccgcagcta aagaaagaat aaacttagct aaattagcag tagaagaaac aggaataggt 9840
   cttgtagaag ataaaattat aaaaaatcat tttgcagcag aatatatata caataaatat 9900
   aaaaatgaaa aaacttgtgg cataatagac catgacgatt ctttaggcat aacaaaggtt 9960
   gctgaaccaa ttggaattgt tgcagccata gttcctacta ctaatccaac ttccacagca 10020
   attttcaaat cattaatttc tttaaaaaca agaaacgcaa tattcttttc accacatcca 10080
   cgtgcaaaaa aatctacaat tgctgcagca aaattaattt tagatgcagc tgttaaagca 10140
   ggagcaccta aaaatataat aggctggata gatgagccat caatagaact ttctcaagat 10200
   ttgatgagtg aagctgatat aatattagca acaggaggtc cttcaatggt taaagcggcc 10260
   tattcatctg gaaaacctgc aattggtgtt ggagcaggaa atacaccagc aataatagat 10320
   gagagtgcag atatagatat ggcagtaagc tccataattt tatcaaagac ttatgacaat 10380
   ggagtaatat gcgcttctga acaatcaata ttagttatga attcaatata cgaaaaagtt 10440
   aaagaggaat ttgtaaaacg aggatcatat atactcaatc aaaatgaaat agctaaaata 10500
   aaagaaacta tgtttaaaaa tggagctatt aatgctgaca tagttggaaa atctgcttat 10560
   ataattgcta aaatggcagg aattgaagtt cctcaaacta caaagatact tataggcgaa 10620
   gtacaatctg ttgaaaaaag cgagctgttc tcacatgaaa aactatcacc agtacttgca 10680
   atgtataaag ttaaggattt tgatgaagct ctaaaaaagg cacaaaggct aatagaatta 10740
   ggtggaagtg gacacacgtc atctttatat atagattcac aaaacaataa ggataaagtt 10800
   aaagaatttg gattagcaat gaaaacttca aggacattta ttaacatgcc ttcttcacag 10860
   ggagcaagcg gagatttata caattttgcg atagcaccat catttactct tggatgcggc 10920
   acttggggag gaaactctgt atcgcaaaat gtagagccta aacatttatt aaatattaaa 10980
   agtgttgctg aaagaaggga aaatatgctt tggtttaaag tgccacaaaa aatatatttt 11040
   aaatatggat gtcttagatt tgcattaaaa gaattaaaag atatgaataa gaaaagagcc 11100
   tttatagtaa cagataaaga tctttttaaa cttggatatg ttaataaaat aacaaaggta 11160
   ctagatgaga tagatattaa atacagtata tttacagata ttaaatctga tccaactatt 11220
   gattcagtaa aaaaaggtgc taaagaaatg cttaactttg aacctgatac tataatctct 11280
   attggtggtg gatcgccaat ggatgcagca aaggttatgc acttgttata tgaatatcca 11340
   gaagcagaaa ttgaaaatct agctataaac tttatggata taagaaagag aatatgcaat 11400
   ttccctaaat taggtacaaa ggcgatttca gtagctattc ctacaactgc tggtaccggt  11460
   tcagaggcaa caccttttgc agttataact aatgatgaaa caggaatgaa atacccttta  11520
   acttcttatg aattgacccc aaacatggca ataatagata ctgaattaat gttaaatatg 11580
   cctagaaaat taacagcagc aactggaata gatgcattag ttcatgctat agaagcatat 11640
   gtttcggtta tggctacgga ttatactgat gaattagcct taagagcaat aaaaatgata 11700
   tttaaatatt tgcctagagc ctataaaaat gggactaacg acattgaagc aagagaaaaa 11760
   atggcacatg cctctaatat tgcggggatg gcatttgcaa atgctttctt aggtgtatgc 11820
   cattcaatgg ctcataaact tggggcaatg catcacgttc cacatggaat tgcttgtgct 11880
   gtattaatag aagaagttat taaatataac gctacagact gtccaacaaa gcaaacagca 11940
   ttccctcaat ataaatctcc taatgctaag agaaaatatg ctgaaattgc agagtatttg 12000
   aatttaaagg gtactagcga taccgaaaag gtaacagcct taatagaagc tatttcaaag 12060
   ttaaagatag atttgagtat tccacaaaat ataagtgccg ctggaataaa taaaaaagat 12120
   ttttataata cgctagataa aatgtcagag cttgcttttg atgaccaatg tacaacagct 12180
   aatcctaggt atccacttat aagtgaactt aaggatatct atataaaatc attttaaccc 12240
   gggccccggg aacccagctt tcttgtacaa agtggtgatc tcgagacctt aagtaataaa 12300
   aataagagtt accttaaatg gtaactctta tttttttaat gtcgactcat agaattcgta 12360
   atcatggtca tagctgtttc ctgtgtgaaa ttgttatccg ctcacaattc cacacaacat 12420
   acgagccgga agcataaagt gtaaagcctg gggtgcctaa tgagtgagct aactcacatt 12480
   aattgcgttg cgctcactgc ccgctttcca gtcgggaaac ctgtcgtgcc agctgcatta 12540
   atgaatcggc caacgcgcgg ggagaggcgg tttgcgtatt gggcgctctt ccgcttcctc 12600
   gctcactgac tcgctgcgct cggtcgttcg gctgcggcga gcggtatcag ctcactcaaa 12660
   ggcggtaata cggttatcca cagaatcagg ggataacgca ggaaagaaca tgtgagcaaa 12720
   aggccagcaa aaggccagga accgtaaaaa ggccgcgttg ctggcgtttt tccataggct 12780
   ccgcccccct gacgagcatc acaaaaatcg acgctcaagt cagaggtggc gaaacccgac 12840
   aggactataa agataccagg cgtttccccc tggaagctcc ctcgtgcgct ctcctgttcc 12900
   gaccctgccg cttaccggat acctgtccgc ctttctccct tcgggaagcg tggcgctttc 12960
   tcatagctca cgctgtaggt atctcagttc ggtgtaggtc gttcgctcca agctgggctg 13020
   tgtgcacgaa ccccccgttc agcccgaccg ctgcgcctta tccggtaact atcgtcttga 13080
   gtccaacccg gtaagacacg acttatcgcc actggcagca gccactggta acaggattag 13140
   cagagcgagg tatgtaggcg gtgctacaga gttcttgaag tggtggccta actacggcta 13200
   cactagaaga acagtatttg gtatctgcgc tctgctgaag ccagttacct tcggaaaaag 13260
   agttggtagc tcttgatccg gcaaacaaac caccgctggt agcggtggtt tttttgtttg 13320
   caagcagcag attacgcgca gaaaaaaagg atctcaagaa gatcctttga tcttttctac 13380
   ggggtctgac gctcagtgga acgaaaactc acgttaaggg attttggtca tgagattatc 13440
   aaaaaggatc ttcacctaga tccttttaaa ttaaaaatga agttttaaat caatctaaag 13500
   tatatatgag taaacttggt ctgacagtta ccaatgctta atcagtgagg cacctatctc 13560
   agcgatctgt ctatttcgtt catccatagt tgcctgactc cccgtcgtgt agataactac 13620
   gatacgggag ggcttaccat ctggccccag tgctgcaatg ataccgcgag acccacgctc 13680
   accggctcca gatttatcag caataaacca gccagccgga agggccgagc gcagaagtgg 13740
   tcctgcaact ttatccgcct ccatccagtc tattaattgt tgccgggaag ctagagtaag 13800
   tagttcgcca gttaatagtt tgcgcaacgt tgttgccatt gctacaggca tcgtggtgtc 13860
   acgctcgtcg tttggtatgg cttcattcag ctccggttcc caacgatcaa ggcgagttac 13920
   atgatccccc atgttgtgca aaaaagcggt tagctccttc ggtcctccga tcgttgtcag 13980
   aagtaagttg gccgcagtgt tatcactcat ggttatggca gcactgcata attctcttac 14040
   tgtcatgcca tccgtaagat gcttttctgt gactggtgag tactcaacca agtcattctg 14100
   agaatagtgt atgcggcgac cgagttgctc ttgcccggcg tcaatacggg ataataccgc 14160
   gccacatagc agaactttaa aagtgctcat cattggaaaa cgttcttcgg ggcgaaaact 14220
   ctcaaggatc ttaccgctgt tgagatccag ttcgatgtaa cccactcgtg cacccaactg 14280
   atcttcagca tcttttactt tcaccagcgt ttctgggtga gcaaaaacag gaaggcaaaa 14340
   tgccgcaaaa aagggaataa gggcgacacg gaaatgttga atactcatac tcttcctttt 14400
   tcaatattat tgaagcattt atcagggtta ttgtctcatg agcggataca tatttgaatg 14460
   tatttagaaa aataaacaaa taggggttcc gcgcacattt ccccgaaaag tgccacctga  14520
   cgtctaagaa accattatta tcatgacatt aacctataaa aataggcgta tcacgaggcc  14580
   ctttcgtc                                                           14588
<210> 2
   <211> 15674
   <212> DNA
   <213> Artificial
<220>
   <223> Clostridium/E. coli shuttle vector pGn15_AKG(op)
<400> 2
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcg     240
   ccattcgcca ttcaggctgc gcaactgttg ggaagggcga tcggtgcggg cctcttcgct     300
   attacgccag tctagacagg gccccctgct tcggggtcat tatagcgatt ttttcggtat     360
   atccatcctt tttcgcacga tatacaggat tttgccaaag ggttcgtgta gactttcctt     420
   ggtgtatcca acggcgtcag ccgggcagga taggtgaagt aggcccaccc gcgagcgggt     480
   gttccttctt cactgtccct tattcgcacc tggcggtgct caacgggaat cctgctctgc     540
   gaggctggcc ggctaccgcc ggcgtaacag atgagggcaa gcggatggct gatgaaacca     600
   agccaaccag gaagggcagc ccacctatca aggtgtactg ccttccagac gaacgaagag     660
   cgattgagga aaaggcggcg gcggccggca tgagcctgtc ggcctacctg ctggccgtcg     720
   gccagggcta caaaatcacg ggcgtcgtgg actatgagca cgtccgcgag ctggcccgca     780
   tcaatggcga cctgggccgc ctgggcggcc tgctgaaact ctggctcacc gacgacccgc     840
   gcacggcgcg gttcggtgat gccacgatcc tcgccctgct ggcgaagatc gaagagaagc     900
   aggacgagct tggcaaggtc atgatgggcg tggtccgccc gagggcagag ccatgacttt     960
   tttagccgct aaaacggccg gggggtgcgc gtgattgcca agcacgtccc catgcgctcc    1020
   atcaagaaga gcgacttcgc ggagctggtg aagtacatca ccgacgagca aggcaagacc    1080
   gatcgggccc cctaggcctc tggcgaaagg gggatgtgct gcaaggcgat taagttgggt    1140
   aacgccaggg ttttcccagt cacgacgttg taaaacgacg gccagtgcca agcttgagct    1200
   taatcgcact ggccggccgc ttataatcca taacaatcat cctttctgtg acactgtcag    1260
   acacttatca cattaagtat atactattat taaactattc tatatactta atttatttta    1320
   atagaaaaac ataatatcat aataacttca aaattaaact ttatttatga tttcatactt    1380
   gactttgatt ttagaaagga tatacttttt agcagatttg gaaacggctt tggacgtagt 1440
   ttgcccatag atgaacaaac aaactacatc caaaaattat acttttccct tcattggtat 1500
   ccgtattttt acatcttaat agcgtatgta ttacaacaca cctaaacaac gaccttacgg 1560
   tctgctactg catatcctag cttgattgtt tagttgcctc aactatgctt aaccctaccc 1620
   cgaactcttt ttttattgtg ggttttcgtc gtgaagtccc accgacacat aatcataaca 1680
   taagatgtat tatgaaaatg cgagtgacta tccttttgta tcggctcact acaccacaga 1740
   tatatttttt agtgcatact gtgtcggcac tctcaatatt aaattaataa ataaattatt 1800
   ttttcttttt actcttcttt acatgagctt ttttaaagct ccttgcataa tatttaatgc 1860
   atgtacgttc ttttttctgt tcttcctctg taaaacatct catttttatg gcacaccatc 1920
   catatcggtt catctttgaa caattaatac attggacttt ccctttatgt aaacatcttg 1980
   actcattgta tttactgcaa taggttgcta ctgttttatc atgaatcatc aacgaatgta 2040
   ttttgcatat ttcagtatct ttaataaact ctttgcaatt tttacaagtt ttcatatacg 2100
   cccttctttt cataaattaa tttatgaatt ctatgtattc caaggagctt tttaaagctc 2160
   cccctttcgt actacttagc tacaagcact ataaaagtca taatgtttac tgctaaagtc 2220
   aataatgata ttgctaatac cttgttattt gataagatac tgctttcctc tgtcactttg 2280
   ctcaccccct ttcattttca taaattaatt atgaaaaaat aaatatactt ctaattttta 2340
   tcaaataaaa aagcctttgc gtactgcttc caatacacaa aggctataaa cttctaaatc 2400
   ttacttattg caatttacat ttatatctgt taagataatc tcataaattg aatatatata 2460
   gttaatgttt ctcttgtatg tcggtacatt tgaaatattg ctatagatag agttctctaa 2520
   cggcttgatg tgttggtagc acatttaagt tttggcttat atactaagtt ggtagcttaa 2580
   tatataagag ctgaggactt atttttttat taaatttttc aacttgtcta tattttaacc 2640
   cgtaattgaa tacataacaa gtattttttt ttgtattcaa ttaaacattc ataaatgagt 2700
   ataattaatc atactaaatt ctataatttt cttttctgta aatttctttc tattcagcac 2760
   tgttatgcct tttgactatc acttaataaa aaataagaaa tgaattgtca attgttcaaa 2820
   aaaataatgg cggcgcgcca tgcaaaagac ataatcgatt cacaaaaaat aggtacacga 2880
   aaaacaagtt aagggatgca gtttatgcat cccttaactt acttattaaa taatttatag 2940
   ctattgaaaa gagataagaa ttgttcaaag ctaatattgt ttaaatcgtc aattcctgca 3000
   tgttttaagg aattgttaaa ttgatttttt gtaaatattt tcttgtattc tttgttaacc 3060
   catttcataa cgaaataatt atacttctgt ttatctttgt gtgatattct tgattttttt 3120
   ctatttaatc tgataagtga gctattcact ttaggtttag gatgaaaata ttctcttgga 3180
   accatactta atatagaaat atcaacttct gccattaaaa ataatgccaa tgagcgtttt 3240
   gtatttaata atcttttagc aaacccgtat tccacgatta aataaatctc atcagctata 3300
   ctatcaaaaa caattttgcg tattatatcc gtacttatgt tataaggtat attaccaaat   3360
   attttatagg attggttttt aggaaattta aactgcaata tatccttgtt taaaacttgg   3420
   aaattatcgt gatcaacaag tttattttct gtagttttgc ataatttatg gtctatttca   3480
   atggcagtta cgaaattaca cctctgtact aattcaaggg taaaatgccc ttttcctgag   3540
   ccgatttcaa agatattatc atgttcattt aatcttatat ttgtcattat tttatctata   3600
   ttatgttttg aagtaataaa gttttgactg tgttttatat ttttctcgtt cattataacc   3660
   ctctttattt tttcctcctt ataaaattag tataattata gcacgagctc tgataaatat   3720
   gaacatgatg agtgatcgtt aaatttatac tgcaatctga tgcgattatt gaataaaaga   3780
   tatgagagat ttatctagtt tcttttttta caagaaaaaa gaaagttctt aaaggtttta   3840
   tacttttggt cgtagagcac acggtttaac gacttaatta cgaagtaaat aagtctagtg   3900
   tgttagactt taatgttttt ttaaggcatt agtgcattta agcgtcagag catggcttta   3960
   tgccgagaaa actattggtt ggaatggcgt gtgtgttagc caaagctcct gcagtaacta   4020
   gtatcacaag tttgtacaaa aaagcaggct taaggctcct aagtgaaata tatacatatt   4080
   ataacaataa aataagtatt agtgtaggat ttttaaatag agtatctatt ttcagattaa   4140
   atttttgctt atttgattta cattatataa tattgagtaa agtattgact agcaaaattt   4200
   tttgatactt taatttgtga aatttcttat caaaagttat atttttgaat gatttttatt   4260
   gaaaaataca actaaaaagg attatagtat aagtgtgtgt aattttgtgt taaatttaag   4320
   ccagctgtag gagggaagca tgtacagaaa atttagagat gatcctagta gtgtagatcc   4380
   tagttggcat gaatttttag ttgattatag tcctgaacct acatcacaac ctgcagcaga   4440
   acctacaaga gttacatcac ctttagttgc agaaagagca gcagcagcag cacctcaagc   4500
   acctcctaaa cctgcagata cagcagcagc aggaaatgga gttgttgcag cattagcagc   4560
   aaaaacagct gttcctcctc ctgcagaagg agatgaagtt gcagttttaa gaggagcagc   4620
   agctgcagtt gttaaaaaca tgagtgctag tttagaagtt cctacagcaa caagtgttag   4680
   agcagttcct gcaaaattac tgatagacaa cagaatagtt ataaacaatc aattaaaaag   4740
   aacaagagga ggaaaaataa gttttacaca tttattagga tatgcattag ttcaagcagt   4800
   taaaaaattt cctaatatga acagacacta tacagaagtc gatggaaaac ctacagcagt   4860
   tacacctgca cacacaaact taggattagc gatagatcta caaggaaaag atggaaaaag   4920
   aagtttagta gttgcaggta taaagagatg tgaaacaatg agatttgcac aatttgttac   4980
   agcatatgaa gatatagtac gtagagcaag agatggaaaa ttaacgacag aagattttgc   5040
   aggagttaca ataagtctta caaatcctgg aacaatagga acagttcata gtgttcctag   5100
   attaatgcca ggacaaggag caataatagg agttggagca atggaatatc ctgctgaatt   5160
   tcaaggagca tctgaagaaa gaatagctga attaggtatt gggaaattaa taaccttaac 5220
   atctacatat gatcatagaa taatacaagg agcagaaagt ggagattttt taagaacgat 5280
   acatgaatta ctgctcagtg atggattttg ggatgaagtg tttagagaat tgtccatacc 5340
   atatttacct gttagatggt ctacagataa tcctgatagt atagttgata aaaatgcaag 5400
   agttatgaat ttaatagcag catatagaaa tagaggacat ttaatggcag atacagatcc 5460
   cttacgatta gataaagcaa gatttagaag tcatcctgat ttagaagttt taacacatgg 5520
   attaacactt tgggatttag atagagtttt caaagttgat ggatttgcag gagcacaata 5580
   taagaaactc agagatgtct taggattgtt aagagatgca tattgtcgcc atataggagt 5640
   tgaatatgca catatcttag atcctgaaca aaaggaatgg ttagaacaaa gagttgagac 5700
   taaacatgtc aaacctacag ttgcacaaca aaagtatatc ttaagcaaat taaatgcagc 5760
   tgaagcattt gaaacatttt tacagacaaa atatgttgga cagaaaagat ttagtttaga 5820
   aggagcagaa agtgttatac ctatgatgga tgcagcaata gatcaatgtg cagaacatgg 5880
   attagatgaa gtagttatag gaatgcctca tagaggaaga ctaaatgttc tagcaaatat 5940
   agttgggaaa ccgtatagtc aaatattcac agaattcgaa ggaaatctga atccaagtca 6000
   agcacatgga agtggagatg ttaaatatca tttaggagca acaggattat acttacaaat 6060
   gtttggagat aacgatatac aagtttcttt aacagcaaat cctagtcatt tagaagcagt 6120
   tgaccctgtt ttagaaggat tagttagagc aaaacaagat ttactcgatc atggaagtat 6180
   cgatagtgat ggccaaagag cattttcagt tgttccgtta atgttacatg gagatgcagc 6240
   atttgcagga caaggagttg ttgcagaaac actgaattta gcaaatttac ctggatatag 6300
   agttggagga acaatacaca tcatagttaa taatcagata ggatttacaa cagcacctga 6360
   atatagtcgt agtagtgaat attgtacaga tgttgcaaaa atgataggag ctcctatatt 6420
   tcatgttaat ggagatgatc ctgaagcatg tgtttgggtt gcaagattag cagttgattt 6480
   tagacaaaga tttaagaaag atgttgttat agacatgcta tgctatagaa gaagaggaca 6540
   taacgaagga gatgatccta gtatgacaaa tccttacatg tatgacgttg ttgatacgaa 6600
   aagaggagct agaaagagtt atacagaagc attaatagga agaggagata taagtatgaa 6660
   agaagcagaa gatgcattaa gggattatca aggacaatta gaaagagttt tcaatgaagt 6720
   tagagaatta gaaaaacatg gcgttcaacc tagtgaaagt gtagaaagtg accaaatgat 6780
   tcctgcagga ttagctacag cagttgataa aagtctgtta gcaagaatag gagatgcatt 6840
   tttagcatta cctaatggat ttactgcaca tcctagagtt caacctgtgt tagaaaagag 6900
   aagagaaatg gcatatgagg gaaaaataga ttgggcattt ggagaactat tagcattagg 6960
   gagtttagtt gcagaaggaa aattagtgcg cttaagtgga caagatagta gaagaggtac 7020
   cttcagtcaa agacacagtg ttttaataga cagacataca ggtgaagagt ttacaccgtt 7080
   acaattgtta gcaacaaata gtgatggaag tcctacagga gggaaatttc tcgtttatga 7140
   tagtccttta agtgaatatg cagcagttgg atttgagtat ggatatacag ttggaaatcc 7200
   tgatgcagtt gttttatggg aagcacaatt tggagatttt gttaatggag cacagagtat 7260
   aatcgacgaa tttatatcta gtggagaggc aaaatgggga caattaagca atgtcgtttt 7320
   attgttacct catggacatg aaggacaagg acctgatcat acaagtgcaa gaatcgaaag 7380
   atttttacaa ctatgggcag aaggaagtat gacaattgca atgcctagta caccttccaa 7440
   ttactttcat cttttacgta gacatgcatt agatggaatt caaagaccct taatagtttt 7500
   tacacccaaa agtatgctta gacataaagc tgcagttagt gagataaagg actttacaga 7560
   aatcaaattt cgaagtgttt tagaagagcc tacttatgaa gatggaatag gagatagaaa 7620
   taaagtttca agaatattac tcacaagtgg aaagttatat tatgagttag cagcaagaaa 7680
   ggcaaaagac aatagaaatg atttagcaat agttagatta gaacaattag cacctttacc 7740
   aagaagaaga ctaagagaaa cattagatag atatgagaac gttaaagaat tcttttgggt 7800
   tcaagaagaa cctgcaaatc aaggagcatg gcctagattt gggttagaat tgcctgaatt 7860
   attacctgat aaactagctg gaataaaaag aatatcaaga agagcaatga gtgcacctag 7920
   tagtggaagt agtaaagttc atgcagttga acaacaagag atattagatg aagcatttgg 7980
   atagaagagg aggttaggca tgcagctgtt taaactcaag agtgtaacac atcatttcga 8040
   tacttttgca gaatttgcta aagagttctg tttaggagaa agagatcttg tgatcactaa 8100
   cgaatttatc tacgaaccat atatgaaagc ttgtcaatta ccttgtcact ttgttatgca 8160
   ggaaaagtat ggtcaaggtg aacctagtga tgaaatgatg aacaatatac tcgcagacat 8220
   tcgtaatatt cagtttgata gagtcatagg aataggagga ggtactgtga tagacatcag 8280
   taagctattt gttcttaaag gtctcaatga cgttttagat gcatttgata gaaaaatccc 8340
   cttaatcaaa gagaaggaac tcatcatagt tcctacaaca tgtggtacag gaagtgaagt 8400
   aacgaatata agcatagctg aaatcaaatc aagacacact aaaatgggtt tagctgatga 8460
   tgcaatagta gcagatcatg cgattataat acccgaacta cttaaatctc tcccttttca 8520
   tttttatgct tgtagtgcta ttgatgcatt gattcatgca attgagtctt atgtaagccc 8580
   taaagcaagt ccatatagca gattattcag tgaagcagca tgggatataa tactcgaggt 8640
   ttttaaaaag attgctgaac atggaccaga atataggttt gagaagttag gagagatgat 8700
   aatggcttcg aattatgcag gtattgcatt tggaaatgct ggtgttggtg ctgtacatgc 8760
   attatcttac cctttaggtg gaaattatca tgttccacat ggagaagcaa actatcaatt 8820
   ttttaccgaa gtttttaaag tttatcaaaa gaaaaatccg tttggatata tcgttgagtt 8880
   aaactggaaa ctttccaaaa ttctcaattg tcaacctgaa tacgtatatc ctaagctaga 8940
   tgaacttctc ggttgtcttt tgaccaaaaa acccttacat gaatatggaa tgaaagatga 9000
   agaagttaga ggatttgctg aatctgtgtt aaagacgcaa caaagattat tggctaacaa 9060
   ttatgtcgaa cttacagtag atgagataga agggatttat aggagacttt actagagctt 9120
   ccgcggaggc cggcgcggcc gatttaaatc ctcagcctga gcccccgggc ggtatcagct 9180
   cactcaaagg cggtaataac aactttgtat acaaaagttg tagtgtagta gcctgtgaaa 9240
   taagtaagga aaaaaaagaa gtaagtgtta tatatgatga ttattttgta gatgtagata 9300
   ggataataga atccatagaa aatataggtt atacagttat ataaaaatta ctttaaaaat 9360
   taataaaaac atggtaaaat ataaatcgta taaagttgtg taatttttaa ggaggtgtgt 9420
   tacatatgca gtggcaagaa ctgtatcgcc agagagtgtg ctcagcggat gaagccgtag 9480
   tggatagcct gaaaccggga acgaaagtgg tgtttggtca tgcagctgcg gcccctgtaa 9540
   gattttctca ggctatgtat cgccaaagag aaaaactgga aaatattaca gtgtttcata 9600
   tgctttattt tggcgatgcc cctcaccttg caccggaaat gcgtagccat gttcacccta 9660
   ctcttaattt tcttgagggt aattctcgtc cggccagccg cgatagacgt gtggatttta 9720
   taccgtgtca ttttcacgaa gttccagaac tgtttcgcca gggtttcttt ccattagatg 9780
   ttgcagttgt acaagtatct acccctaatg aagaaggata ttgcagtttt ggcgtttcat 9840
   gcgattatac gaaagcagct gcggaatgcg ccccggtggt tgtagcagaa gtaaataaac 9900
   agatgccatt tattggtgga gaaaatctga ttcatatttc aaaacttacc cacataattg 9960
   aagtagatga accgatagct gaagtgcttc cgccagcgat tagtgatctg gaattacgta 10020
   ttggtcagaa ttgtgcatca ctgattaaag atggcgatac acttcaactg ggtattggcg 10080
   gtataccaga tgctgtgctg cgcgcgttag aaggtcataa agatttagga attcacaccg 10140
   aaatgtttac ggatggagta atgcgcatga taagaaaagg cataattaat ggtaaaaaga 10200
   aaacacttca tccagaaaaa gtggttacgt ctcttatttt tggcagcaaa gaactgtatg 10260
   attttgtgaa taataatcca gttattgaat gttatcctgt ggattatata aataatcctg 10320
   atgttatagg aaaaaatgat cgtatggtat ctattaattc atgccttgaa atggatctga 10380
   tgggccaggc cgcaagtgaa tcaataggat atgaacagtt ttctggcagc ggaggccaag 10440
   ttgattttct gagaggtgct aaacgtagta aaggtggaat atcaattatg gcttttcctt 10500
   ctacagcaaa gaaaggtact gaaagccgca ttgttccgat attaaaagaa ggtgcttgcg 10560
   ttacaactgg aagaaatgaa gtagattatg tagtgactga atatggcgta gcccgtttac 10620
   gcggtgcaac acttagacaa cgtgctgaag cgttaactgc tatagcacat ccagatttta 10680
   gaccagcatt agaagaggaa atccgccgac gcttcgaata aggcatgcac ggtagccagg 10740
   aggttaatga aagttacaaa tcaaaaagaa ctaaaacaaa agctaaatga attgagagaa 10800
   gcgcaaaaga agtttgcaac ctatactcaa gagcaagttg ataaaatttt taaacaatgt 10860
   gccatagccg cagctaaaga aagaataaac ttagctaaat tagcagtaga agaaacagga 10920
   ataggtcttg tagaagataa aattataaaa aatcattttg cagcagaata tatatacaat 10980
   aaatataaaa atgaaaaaac ttgtggcata atagaccatg acgattcttt aggcataaca 11040
   aaggttgctg aaccaattgg aattgttgca gccatagttc ctactactaa tccaacttcc 11100
   acagcaattt tcaaatcatt aatttcttta aaaacaagaa acgcaatatt cttttcacca 11160
   catccacgtg caaaaaaatc tacaattgct gcagcaaaat taattttaga tgcagctgtt 11220
   aaagcaggag cacctaaaaa tataataggc tggatagatg agccatcaat agaactttct 11280
   caagatttga tgagtgaagc tgatataata ttagcaacag gaggtccttc aatggttaaa 11340
   gcggcctatt catctggaaa acctgcaatt ggtgttggag caggaaatac accagcaata 11400
   atagatgaga gtgcagatat agatatggca gtaagctcca taattttatc aaagacttat 11460
   gacaatggag taatatgcgc ttctgaacaa tcaatattag ttatgaattc aatatacgaa 11520
   aaagttaaag aggaatttgt aaaacgagga tcatatatac tcaatcaaaa tgaaatagct 11580
   aaaataaaag aaactatgtt taaaaatgga gctattaatg ctgacatagt tggaaaatct 11640
   gcttatataa ttgctaaaat ggcaggaatt gaagttcctc aaactacaaa gatacttata 11700
   ggcgaagtac aatctgttga aaaaagcgag ctgttctcac atgaaaaact atcaccagta 11760
   cttgcaatgt ataaagttaa ggattttgat gaagctctaa aaaaggcaca aaggctaata 11820
   gaattaggtg gaagtggaca cacgtcatct ttatatatag attcacaaaa caataaggat 11880
   aaagttaaag aatttggatt agcaatgaaa acttcaagga catttattaa catgccttct 11940
   tcacagggag caagcggaga tttatacaat tttgcgatag caccatcatt tactcttgga 12000
   tgcggcactt ggggaggaaa ctctgtatcg caaaatgtag agcctaaaca tttattaaat 12060
   attaaaagtg ttgctgaaag aagggaaaat atgctttggt ttaaagtgcc acaaaaaata 12120
   tattttaaat atggatgtct tagatttgca ttaaaagaat taaaagatat gaataagaaa 12180
   agagccttta tagtaacaga taaagatctt tttaaacttg gatatgttaa taaaataaca 12240
   aaggtactag atgagataga tattaaatac agtatattta cagatattaa atctgatcca 12300
   actattgatt cagtaaaaaa aggtgctaaa gaaatgctta actttgaacc tgatactata 12360
   atctctattg gtggtggatc gccaatggat gcagcaaagg ttatgcactt gttatatgaa 12420
   tatccagaag cagaaattga aaatctagct ataaacttta tggatataag aaagagaata 12480
   tgcaatttcc ctaaattagg tacaaaggcg atttcagtag ctattcctac aactgctggt 12540
   accggttcag aggcaacacc ttttgcagtt ataactaatg atgaaacagg aatgaaatac 12600
   cctttaactt cttatgaatt gaccccaaac atggcaataa tagatactga attaatgtta 12660
   aatatgccta gaaaattaac agcagcaact ggaatagatg cattagttca tgctatagaa 12720
   gcatatgttt cggttatggc tacggattat actgatgaat tagccttaag agcaataaaa 12780
   atgatattta aatatttgcc tagagcctat aaaaatggga ctaacgacat tgaagcaaga 12840
   gaaaaaatgg cacatgcctc taatattgcg gggatggcat ttgcaaatgc tttcttaggt 12900
   gtatgccatt caatggctca taaacttggg gcaatgcatc acgttccaca tggaattgct 12960
   tgtgctgtat taatagaaga agttattaaa tataacgcta cagactgtcc aacaaagcaa 13020
   acagcattcc ctcaatataa atctcctaat gctaagagaa aatatgctga aattgcagag 13080
   tatttgaatt taaagggtac tagcgatacc gaaaaggtaa cagccttaat agaagctatt 13140
   tcaaagttaa agatagattt gagtattcca caaaatataa gtgccgctgg aataaataaa 13200
   aaagattttt ataatacgct agataaaatg tcagagcttg cttttgatga ccaatgtaca 13260
   acagctaatc ctaggtatcc acttataagt gaacttaagg atatctatat aaaatcattt 13320
   taacccgggc cccgggaacc cagctttctt gtacaaagtg gtgatctcga gaccttaagt 13380
   aataaaaata agagttacct taaatggtaa ctcttatttt tttaatgtcg actcatagaa 13440
   ttcgtaatca tggtcatagc tgtttcctgt gtgaaattgt tatccgctca caattccaca 13500
   caacatacga gccggaagca taaagtgtaa agcctggggt gcctaatgag tgagctaact 13560
   cacattaatt gcgttgcgct cactgcccgc tttccagtcg ggaaacctgt cgtgccagct 13620
   gcattaatga atcggccaac gcgcggggag aggcggtttg cgtattgggc gctcttccgc 13680
   ttcctcgctc actgactcgc tgcgctcggt cgttcggctg cggcgagcgg tatcagctca 13740
   ctcaaaggcg gtaatacggt tatccacaga atcaggggat aacgcaggaa agaacatgtg 13800
   agcaaaaggc cagcaaaagg ccaggaaccg taaaaaggcc gcgttgctgg cgtttttcca 13860
   taggctccgc ccccctgacg agcatcacaa aaatcgacgc tcaagtcaga ggtggcgaaa 13920
   cccgacagga ctataaagat accaggcgtt tccccctgga agctccctcg tgcgctctcc 13980
   tgttccgacc ctgccgctta ccggatacct gtccgccttt ctcccttcgg gaagcgtggc 14040
   gctttctcat agctcacgct gtaggtatct cagttcggtg taggtcgttc gctccaagct 14100
   gggctgtgtg cacgaacccc ccgttcagcc cgaccgctgc gccttatccg gtaactatcg 14160
   tcttgagtcc aacccggtaa gacacgactt atcgccactg gcagcagcca ctggtaacag 14220
   gattagcaga gcgaggtatg taggcggtgc tacagagttc ttgaagtggt ggcctaacta 14280
   cggctacact agaagaacag tatttggtat ctgcgctctg ctgaagccag ttaccttcgg 14340
   aaaaagagtt ggtagctctt gatccggcaa acaaaccacc gctggtagcg gtggtttttt 14400
   tgtttgcaag cagcagatta cgcgcagaaa aaaaggatct caagaagatc ctttgatctt 14460
   ttctacgggg tctgacgctc agtggaacga aaactcacgt taagggattt tggtcatgag 14520
   attatcaaaa aggatcttca cctagatcct tttaaattaa aaatgaagtt ttaaatcaat 14580
   ctaaagtata tatgagtaaa cttggtctga cagttaccaa tgcttaatca gtgaggcacc 14640
   tatctcagcg atctgtctat ttcgttcatc catagttgcc tgactccccg tcgtgtagat  14700
   aactacgata cgggagggct taccatctgg ccccagtgct gcaatgatac cgcgagaccc  14760
   acgctcaccg gctccagatt tatcagcaat aaaccagcca gccggaaggg ccgagcgcag  14820
   aagtggtcct gcaactttat ccgcctccat ccagtctatt aattgttgcc gggaagctag  14880
   agtaagtagt tcgccagtta atagtttgcg caacgttgtt gccattgcta caggcatcgt  14940
   ggtgtcacgc tcgtcgtttg gtatggcttc attcagctcc ggttcccaac gatcaaggcg  15000
   agttacatga tcccccatgt tgtgcaaaaa agcggttagc tccttcggtc ctccgatcgt  15060
   tgtcagaagt aagttggccg cagtgttatc actcatggtt atggcagcac tgcataattc  15120
   tcttactgtc atgccatccg taagatgctt ttctgtgact ggtgagtact caaccaagtc  15180
   attctgagaa tagtgtatgc ggcgaccgag ttgctcttgc ccggcgtcaa tacgggataa  15240
   taccgcgcca catagcagaa ctttaaaagt gctcatcatt ggaaaacgtt cttcggggcg  15300
   aaaactctca aggatcttac cgctgttgag atccagttcg atgtaaccca ctcgtgcacc  15360
   caactgatct tcagcatctt ttactttcac cagcgtttct gggtgagcaa aaacaggaag  15420
   gcaaaatgcc gcaaaaaagg gaataagggc gacacggaaa tgttgaatac tcatactctt  15480
   cctttttcaa tattattgaa gcatttatca gggttattgt ctcatgagcg gatacatatt  15540
   tgaatgtatt tagaaaaata aacaaatagg ggttccgcgc acatttcccc gaaaagtgcc  15600
   acctgacgtc taagaaacca ttattatcat gacattaacc tataaaaata ggcgtatcac  15660
   gaggcccttt cgtc                                                    15674
<210> 3
   <211> 15547
   <212> DNA
   <213> Artificial
<220>
   <223> Clostridium/E. coli shuttle vector pM9_SUC(op)
<400> 3
   acaggttggc tgataagtcc ccggtctgaa gttcctattc tctagaaagt ataggaactt     60
   cttccgacgc ttattcgctt cgctcatatt tatatagaaa agaagtgaat gcgcaaaaga    120
   cataatcgat tcacaaaaaa taggtacacg aaaaacaagt taagggatgc agtttatgca    180
   tcccttaact tacttattaa ataatttata gctattgaaa agagataaga attgttcaaa    240
   gctaatattg tttaaatcgt caattcctgc atgttttaag gaattgttaa attgattttt    300
   tgtaaatatt ttcttgtatt ctttgttaac ccatttcata acgaaataat tatacttctg    360
   tttatctttg tgtgatattc ttgatttttt tctatttaat ctgataagtg agctattcac    420
   tttaggttta ggatgaaaat attctcttgg aaccatactt aatatagaaa tatcaacttc    480
   tgccattaaa aataatgcca atgagcgttt tgtatttaat aatcttttag caaacccgta    540
   ttccacgatt aaataaatct catcagctat actatcaaaa acaattttgc gtattatatc    600
   cgtacttatg ttataaggta tattaccaaa tattttatag gattggtttt taggaaattt    660
   aaactgcaat atatccttgt ttaaaacttg gaaattatcg tgatcaacaa gtttattttc    720
   tgtagttttg cataatttat ggtctatttc aatggcagtt acgaaattac acctctgtac    780
   taattcaagg gtaaaatgcc cttttcctga gccgatttca aagatattat catgttcatt    840
   taatcttata tttgtcatta ttttatctat attatgtttt gaagtaataa agttttgact    900
   gtgttttata tttttctcgt tcattataac cctctttatt ttttcctcct tataaaatta    960
   gtataattat agcacgagct ctgataaata tgaacatgat gagtgatcgt taaatttata   1020
   ctgcaatctg atgcgattat tgaagttcct attctctaga aagtatagga acttcacggc   1080
   agtgacggtt tccctctaga tcacaagttt gtacaaaaaa gcaggcttaa ggctcctaag   1140
   tgaaatatat acatattata acaataaaat aagtattagt gtaggatttt taaatagagt   1200
   atctattttc agattaaatt tttgcttatt tgatttacat tatataatat tgagtaaagt   1260
   attgactagc aaaatttttt gatactttaa tttgtgaaat ttcttatcaa aagttatatt   1320
   tttgaatgat ttttattgaa aaatacaact aaaaaggatt atagtataag tgtgtgtaat   1380
   tttgtgttaa atttaaggca gcgttccagg agggacagaa caatgaactt acatgaatat   1440
   caggcaaaac aactttttgc ccgctatggc ttaccagcac cggtgggtta tgcctgtact   1500
   actccgcgcg aagcagaaga agccgcttca aaaatcggtg ccggtccgtg ggtagtgaaa   1560
   tgtcaggttc acgctggtgg ccgcggtaaa gcgggcggtg tgaaagttgt aaacagcaaa   1620
   gaagacatcc gtgcttttgc agaaaactgg ctgggcaagc gtctggtaac gtatcaaaca   1680
   gatgccaatg gccaaccggt taaccagatt ctggttgaag cagcgaccga tatcgctaaa   1740
   gagctgtatc tcggtgccgt tgttgaccgt agttcccgtc gtgtggtctt tatggcctcc   1800
   accgaaggcg gcgtggaaat cgaaaaagtg gcggaagaaa ctccgcacct gatccataaa   1860
   gttgcgcttg atccgctgac tggcccgatg ccgtatcagg gacgcgagct ggcgttcaaa   1920
   ctgggtctgg aaggtaaact ggttcagcag ttcaccaaaa tcttcatggg cctggcgacc   1980
   attttcctgg agcgcgacct ggcgttgatc gaaatcaacc cgctggtcat caccaaacag   2040
   ggcgatctga tttgcctcga cggcaaactg ggcgctgacg gcaacgcact gttccgccag   2100
   cctgatctgc gcgaaatgcg tgaccagtcg caggaagatc cgcgtgaagc acaggctgca   2160
   cagtgggaac tgaactacgt tgcgctggac ggtaacatcg gttgtatggt taacggcgca   2220
   ggtctggcga tgggtacgat ggacatcgtt aaactgcacg gcggcgaacc ggctaacttc   2280
   cttgacgttg gcggcggcgc aaccaaagaa cgtgtaaccg aagcgttcaa aatcatcctc   2340
   tctgacgaca aagtgaaagc cgttctggtt aacatcttcg gcggtatcgt tcgttgcgac   2400
   ctgatcgctg acggtatcat cggcgcggta gcagaagtgg gtgttaacgt accggtcgtg 2460
   gtacgtctgg aaggtaacaa cgccgaactc ggcgcgaaga aactggctga cagcggcctg 2520
   aatattattg cagcaaaagg tctgacggat gcagctcagc aggttgttgc cgcagtggag 2580
   gggaaataac aaggcaaagt gaggaggtaa ggtatgagta atgaagtatc tataaaagaa 2640
   ttaattgaaa aggcaaaggt ggcacaaaaa aaattggaag cctatagtca agaacaagtt 2700
   gatgtactag taaaagcact aggaaaagtg gtttatgata atgcagaaat gtttgcaaaa 2760
   gaagcagttg aagaaacaga aatgggtgtt tatgaagata aagtagctaa atgtcatttg 2820
   aaatcaggag ctatttggaa tcatataaaa gacaagaaaa ctgtaggcat aataaaagaa 2880
   gaacctgaaa gggcacttgt ttatgttgct aagccaaagg gagttgtggc agctactacg 2940
   cctataacta atccagtggt aactcctatg tgtaatgcaa tggctgctat aaagggcaga 3000
   aatacaataa tagtagcacc acatcctaaa gcaaagaaag tttcagctca tactgtagaa 3060
   cttatgaatg ctgagcttaa aaaattggga gcaccagaaa atatcataca gatagtagaa 3120
   gcaccatcaa gagaagctgc taaggaactt atggaaagtg ctgatgtagt tattgctaca 3180
   ggcggtgctg gaagagttaa agctgcttac tccagtggaa gaccagctta tggcgttgga 3240
   cctggaaatt cacaggtaat agttgataag ggatacgatt ataacaaagc tgcacaggat 3300
   ataataacag gaagaaaata tgacaatgga attatatgtt cttcagagca atcagttata 3360
   gctcctgctg aagattatga taaggtaata gcagcttttg tagaaaatgg ggcattctat 3420
   gtagaagatg aggaaacagt agaaaagttt agatcaactt tatttaaaga tggaaaaata 3480
   aacagcaaga ttataggtaa atccgtccaa attattgcgg atcttgcagg agtaaaagta 3540
   ccagaaggta ctaaggttat agtacttaag ggtaaaggtg caggagaaaa agatgtactt 3600
   tgtaaagaaa aaatgtgtcc agttttagta gcattgaaat atgatacttt tgaagaagca 3660
   gttgaaatag ctatggctaa ttatatgtat gaaggagctg gtcatacagc aggcatacat 3720
   tctgacaatg acgagaacat aagatatgca ggaactgtat tacctataag cagattagtt 3780
   gtaaatcagc ctgcaactac tgctggagga agtttcaata atggatttaa ccctactact 3840
   acactaggct gcggatcatg ggcagaaaac agtatttcag aaaatcttac ttacgagcat 3900
   cttataaatg tttcaagaat agggtatttc aataaagaag caaaagttcc tagctatgag 3960
   gaaatatggg gataaaagag gaggttaggc atgcagctgt ttaaactcaa gagtgtaaca 4020
   catcatttcg atacttttgc agaatttgct aaagagttct gtttaggaga aagagatctt 4080
   gtgatcacta acgaatttat ctacgaacca tatatgaaag cttgtcaatt accttgtcac 4140
   tttgttatgc aggaaaagta tggtcaaggt gaacctagtg atgaaatgat gaacaatata 4200
   ctcgcagaca ttcgtaatat tcagtttgat agagtcatag gaataggagg aggtactgtg 4260
   atagacatca gtaagctatt tgttcttaaa ggtctcaatg acgttttaga tgcatttgat    4320
   agaaaaatcc ccttaatcaa agagaaggaa ctcatcatag ttcctacaac atgtggtaca    4380
   ggaagtgaag taacgaatat aagcatagct gaaatcaaat caagacacac taaaatgggt    4440
   ttagctgatg atgcaatagt agcagatcat gcgattataa tacccgaact acttaaatct    4500
   ctcccttttc atttttatgc ttgtagtgct attgatgcat tgattcatgc aattgagtct    4560
   tatgtaagcc ctaaagcaag tccatatagc agattattca gtgaagcagc atgggatata    4620
   atactcgagg tttttaaaaa gattgctgaa catggaccag aatataggtt tgagaagtta    4680
   ggagagatga taatggcttc gaattatgca ggtattgcat ttggaaatgc tggtgttggt    4740
   gctgtacatg cattatctta ccctttaggt ggaaattatc atgttccaca tggagaagca    4800
   aactatcaat tttttaccga agtttttaaa gtttatcaaa agaaaaatcc gtttggatat    4860
   atcgttgagt taaactggaa actttccaaa attctcaatt gtcaacctga atacgtatat    4920
   cctaagctag atgaacttct cggttgtctt ttgaccaaaa aacccttaca tgaatatgga    4980
   atgaaagatg aagaagttag aggatttgct gaatctgtgt taaagacgca acaaagatta    5040
   ttggctaaca attatgtcga acttacagta gatgagatag aagggattta taggagactt    5100
   tactagagct tccgcggagg ccggcgcggc cgatttaaat cctcagcctg agcccccggg    5160
   cggtatcagc tcactcaaag gcggtaataa caactttgta tacaaaagtt gtagtgtagt    5220
   agcctgtgaa ataagtaagg aaaaaaaaga agtaagtgtt atatatgatg attattttgt    5280
   agatgtagat aggataatag aatccataga aaatataggt tatacagtta tataaaaatt    5340
   actttaaaaa ttaataaaaa catggtaaaa tataaatcgt ataaagttgt gtaattttta    5400
   aggaggtgtg ttacatatgc agtggcaaga actgtatcgc cagagagtgt gctcagcgga    5460
   tgaagccgta gtggatagcc tgaaaccggg aacgaaagtg gtgtttggtc atgcagctgc    5520
   ggcccctgta agattttctc aggctatgta tcgccaaaga gaaaaactgg aaaatattac    5580
   agtgtttcat atgctttatt ttggcgatgc ccctcacctt gcaccggaaa tgcgtagcca    5640
   tgttcaccct actcttaatt ttcttgaggg taattctcgt ccggccagcc gcgatagacg    5700
   tgtggatttt ataccgtgtc attttcacga agttccagaa ctgtttcgcc agggtttctt    5760
   tccattagat gttgcagttg tacaagtatc tacccctaat gaagaaggat attgcagttt    5820
   tggcgtttca tgcgattata cgaaagcagc tgcggaatgc gccccggtgg ttgtagcaga    5880
   agtaaataaa cagatgccat ttattggtgg agaaaatctg attcatattt caaaacttac    5940
   ccacataatt gaagtagatg aaccgatagc tgaagtgctt ccgccagcga ttagtgatct    6000
   ggaattacgt attggtcaga attgtgcatc actgattaaa gatggcgata cacttcaact    6060
   gggtattggc ggtataccag atgctgtgct gcgcgcgtta gaaggtcata aagatttagg    6120
   aattcacacc gaaatgttta cggatggagt aatgcgcatg ataagaaaag gcataattaa    6180
   tggtaaaaag aaaacacttc atccagaaaa agtggttacg tctcttattt ttggcagcaa 6240
   agaactgtat gattttgtga ataataatcc agttattgaa tgttatcctg tggattatat 6300
   aaataatcct gatgttatag gaaaaaatga tcgtatggta tctattaatt catgccttga 6360
   aatggatctg atgggccagg ccgcaagtga atcaatagga tatgaacagt tttctggcag 6420
   cggaggccaa gttgattttc tgagaggtgc taaacgtagt aaaggtggaa tatcaattat 6480
   ggcttttcct tctacagcaa agaaaggtac tgaaagccgc attgttccga tattaaaaga 6540
   aggtgcttgc gttacaactg gaagaaatga agtagattat gtagtgactg aatatggcgt 6600
   agcccgttta cgcggtgcaa cacttagaca acgtgctgaa gcgttaactg ctatagcaca 6660
   tccagatttt agaccagcat tagaagagga aatccgccga cgcttcgaat aaggcatgca 6720
   cggtagccag gaggttaatg aaagttacaa atcaaaaaga actaaaacaa aagctaaatg 6780
   aattgagaga agcgcaaaag aagtttgcaa cctatactca agagcaagtt gataaaattt 6840
   ttaaacaatg tgccatagcc gcagctaaag aaagaataaa cttagctaaa ttagcagtag 6900
   aagaaacagg aataggtctt gtagaagata aaattataaa aaatcatttt gcagcagaat 6960
   atatatacaa taaatataaa aatgaaaaaa cttgtggcat aatagaccat gacgattctt 7020
   taggcataac aaaggttgct gaaccaattg gaattgttgc agccatagtt cctactacta 7080
   atccaacttc cacagcaatt ttcaaatcat taatttcttt aaaaacaaga aacgcaatat 7140
   tcttttcacc acatccacgt gcaaaaaaat ctacaattgc tgcagcaaaa ttaattttag 7200
   atgcagctgt taaagcagga gcacctaaaa atataatagg ctggatagat gagccatcaa 7260
   tagaactttc tcaagatttg atgagtgaag ctgatataat attagcaaca ggaggtcctt 7320
   caatggttaa agcggcctat tcatctggaa aacctgcaat tggtgttgga gcaggaaata 7380
   caccagcaat aatagatgag agtgcagata tagatatggc agtaagctcc ataattttat 7440
   caaagactta tgacaatgga gtaatatgcg cttctgaaca atcaatatta gttatgaatt 7500
   caatatacga aaaagttaaa gaggaatttg taaaacgagg atcatatata ctcaatcaaa 7560
   atgaaatagc taaaataaaa gaaactatgt ttaaaaatgg agctattaat gctgacatag 7620
   ttggaaaatc tgcttatata attgctaaaa tggcaggaat tgaagttcct caaactacaa 7680
   agatacttat aggcgaagta caatctgttg aaaaaagcga gctgttctca catgaaaaac 7740
   tatcaccagt acttgcaatg tataaagtta aggattttga tgaagctcta aaaaaggcac 7800
   aaaggctaat agaattaggt ggaagtggac acacgtcatc tttatatata gattcacaaa 7860
   acaataagga taaagttaaa gaatttggat tagcaatgaa aacttcaagg acatttatta 7920
   acatgccttc ttcacaggga gcaagcggag atttatacaa ttttgcgata gcaccatcat 7980
   ttactcttgg atgcggcact tggggaggaa actctgtatc gcaaaatgta gagcctaaac 8040
   atttattaaa tattaaaagt gttgctgaaa gaagggaaaa tatgctttgg tttaaagtgc 8100
   cacaaaaaat atattttaaa tatggatgtc ttagatttgc attaaaagaa ttaaaagata 8160
   tgaataagaa aagagccttt atagtaacag ataaagatct ttttaaactt ggatatgtta 8220
   ataaaataac aaaggtacta gatgagatag atattaaata cagtatattt acagatatta 8280
   aatctgatcc aactattgat tcagtaaaaa aaggtgctaa agaaatgctt aactttgaac 8340
   ctgatactat aatctctatt ggtggtggat cgccaatgga tgcagcaaag gttatgcact 8400
   tgttatatga atatccagaa gcagaaattg aaaatctagc tataaacttt atggatataa 8460
   gaaagagaat atgcaatttc cctaaattag gtacaaaggc gatttcagta gctattccta 8520
   caactgctgg taccggttca gaggcaacac cttttgcagt tataactaat gatgaaacag 8580
   gaatgaaata ccctttaact tcttatgaat tgaccccaaa catggcaata atagatactg 8640
   aattaatgtt aaatatgcct agaaaattaa cagcagcaac tggaatagat gcattagttc 8700
   atgctataga agcatatgtt tcggttatgg ctacggatta tactgatgaa ttagccttaa 8760
   gagcaataaa aatgatattt aaatatttgc ctagagccta taaaaatggg actaacgaca 8820
   ttgaagcaag agaaaaaatg gcacatgcct ctaatattgc ggggatggca tttgcaaatg 8880
   ctttcttagg tgtatgccat tcaatggctc ataaacttgg ggcaatgcat cacgttccac 8940
   atggaattgc ttgtgctgta ttaatagaag aagttattaa atataacgct acagactgtc 9000
   caacaaagca aacagcattc cctcaatata aatctcctaa tgctaagaga aaatatgctg 9060
   aaattgcaga gtatttgaat ttaaagggta ctagcgatac cgaaaaggta acagccttaa 9120
   tagaagctat ttcaaagtta aagatagatt tgagtattcc acaaaatata agtgccgctg 9180
   gaataaataa aaaagatttt tataatacgc tagataaaat gtcagagctt gcttttgatg 9240
   accaatgtac aacagctaat cctaggtatc cacttataag tgaacttaag gatatctata 9300
   taaaatcatt ttaacccggg ccccgggaac ccagctttct tgtacaaagt ggtgatgatc 9360
   cggctgctaa cctcgagaac atgtgagcaa taaaaataag aagcctgcat ttgcaggctt 9420
   cttattttta tagaccgggg acttatcagc caacctgtag gccagcaaaa ggccaggaac 9480
   cgtaaaaagg ccgcgttgct ggcgtttttc cataggctcc gcccccctga cgagcatcac 9540
   aaaaatcgac gctcaagtca gaggtggcga aacccgacag gactataaag ataccaggcg 9600
   tttccccctg gaagctccct cgtgcgctct cctgttccga ccctgccgct taccggatac 9660
   ctgtccgcct ttctcccttc gggaagcgtg gcgctttctc atagctcacg ctgtaggtat 9720
   ctcagttcgg tgtaggtcgt tcgctccaag ctgggctgtg tgcacgaacc ccccgttcag 9780
   cccgaccgct gcgccttatc cggtaactat cgtcttgagt ccaacccggt aagacacgac 9840
   ttatcgccac tggcagcagc cactggtaac aggattagca gagcgaggta tgtaggcggt 9900
   gctacagagt tcttgaagtg gtggcctaac tacggctaca ctagaagaac agtatttggt 9960
   atctgcgctc tgctgaagcc agttaccttc ggaaaaagag ttggtagctc ttgatccggc 10020
   aaacaaacca ccgctggtag cggtggtttt tttgtttgca agcagcagat tacgcgcaga 10080
   aaaaaaggat ctcaagaaga tcctttgatc ttttctacgg ggtctgacgc tcagtggaac 10140
   gaaaactcac gttaagggat tttggtcatg agattatcaa aaaggatctt cacctagatc 10200
   cttttaaatt aaaaatgaag ttttaaatca atctaaagta tatatgagta aacttggtct 10260
   gacagttacc aatgcttaat cagtgaggca cctatctcag cgatctgtct atttcgttca 10320
   tccatagttg cctgactccc cgtcgtgtag ataactacga tacgggaggg cttaccatct 10380
   ggccccagtg ctgcaatgat accgcgagac ccacgctcac cggctccaga tttatcagca 10440
   ataaaccagc cagccggaag ggccgagcgc agaagtggtc ctgcaacttt atccgcctcc 10500
   atccagtcta ttaattgttg ccgggaagct agagtaagta gttcgccagt taatagtttg 10560
   cgcaacgttg ttgccattgc tacaggcatc gtggtgtcac gctcgtcgtt tggtatggct 10620
   tcattcagct ccggttccca acgatcaagg cgagttacat gatcccccat gttgtgcaaa 10680
   aaagcggtta gctccttcgg tcctccgatc gttgtcagaa gtaagttggc cgcagtgtta 10740
   tcactcatgg ttatggcagc actgcataat tctcttactg tcatgccatc cgtaagatgc 10800
   ttttctgtga ctggtgagta ctcaaccaag tcattctgag aatagtgtat gcggcgaccg 10860
   agttgctctt gcccggcgtc aatacgggat aataccgcgc cacatagcag aactttaaaa 10920
   gtgctcatca ttggaaaacg ttcttcgggg cgaaaactct caaggatctt accgctgttg 10980
   agatccagtt cgatgtaacc cactcgtgca cccaactgat cttcagcatc ttttactttc 11040
   accagcgttt ctgggtgagc aaaaacagga aggcaaaatg ccgcaaaaaa gggaataagg 11100
   gcgacacgga aatgttgaat actcatactc ttcctttttc aatattattg aagcatttat 11160
   cagggttatt gtctcatgag cggatacata tttgaatgta tttagaaaaa taaacaaata 11220
   ggggttccgc gcacatttcc ccgaaaagtg ccacctgacg gtcctgcagg taatacgact 11280
   cactataggg ataacttaaa tttatatttt ttactttata atatataatt gattatagaa 11340
   tccaaattag ataggaggac caggtaaatg gagaataatt ttattgtcaa cgaaaacgag 11400
   aaaagggttt taaagcagat atttaacaac tccaatatca gtagaactca aatttccaag 11460
   aatttagaac tgaataaagc aacaatttcg aacattctta acaatctaaa gcataaaagt 11520
   ttggtaaatg aagttggtga aggaaattct actaaaagtg gtggaagaaa acctatactt 11580
   ctcgaaatta atcaaaagta tgggtattat ataagcatgg atttgacata tgattctgtt 11640
   gagttaatgt ataactattt tgatgctaca attctcaaac aagatagtta tgaactgaat 11700
   gataagaatg taagtagcat attacagata ctcaaatcta acataaatgt tagcgaaaaa 11760
   tacgacacat tatacggttt attagggata agtatatcca tacatgggat tgtagatgat 11820
   gaacaaaaca tcattaatct accatttcac aagaatgaaa aaagaacgtt tactgatgaa 11880
   cttaagtctt tcactaatgt accagtggtt attgaaaatg aggcaaattt aagtgcatta 11940
   tacgagaaaa gtttatacat caattcgaat attaacaact tgatcacatt gtctatacat 12000
   aaaggtatag gagctggcat cataatcaat aagaaactct atagaggtag taatggagaa 12060
   gctggagaaa taggaaagac tcttgtttta gagtctatca ataacaatga taacaagtac 12120
   tataaaatag aggatatttg tagtcaagac gctttgatac aaaagattaa taacagatta 12180
   ggagttacac ttacatttac cgaactaata cagtattata acgaaggaaa tagtattgta 12240
   gctcatgaaa tcaaacagtt tatcaacaaa atgactgtct taatacacaa tcttaatact 12300
   cagtttaatc cagatgcaat atacatcaat tgtcccttga ttaatgaact tcctaatatt 12360
   ctaaacgaaa tcaaagaaca attctcttgt ttttcacaag gaagtcctat acaattgcat 12420
   ttaaccacta atgttaaaca agctacatta ttaggtggaa cattagcaat tatgcagaaa 12480
   acccttaaca taaacaatat ccaaatgaat attaaatagt tacagcagtc tgagttataa 12540
   aatagatatc tcggaccgtc tattcttcct tatttgagtg ggtaccaatg aaatctttcg 12600
   agctaaatcg atttctggcc cactcttttt tattttttag taagcgttta caaaaaatga 12660
   acaatgtgct atattacctc taaagttagt ttgtttatta aattaaccaa ctaaaatgtc 12720
   gggatccaat taggaggaat ctgcgatgga aaagaaggaa tttagagttt taataaagta 12780
   ttgttttctt aaaggaaaga atacagtaga agctaagact tggttagata atgaatttcc 12840
   agattctgct cctggtaaaa gtacaattat agattggtat gcaaaattta aaagaggaga 12900
   aatgtcaaca gaagatggag aaagatctgg tagaccaaaa gaagttgtaa ctgatgaaaa 12960
   tataaagaag atacataaaa tgatattaaa tgatagaaag atgaagttaa tagaaatagc 13020
   agaagcttta aaaatatcta aagaaagagt tggacatata atacatcaat atttagatat 13080
   gagaaaatta tgtgcaaaat gggttccaag agaattaact tttgatcaaa agcaaagaag 13140
   agtagatgat agtaagagat gtttacaatt attaacaaga aatactccag aatttttcag 13200
   aagatatgta acaatggatg aaacttggtt acatcattat acacctgaaa gtaatagaca 13260
   atcagcagaa tggacagcta ctggagaacc atcacctaaa agaggaaaga ctcaaaagag 13320
   tgctggaaag gttatggcat cagtattttg ggatgctcat ggtataatat ttatagatta 13380
   tttagaaaaa ggaaaaacaa taaattctga ttattatatg gcattattag aaagattaaa 13440
   agttgaaata gcagctaaaa gaccacatat gaagaagaaa aaagtattat ttcatcaaga 13500
   taatgctcct tgtcataagt cattaagaac tatggcaaag atacatgaat taggttttga 13560
   attattacca catccacctt attcaccaga tttagctcct tctgatttct tttatattag 13620
   tgatttaaag agaatgttag caggaaagaa gtttggttgt aatgaagaag ttatagcaga 13680
   aacagaagct tattttgaag caaaacctaa ggaatattat caaaatggaa taaagaagtt 13740
   agaaggtaga tataatagat gtatagcatt agaaggaaat tatgtagaat aagaattcct 13800
   taattaagga gctcgataaa aataagaagc ctgcatttgc aggcttctta tttttatggt 13860
   catagctgtt tcctggcgcg ccgcattcac ttcttttcta tataaatatg agcgaagcga 13920
   ataagcgtcg gaaaagcagc aaaaagtttc ctttttgctg ttggagcatg ggggttcagg 13980
   gggtgcagta tctgacgtca atgccgagcg aaagcgagcc gaagggtagc atttacgtta 14040
   gataaccccc tgatatgctc cgacgcttta tatagaaaag aagattcaac taggtaaaat 14100
   cttaatatag gttgagatga taaggtttat aaggaatttg tttgttctaa tttttcactc 14160
   attttgttct aatttctttt aacaaatgtt cttttttttt tagaacagtt atgatatagt 14220
   tagaatagtt taaaataagg agtgagaaaa agatgaaaga aagatatgga acagtctata 14280
   aaggctctca gaggctcata gacgaagaaa gtggagaagt catagaggta gacaagttat 14340
   accgtaaaca aacgtctggt aacttcgtaa aggcatatat agtgcaatta ataagtatgt 14400
   tagatatgat tggcggaaaa aaacttaaaa tcgttaacta tatcctagat aatgtccact 14460
   taagtaacaa tacaatgata gctacaacaa gagaaatagc aaaagctaca ggaacaagtc 14520
   tacaaacagt aataacaaca cttaaaatct tagaagaagg aaatattata aaaagaaaaa 14580
   ctggagtatt aatgttaaac cctgaactac taatgagagg cgacgaccaa aaacaaaaat 14640
   acctcttact cgaatttggg aactttgagc aagaggcaaa tgaaatagat tgacctccca 14700
   ataacaccac gtagttattg ggaggtcaat ctatgaaatg cgattaaggg ccggccagtg 14760
   ggcaagttga aaaattcaca aaaatgtggt ataatatctt tgttcattag agcgataaac 14820
   ttgaatttga gagggaactt agatggtatt tgaaaaaatt gataaaaata gttggaacag 14880
   aaaagagtat tttgaccact actttgcaag tgtaccttgt acctacagca tgaccgttaa 14940
   agtggatatc acacaaataa aggaaaaggg aatgaaacta tatcctgcaa tgctttatta 15000
   tattgcaatg attgtaaacc gccattcaga gtttaggacg gcaatcaatc aagatggtga 15060
   attggggata tatgatgaga tgataccaag ctatacaata tttcacaatg atactgaaac 15120
   attttccagc ctttggactg agtgtaagtc tgactttaaa tcatttttag cagattatga 15180
   aagtgatacg caacggtatg gaaacaatca tagaatggaa ggaaagccaa atgctccgga 15240
   aaacattttt aatgtatcta tgataccgtg gtcaaccttc gatggcttta atctgaattt 15300
   gcagaaagga tatgattatt tgattcctat ttttactatg gggaaatatt ataaagaaga 15360
   taacaaaatt atacttcctt tggcaattca agttcatcac gcagtatgtg acggatttca 15420
   catttgccgt tttgtaaacg aattgcagga attgataaat agttaacttc aggtttgtct 15480
   gtaactaaaa acaagtattt aagcaaaaac atcgtagaaa tacggtgttt tttgttaccc 15540
   taagttt 15547
<210> 4
   <211> 16625
   <212> **DNA**
   <213> Artificial
<220>
   <223> Clostridium/E. coli shuttle vector pM9_AKG
<400> 4
   acaggttggc tgataagtcc ccggtctgaa gttcctattc tctagaaagt ataggaactt     60
   cttccgacgc ttattcgctt cgctcatatt tatatagaaa agaagtgaat gcgcaaaaga    120
   cataatcgat tcacaaaaaa taggtacacg aaaaacaagt taagggatgc agtttatgca    180
   tcccttaact tacttattaa ataatttata gctattgaaa agagataaga attgttcaaa    240
   gctaatattg tttaaatcgt caattcctgc atgttttaag gaattgttaa attgattttt    300
   tgtaaatatt ttcttgtatt ctttgttaac ccatttcata acgaaataat tatacttctg    360
   tttatctttg tgtgatattc ttgatttttt tctatttaat ctgataagtg agctattcac    420
   tttaggttta ggatgaaaat attctcttgg aaccatactt aatatagaaa tatcaacttc    480
   tgccattaaa aataatgcca atgagcgttt tgtatttaat aatcttttag caaacccgta    540
   ttccacgatt aaataaatct catcagctat actatcaaaa acaattttgc gtattatatc    600
   cgtacttatg ttataaggta tattaccaaa tattttatag gattggtttt taggaaattt    660
   aaactgcaat atatccttgt ttaaaacttg gaaattatcg tgatcaacaa gtttattttc    720
   tgtagttttg cataatttat ggtctatttc aatggcagtt acgaaattac acctctgtac    780
   taattcaagg gtaaaatgcc cttttcctga gccgatttca aagatattat catgttcatt    840
   taatcttata tttgtcatta ttttatctat attatgtttt gaagtaataa agttttgact    900
   gtgttttata tttttctcgt tcattataac cctctttatt ttttcctcct tataaaatta    960
   gtataattat agcacgagct ctgataaata tgaacatgat gagtgatcgt taaatttata   1020
   ctgcaatctg atgcgattat tgaagttcct attctctaga aagtatagga acttcacggc   1080
   agtgacggtt tccctctaga tcacaagttt gtacaaaaaa gcaggcttaa ggctcctaag   1140
   tgaaatatat acatattata acaataaaat aagtattagt gtaggatttt taaatagagt   1200
   atctattttc agattaaatt tttgcttatt tgatttacat tatataatat tgagtaaagt   1260
   attgactagc aaaatttttt gatactttaa tttgtgaaat ttcttatcaa aagttatatt   1320
   tttgaatgat ttttattgaa aaatacaact aaaaaggatt atagtataag tgtgtgtaat   1380
   tttgtgttaa atttaagcca gctgtaggag ggaagcatgt acagaaaatt tagagatgat   1440
   cctagtagtg tagatcctag ttggcatgaa tttttagttg attatagtcc tgaacctaca   1500
   tcacaacctg cagcagaacc tacaagagtt acatcacctt tagttgcaga aagagcagca   1560
   gcagcagcac ctcaagcacc tcctaaacct gcagatacag cagcagcagg aaatggagtt   1620
   gttgcagcat tagcagcaaa aacagctgtt cctcctcctg cagaaggaga tgaagttgca 1680
   gttttaagag gagcagcagc tgcagttgtt aaaaacatga gtgctagttt agaagttcct 1740
   acagcaacaa gtgttagagc agttcctgca aaattactga tagacaacag aatagttata 1800
   aacaatcaat taaaaagaac aagaggagga aaaataagtt ttacacattt attaggatat 1860
   gcattagttc aagcagttaa aaaatttcct aatatgaaca gacactatac agaagtcgat 1920
   ggaaaaccta cagcagttac acctgcacac acaaacttag gattagcgat agatctacaa 1980
   ggaaaagatg gaaaaagaag tttagtagtt gcaggtataa agagatgtga aacaatgaga 2040
   tttgcacaat ttgttacagc atatgaagat atagtacgta gagcaagaga tggaaaatta 2100
   acgacagaag attttgcagg agttacaata agtcttacaa atcctggaac aataggaaca 2160
   gttcatagtg ttcctagatt aatgccagga caaggagcaa taataggagt tggagcaatg 2220
   gaatatcctg ctgaatttca aggagcatct gaagaaagaa tagctgaatt aggtattggg 2280
   aaattaataa ccttaacatc tacatatgat catagaataa tacaaggagc agaaagtgga 2340
   gattttttaa gaacgataca tgaattactg ctcagtgatg gattttggga tgaagtgttt 2400
   agagaattgt ccataccata tttacctgtt agatggtcta cagataatcc tgatagtata 2460
   gttgataaaa atgcaagagt tatgaattta atagcagcat atagaaatag aggacattta 2520
   atggcagata cagatccctt acgattagat aaagcaagat ttagaagtca tcctgattta 2580
   gaagttttaa cacatggatt aacactttgg gatttagata gagttttcaa agttgatgga 2640
   tttgcaggag cacaatataa gaaactcaga gatgtcttag gattgttaag agatgcatat 2700
   tgtcgccata taggagttga atatgcacat atcttagatc ctgaacaaaa ggaatggtta 2760
   gaacaaagag ttgagactaa acatgtcaaa cctacagttg cacaacaaaa gtatatctta 2820
   agcaaattaa atgcagctga agcatttgaa acatttttac agacaaaata tgttggacag 2880
   aaaagattta gtttagaagg agcagaaagt gttataccta tgatggatgc agcaatagat 2940
   caatgtgcag aacatggatt agatgaagta gttataggaa tgcctcatag aggaagacta 3000
   aatgttctag caaatatagt tgggaaaccg tatagtcaaa tattcacaga attcgaagga 3060
   aatctgaatc caagtcaagc acatggaagt ggagatgtta aatatcattt aggagcaaca 3120
   ggattatact tacaaatgtt tggagataac gatatacaag tttctttaac agcaaatcct 3180
   agtcatttag aagcagttga ccctgtttta gaaggattag ttagagcaaa acaagattta 3240
   ctcgatcatg gaagtatcga tagtgatggc caaagagcat tttcagttgt tccgttaatg 3300
   ttacatggag atgcagcatt tgcaggacaa ggagttgttg cagaaacact gaatttagca 3360
   aatttacctg gatatagagt tggaggaaca atacacatca tagttaataa tcagatagga 3420
   tttacaacag cacctgaata tagtcgtagt agtgaatatt gtacagatgt tgcaaaaatg 3480
   ataggagctc ctatatttca tgttaatgga gatgatcctg aagcatgtgt ttgggttgca 3540
   agattagcag ttgattttag acaaagattt aagaaagatg ttgttataga catgctatgc 3600
   tatagaagaa gaggacataa cgaaggagat gatcctagta tgacaaatcc ttacatgtat 3660
   gacgttgttg atacgaaaag aggagctaga aagagttata cagaagcatt aataggaaga 3720
   ggagatataa gtatgaaaga agcagaagat gcattaaggg attatcaagg acaattagaa 3780
   agagttttca atgaagttag agaattagaa aaacatggcg ttcaacctag tgaaagtgta 3840
   gaaagtgacc aaatgattcc tgcaggatta gctacagcag ttgataaaag tctgttagca 3900
   agaataggag atgcattttt agcattacct aatggattta ctgcacatcc tagagttcaa 3960
   cctgtgttag aaaagagaag agaaatggca tatgagggaa aaatagattg ggcatttgga 4020
   gaactattag cattagggag tttagttgca gaaggaaaat tagtgcgctt aagtggacaa 4080
   gatagtagaa gaggtacctt cagtcaaaga cacagtgttt taatagacag acatacaggt 4140
   gaagagttta caccgttaca attgttagca acaaatagtg atggaagtcc tacaggaggg 4200
   aaatttctcg tttatgatag tcctttaagt gaatatgcag cagttggatt tgagtatgga 4260
   tatacagttg gaaatcctga tgcagttgtt ttatgggaag cacaatttgg agattttgtt 4320
   aatggagcac agagtataat cgacgaattt atatctagtg gagaggcaaa atggggacaa 4380
   ttaagcaatg tcgttttatt gttacctcat ggacatgaag gacaaggacc tgatcataca 4440
   agtgcaagaa tcgaaagatt tttacaacta tgggcagaag gaagtatgac aattgcaatg 4500
   cctagtacac cttccaatta ctttcatctt ttacgtagac atgcattaga tggaattcaa 4560
   agacccttaa tagtttttac acccaaaagt atgcttagac ataaagctgc agttagtgag 4620
   ataaaggact ttacagaaat caaatttcga agtgttttag aagagcctac ttatgaagat 4680
   ggaataggag atagaaataa agtttcaaga atattactca caagtggaaa gttatattat 4740
   gagttagcag caagaaaggc aaaagacaat agaaatgatt tagcaatagt tagattagaa 4800
   caattagcac ctttaccaag aagaagacta agagaaacat tagatagata tgagaacgtt 4860
   aaagaattct tttgggttca agaagaacct gcaaatcaag gagcatggcc tagatttggg 4920
   ttagaattgc ctgaattatt acctgataaa ctagctggaa taaaaagaat atcaagaaga 4980
   gcaatgagtg cacctagtag tggaagtagt aaagttcatg cagttgaaca acaagagata 5040
   ttagatgaag catttggata gaagaggagg ttaggcatgc agctgtttaa actcaagagt 5100
   gtaacacatc atttcgatac ttttgcagaa tttgctaaag agttctgttt aggagaaaga 5160
   gatcttgtga tcactaacga atttatctac gaaccatata tgaaagcttg tcaattacct 5220
   tgtcactttg ttatgcagga aaagtatggt caaggtgaac ctagtgatga aatgatgaac 5280
   aatatactcg cagacattcg taatattcag tttgatagag tcataggaat aggaggaggt 5340
   actgtgatag acatcagtaa gctatttgtt cttaaaggtc tcaatgacgt tttagatgca 5400
   tttgatagaa aaatcccctt aatcaaagag aaggaactca tcatagttcc tacaacatgt 5460
   ggtacaggaa gtgaagtaac gaatataagc atagctgaaa tcaaatcaag acacactaaa 5520
   atgggtttag ctgatgatgc aatagtagca gatcatgcga ttataatacc cgaactactt 5580
   aaatctctcc cttttcattt ttatgcttgt agtgctattg atgcattgat tcatgcaatt 5640
   gagtcttatg taagccctaa agcaagtcca tatagcagat tattcagtga agcagcatgg 5700
   gatataatac tcgaggtttt taaaaagatt gctgaacatg gaccagaata taggtttgag 5760
   aagttaggag agatgataat ggcttcgaat tatgcaggta ttgcatttgg aaatgctggt 5820
   gttggtgctg tacatgcatt atcttaccct ttaggtggaa attatcatgt tccacatgga 5880
   gaagcaaact atcaattttt taccgaagtt tttaaagttt atcaaaagaa aaatccgttt 5940
   ggatatatcg ttgagttaaa ctggaaactt tccaaaattc tcaattgtca acctgaatac 6000
   gtatatccta agctagatga acttctcggt tgtcttttga ccaaaaaacc cttacatgaa 6060
   tatggaatga aagatgaaga agttagagga tttgctgaat ctgtgttaaa gacgcaacaa 6120
   agattattgg ctaacaatta tgtcgaactt acagtagatg agatagaagg gatttatagg 6180
   agactttact agagcttccg cggaggccgg cgcggccgat ttaaatcctc agcctgagcc 6240
   cccgggcggt atcagctcac tcaaaggcgg taataacaac tttgtataca aaagttgtag 6300
   tgtagtagcc tgtgaaataa gtaaggaaaa aaaagaagta agtgttatat atgatgatta 6360
   ttttgtagat gtagatagga taatagaatc catagaaaat ataggttata cagttatata 6420
   aaaattactt taaaaattaa taaaaacatg gtaaaatata aatcgtataa agttgtgtaa 6480
   tttttaagga ggtgtgttac atatgcaatg gcaagaactt taccgtcagc gcgtttgctc 6540
   tgcagacgaa gctgtcgtgg actctcttaa accgggaacg aaagttgtat tcggtcatgc 6600
   tgctgctgcg cctgtccgtt tctctcaggc tatgtaccgc cagcgtgaaa agttggagaa 6660
   tatcacagtt ttccacatgt tgtatttcgg cgacgcgccg caccttgctc ccgaaatgcg 6720
   ttcgcatgta cacccgactc tcaacttcct tgagggcaac tcccgtccgg caagccgtga 6780
   ccgtcgtgtc gatttcattc cctgccactt ccacgaggta ccggaactgt ttcgtcaggg 6840
   attctttcca ttggatgtag ccgtagtgca ggtatctact cctaacgaag agggttattg 6900
   ctctttcgga gtttcctgcg actacacaaa ggctgccgcc gagtgcgctc cggtagtagt 6960
   agccgaggtg aacaagcaaa tgccattcat cggtggtgaa aacctgattc acatctccaa 7020
   actgacccat atcatcgaag tggacgagcc gattgcagaa gtattgcctc ctgctatcag 7080
   cgaccttgaa ctgaggatag gtcagaattg tgcctcactg atcaaagacg gcgataccct 7140
   ccagttgggt atcggcggta tccccgacgc tgtgttgcgt gcattggaag ggcataaaga 7200
   tctcggtatt cacacggaaa tgtttaccga cggggtgatg cgtatgattc gcaaggggat 7260
   tatcaacggg aagaaaaaaa cattgcatcc cgaaaaagtc gttacctcgc taatcttcgg 7320
   atcgaaagaa ttgtacgatt ttgtcaataa caatccggtg atagaatgct atccggtgga 7380
   ttatatcaac aaccccgatg ttatcggtaa gaatgaccgc atggtttcta tcaattcctg 7440
   cttggagatg gatctcatgg ggcaggcagc ttctgagtcg atcgggtacg aacagttcag 7500
   tggatccgga ggtcaagtcg atttccttcg tggggccaag cgttccaagg gaggaatctc 7560
   cattatggct ttccccagta cggccaagaa agggactgag agtcgcatcg ttcccattct 7620
   gaaagagggt gcttgtgtca cgaccggccg taacgaagtg gactatgtgg tgacggaata 7680
   tggcgtagcg cgtctgcgtg gcgcaacgct tcgtcagcgt gctgaagcct tgactgctat 7740
   agcacatccc gatttccgac cggcccttga ggaggaaatc cgccgacgct tcgaataagc 7800
   atgcacggta gccaggaggt taatgaaagt tacaaatcaa aaagaactaa aacaaaagct 7860
   aaatgaattg agagaagcgc aaaagaagtt tgcaacctat actcaagagc aagttgataa 7920
   aatttttaaa caatgtgcca tagccgcagc taaagaaaga ataaacttag ctaaattagc 7980
   agtagaagaa acaggaatag gtcttgtaga agataaaatt ataaaaaatc attttgcagc 8040
   agaatatata tacaataaat ataaaaatga aaaaacttgt ggcataatag accatgacga 8100
   ttctttaggc ataacaaagg ttgctgaacc aattggaatt gttgcagcca tagttcctac 8160
   tactaatcca acttccacag caattttcaa atcattaatt tctttaaaaa caagaaacgc 8220
   aatattcttt tcaccacatc cacgtgcaaa aaaatctaca attgctgcag caaaattaat 8280
   tttagatgca gctgttaaag caggagcacc taaaaatata ataggctgga tagatgagcc 8340
   atcaatagaa ctttctcaag atttgatgag tgaagctgat ataatattag caacaggagg 8400
   tccttcaatg gttaaagcgg cctattcatc tggaaaacct gcaattggtg ttggagcagg 8460
   aaatacacca gcaataatag atgagagtgc agatatagat atggcagtaa gctccataat 8520
   tttatcaaag acttatgaca atggagtaat atgcgcttct gaacaatcaa tattagttat 8580
   gaattcaata tacgaaaaag ttaaagagga atttgtaaaa cgaggatcat atatactcaa 8640
   tcaaaatgaa atagctaaaa taaaagaaac tatgtttaaa aatggagcta ttaatgctga 8700
   catagttgga aaatctgctt atataattgc taaaatggca ggaattgaag ttcctcaaac 8760
   tacaaagata cttataggcg aagtacaatc tgttgaaaaa agcgagctgt tctcacatga 8820
   aaaactatca ccagtacttg caatgtataa agttaaggat tttgatgaag ctctaaaaaa 8880
   ggcacaaagg ctaatagaat taggtggaag tggacacacg tcatctttat atatagattc 8940
   acaaaacaat aaggataaag ttaaagaatt tggattagca atgaaaactt caaggacatt 9000
   tattaacatg ccttcttcac agggagcaag cggagattta tacaattttg cgatagcacc 9060
   atcatttact cttggatgcg gcacttgggg aggaaactct gtatcgcaaa atgtagagcc 9120
   taaacattta ttaaatatta aaagtgttgc tgaaagaagg gaaaatatgc tttggtttaa 9180
   agtgccacaa aaaatatatt ttaaatatgg atgtcttaga tttgcattaa aagaattaaa 9240
   agatatgaat aagaaaagag cctttatagt aacagataaa gatcttttta aacttggata 9300
   tgttaataaa ataacaaagg tactagatga gatagatatt aaatacagta tatttacaga 9360
   tattaaatct gatccaacta ttgattcagt aaaaaaaggt gctaaagaaa tgcttaactt 9420
   tgaacctgat actataatct ctattggtgg tggatcgcca atggatgcag caaaggttat 9480
   gcacttgtta tatgaatatc cagaagcaga aattgaaaat ctagctataa actttatgga 9540
   tataagaaag agaatatgca atttccctaa attaggtaca aaggcgattt cagtagctat 9600
   tcctacaact gctggtaccg gttcagaggc aacacctttt gcagttataa ctaatgatga 9660
   aacaggaatg aaataccctt taacttctta tgaattgacc ccaaacatgg caataataga 9720
   tactgaatta atgttaaata tgcctagaaa attaacagca gcaactggaa tagatgcatt 9780
   agttcatgct atagaagcat atgtttcggt tatggctacg gattatactg atgaattagc 9840
   cttaagagca ataaaaatga tatttaaata tttgcctaga gcctataaaa atgggactaa 9900
   cgacattgaa gcaagagaaa aaatggcaca tgcctctaat attgcgggga tggcatttgc 9960
   aaatgctttc ttaggtgtat gccattcaat ggctcataaa cttggggcaa tgcatcacgt 10020
   tccacatgga attgcttgtg ctgtattaat agaagaagtt attaaatata acgctacaga 10080
   ctgtccaaca aagcaaacag cattccctca atataaatct cctaatgcta agagaaaata 10140
   tgctgaaatt gcagagtatt tgaatttaaa gggtactagc gataccgaaa aggtaacagc 10200
   cttaatagaa gctatttcaa agttaaagat agatttgagt attccacaaa atataagtgc 10260
   cgctggaata aataaaaaag atttttataa tacgctagat aaaatgtcag agcttgcttt 10320
   tgatgaccaa tgtacaacag ctaatcctag gtatccactt ataagtgaac ttaaggatat 10380
   ctatataaaa tcattttaac ccgggaaccc agctttcttg tacaaagtgg tgatgatccg 10440
   gctgctaacc tcgagaacat gtgagcaata aaaataagaa gcctgcattt gcaggcttct 10500
   tatttttata gaccggggac ttatcagcca acctgtaggc cagcaaaagg ccaggaaccg 10560
   taaaaaggcc gcgttgctgg cgtttttcca taggctccgc ccccctgacg agcatcacaa 10620
   aaatcgacgc tcaagtcaga ggtggcgaaa cccgacagga ctataaagat accaggcgtt 10680
   tccccctgga agctccctcg tgcgctctcc tgttccgacc ctgccgctta ccggatacct 10740
   gtccgccttt ctcccttcgg gaagcgtggc gctttctcat agctcacgct gtaggtatct 10800
   cagttcggtg taggtcgttc gctccaagct gggctgtgtg cacgaacccc ccgttcagcc 10860
   cgaccgctgc gccttatccg gtaactatcg tcttgagtcc aacccggtaa gacacgactt 10920
   atcgccactg gcagcagcca ctggtaacag gattagcaga gcgaggtatg taggcggtgc 10980
   tacagagttc ttgaagtggt ggcctaacta cggctacact agaagaacag tatttggtat 11040
   ctgcgctctg ctgaagccag ttaccttcgg aaaaagagtt ggtagctctt gatccggcaa 11100
   acaaaccacc gctggtagcg gtggtttttt tgtttgcaag cagcagatta cgcgcagaaa 11160
   aaaaggatct caagaagatc ctttgatctt ttctacgggg tctgacgctc agtggaacga 11220
   aaactcacgt taagggattt tggtcatgag attatcaaaa aggatcttca cctagatcct 11280
   tttaaattaa aaatgaagtt ttaaatcaat ctaaagtata tatgagtaaa cttggtctga 11340
   cagttaccaa tgcttaatca gtgaggcacc tatctcagcg atctgtctat ttcgttcatc 11400
   catagttgcc tgactccccg tcgtgtagat aactacgata cgggagggct taccatctgg 11460
   ccccagtgct gcaatgatac cgcgagaccc acgctcaccg gctccagatt tatcagcaat 11520
   aaaccagcca gccggaaggg ccgagcgcag aagtggtcct gcaactttat ccgcctccat 11580
   ccagtctatt aattgttgcc gggaagctag agtaagtagt tcgccagtta atagtttgcg 11640
   caacgttgtt gccattgcta caggcatcgt ggtgtcacgc tcgtcgtttg gtatggcttc 11700
   attcagctcc ggttcccaac gatcaaggcg agttacatga tcccccatgt tgtgcaaaaa 11760
   agcggttagc tccttcggtc ctccgatcgt tgtcagaagt aagttggccg cagtgttatc 11820
   actcatggtt atggcagcac tgcataattc tcttactgtc atgccatccg taagatgctt 11880
   ttctgtgact ggtgagtact caaccaagtc attctgagaa tagtgtatgc ggcgaccgag 11940
   ttgctcttgc ccggcgtcaa tacgggataa taccgcgcca catagcagaa ctttaaaagt 12000
   gctcatcatt ggaaaacgtt cttcggggcg aaaactctca aggatcttac cgctgttgag 12060
   atccagttcg atgtaaccca ctcgtgcacc caactgatct tcagcatctt ttactttcac 12120
   cagcgtttct gggtgagcaa aaacaggaag gcaaaatgcc gcaaaaaagg gaataagggc 12180
   gacacggaaa tgttgaatac tcatactctt cctttttcaa tattattgaa gcatttatca 12240
   gggttattgt ctcatgagcg gatacatatt tgaatgtatt tagaaaaata aacaaatagg 12300
   ggttccgcgc acatttcccc gaaaagtgcc acctgacggt cctgcaggta atacgactca 12360
   ctatagggat aacttaaatt tatatttttt actttataat atataattga ttatagaatc 12420
   caaattagat aggaggacca ggtaaatgga gaataatttt attgtcaacg aaaacgagaa 12480
   aagggtttta aagcagatat ttaacaactc caatatcagt agaactcaaa tttccaagaa 12540
   tttagaactg aataaagcaa caatttcgaa cattcttaac aatctaaagc ataaaagttt 12600
   ggtaaatgaa gttggtgaag gaaattctac taaaagtggt ggaagaaaac ctatacttct 12660
   cgaaattaat caaaagtatg ggtattatat aagcatggat ttgacatatg attctgttga 12720
   gttaatgtat aactattttg atgctacaat tctcaaacaa gatagttatg aactgaatga 12780
   taagaatgta agtagcatat tacagatact caaatctaac ataaatgtta gcgaaaaata 12840
   cgacacatta tacggtttat tagggataag tatatccata catgggattg tagatgatga 12900
   acaaaacatc attaatctac catttcacaa gaatgaaaaa agaacgttta ctgatgaact 12960
   taagtctttc actaatgtac cagtggttat tgaaaatgag gcaaatttaa gtgcattata 13020
   cgagaaaagt ttatacatca attcgaatat taacaacttg atcacattgt ctatacataa 13080
   aggtatagga gctggcatca taatcaataa gaaactctat agaggtagta atggagaagc 13140
   tggagaaata ggaaagactc ttgttttaga gtctatcaat aacaatgata acaagtacta 13200
   taaaatagag gatatttgta gtcaagacgc tttgatacaa aagattaata acagattagg 13260
   agttacactt acatttaccg aactaataca gtattataac gaaggaaata gtattgtagc 13320
   tcatgaaatc aaacagttta tcaacaaaat gactgtctta atacacaatc ttaatactca 13380
   gtttaatcca gatgcaatat acatcaattg tcccttgatt aatgaacttc ctaatattct 13440
   aaacgaaatc aaagaacaat tctcttgttt ttcacaagga agtcctatac aattgcattt 13500
   aaccactaat gttaaacaag ctacattatt aggtggaaca ttagcaatta tgcagaaaac 13560
   ccttaacata aacaatatcc aaatgaatat taaatagtta cagcagtctg agttataaaa 13620
   tagatatctc ggaccgtcta ttcttcctta tttgagtggg taccaatgaa atctttcgag 13680
   ctaaatcgat ttctggccca ctctttttta ttttttagta agcgtttaca aaaaatgaac 13740
   aatgtgctat attacctcta aagttagttt gtttattaaa ttaaccaact aaaatgtcgg 13800
   gatccaatta ggaggaatct gcgatggaaa agaaggaatt tagagtttta ataaagtatt 13860
   gttttcttaa aggaaagaat acagtagaag ctaagacttg gttagataat gaatttccag 13920
   attctgctcc tggtaaaagt acaattatag attggtatgc aaaatttaaa agaggagaaa 13980
   tgtcaacaga agatggagaa agatctggta gaccaaaaga agttgtaact gatgaaaata 14040
   taaagaagat acataaaatg atattaaatg atagaaagat gaagttaata gaaatagcag 14100
   aagctttaaa aatatctaaa gaaagagttg gacatataat acatcaatat ttagatatga 14160
   gaaaattatg tgcaaaatgg gttccaagag aattaacttt tgatcaaaag caaagaagag 14220
   tagatgatag taagagatgt ttacaattat taacaagaaa tactccagaa tttttcagaa 14280
   gatatgtaac aatggatgaa acttggttac atcattatac acctgaaagt aatagacaat 14340
   cagcagaatg gacagctact ggagaaccat cacctaaaag aggaaagact caaaagagtg 14400
   ctggaaaggt tatggcatca gtattttggg atgctcatgg tataatattt atagattatt 14460
   tagaaaaagg aaaaacaata aattctgatt attatatggc attattagaa agattaaaag 14520
   ttgaaatagc agctaaaaga ccacatatga agaagaaaaa agtattattt catcaagata 14580
   atgctccttg tcataagtca ttaagaacta tggcaaagat acatgaatta ggttttgaat 14640
   tattaccaca tccaccttat tcaccagatt tagctccttc tgatttcttt ttatttagtg 14700
   atttaaagag aatgttagca ggaaagaagt ttggttgtaa tgaagaagtt atagcagaaa 14760
   cagaagctta ttttgaagca aaacctaagg aatattatca aaatggaata aagaagttag 14820
   aaggtagata taatagatgt atagcattag aaggaaatta tgtagaataa gaattcctta  14880
   attaaggagc tcgataaaaa taagaagcct gcatttgcag gcttcttatt tttatggtca  14940
   tagctgtttc ctggcgcgcc gcattcactt cttttctata taaatatgag cgaagcgaat  15000
   aagcgtcgga aaagcagcaa aaagtttcct ttttgctgtt ggagcatggg ggttcagggg  15060
   gtgcagtatc tgacgtcaat gccgagcgaa agcgagccga agggtagcat ttacgttaga  15120
   taaccccctg atatgctccg acgctttata tagaaaagaa gattcaacta ggtaaaatct  15180
   taatataggt tgagatgata aggtttataa ggaatttgtt tgttctaatt tttcactcat  15240
   tttgttctaa tttcttttaa caaatgttct ttttttttta gaacagttat gatatagtta  15300
   gaatagttta aaataaggag tgagaaaaag atgaaagaaa gatatggaac agtctataaa  15360
   ggctctcaga ggctcataga cgaagaaagt ggagaagtca tagaggtaga caagttatac  15420
   cgtaaacaaa cgtctggtaa cttcgtaaag gcatatatag tgcaattaat aagtatgtta  15480
   gatatgattg gcggaaaaaa acttaaaatc gttaactata tcctagataa tgtccactta  15540
   agtaacaata caatgatagc tacaacaaga gaaatagcaa aagctacagg aacaagtcta  15600
   caaacagtaa taacaacact taaaatctta gaagaaggaa atattataaa aagaaaaact  15660
   ggagtattaa tgttaaaccc tgaactacta atgagaggcg acgaccaaaa acaaaaatac  15720
   ctcttactcg aatttgggaa ctttgagcaa gaggcaaatg aaatagattg acctcccaat  15780
   aacaccacgt agttattggg aggtcaatct atgaaatgcg attaagggcc ggccagtggg  15840
   caagttgaaa aattcacaaa aatgtggtat aatatctttg ttcattagag cgataaactt  15900
   gaatttgaga gggaacttag atggtatttg aaaaaattga taaaaatagt tggaacagaa  15960
   aagagtattt tgaccactac tttgcaagtg taccttgtac ctacagcatg accgttaaag  16020
   tggatatcac acaaataaag gaaaagggaa tgaaactata tcctgcaatg ctttattata  16080
   ttgcaatgat tgtaaaccgc cattcagagt ttaggacggc aatcaatcaa gatggtgaat  16140
   tggggatata tgatgagatg ataccaagct atacaatatt tcacaatgat actgaaacat  16200
   tttccagcct ttggactgag tgtaagtctg actttaaatc atttttagca gattatgaaa  16260
   gtgatacgca acggtatgga aacaatcata gaatggaagg aaagccaaat gctccggaaa  16320
   acatttttaa tgtatctatg ataccgtggt caaccttcga tggctttaat ctgaatttgc  16380
   agaaaggata tgattatttg attcctattt ttactatggg gaaatattat aaagaagata  16440
   acaaaattat acttcctttg gcaattcaag ttcatcacgc agtatgtgac ggatttcaca  16500
   tttgccgttt tgtaaacgaa ttgcaggaat tgataaatag ttaacttcag gtttgtctgt  16560
   aactaaaaac aagtatttaa gcaaaaacat cgtagaaata cggtgttttt tgttacccta  16620
   agttt                                                              16625
<210> 5
   <211> 6774
   <212> DNA
   <213> Artificial
<220>
   <223> Synthetic gene cluster for 1,4-BDO synthesis in Clostridium ljung dahlii
<400> 5
   agatctatga gtaatgaagt atctataaaa gaattaattg aaaaggcaaa ggtggcacaa      60
   aaaaaattgg aagcctatag tcaagaacaa gttgatgtac tagtaaaagc actaggaaaa     120
   gtggtttatg ataatgcaga aatgtttgca aaagaagcag ttgaagaaac agaaatgggt     180
   gtttatgaag ataaagtagc taaatgtcat ttgaaatcag gagctatttg gaatcatata     240
   aaagacaaga aaactgtagg cataataaaa gaagaacctg aaagggcact tgtttatgtt     300
   gctaagccaa agggagttgt ggcagctact acgcctataa ctaatccagt ggtaactcct     360
   atgtgtaatg caatggctgc tataaagggc agaaatacaa taatagtagc accacatcct     420
   aaagcaaaga aagtttcagc tcatactgta gaacttatga atgctgagct taaaaaattg     480
   ggagcaccag aaaatatcat acagatagta gaagcaccat caagagaagc tgctaaggaa     540
   cttatggaaa gtgctgatgt agttattgct acaggcggtg ctggaagagt taaagctgct     600
   tactccagtg gaagaccagc ttatggcgtt ggacctggaa attcacaggt aatagttgat     660
   aagggatacg attataacaa agctgcacag gatataataa caggaagaaa atatgacaat     720
   ggaattatat gttcttcaga gcaatcagtt atagctcctg ctgaagatta tgataaggta     780
   atagcagctt ttgtagaaaa tggggcattc tatgtagaag atgaggaaac agtagaaaag     840
   tttagatcaa ctttatttaa agatggaaaa ataaacagca agattatagg taaatccgtc     900
   caaattattg cggatcttgc aggagtaaaa gtaccagaag gtactaaggt tatagtactt     960
   aagggtaaag gtgcaggaga aaaagatgta ctttgtaaag aaaaaatgtg tccagtttta    1020
   gtagcattga aatatgatac ttttgaagaa gcagttgaaa tagctatggc taattatatg    1080
   tatgaaggag ctggtcatac agcaggcata cattctgaca atgacgagaa cataagatat    1140
   gcaggaactg tattacctat aagcagatta gttgtaaatc agcctgcaac tactgctgga    1200
   ggaagtttca ataatggatt taaccctact actacactag gctgcggatc atggggcaga    1260
   aacagtattt cagaaaatct tacttacgag catcttataa atgtttcaag aatagggtat    1320
   ttcaataaag aagcaaaagt tcctagctat gaggaaatat ggggataata attcttaaat    1380
   aaacaatact taaaacattt gaggaggtct tgtaaacatg caacttttca aactcaagag    1440
   tgtaacacat cactttgaca cttttgcaga atttgccaag gaattctgtc ttggagaacg    1500
   cgacttggta attaccaacg agttcatcta tgaaccgtat atgaaggcat gccagctccc    1560
   ctgccatttt gttatgcagg agaaatatgg gcaaggcgag ccttctgacg aaatgatgaa    1620
   taacatcttg gcagacatcc gtaatatcca gttcgaccgc gtaatcggta tcggaggagg 1680
   tacggttatt gacatctcta aacttttcgt tctgaaagga ttaaatgatg tactcgatgc 1740
   attcgaccgc aaaatacctc ttatcaaaga gaaagaactg atcattgtgc ccacaacatg 1800
   cggaacgggt agcgaggtga cgaacatttc tatcgcagaa atcaaaagcc gtcacaccaa 1860
   aatgggattg gctgacgatg ccattgttgc agaccatgcc atcatcatac ctgaacttct 1920
   gaagagcttg cctttccact tctacgcatg cagtgcaatc gatgctctta tccatgccat 1980
   cgagtcatac gtatctccta aagccagtcc atattctcgt ctgttcagtg aggcggcttg 2040
   ggacattatc ctggaagtat tcaagaaaat cgccgaacac ggccctgaat accgcttcga 2100
   aaagctggga gaaatgatca tggccagcaa ctatgccggt atagccttcg gaaatgcagg 2160
   agtaggagcc gtccacgcac tatcctaccc gttgggaggc aactatcacg tgccgcatgg 2220
   agaagcaaac tatcagttct tcacagaggt attcaaagta taccaaaaga agaatccttt 2280
   cggctatata gtcgaactca actggaagct ctccaagata ctgaactgcc agcccgaata 2340
   cgtatatccg aagctggatg aacttctcgg atgccttctt accaagaaac ctttgcacga 2400
   atacggcatg aaggacgaag aggtaagagg ctttgcggaa tcagtgctta agacacagca 2460
   aagattgctc gccaacaact acgtagagct tactgtagat gagatcgaag gtatctacag 2520
   aagactctac taataatagc ataacccctt ggggcctcta aacgggtctt gaggggtttt 2580
   ttggcgtgtg tgttagccaa agctcctgca ggtcgacttt ttaacaaaat atattgataa 2640
   aaataataat agtgggtata attaagttgt tagagaaaac gtataaatta gggataaact 2700
   atggaactta tgaaatagat tgaaatggtt tatctgttac cccgtaggat ccagaattta 2760
   aaaggaggga ttaaaatgaa agttacaaat caaaaagaac taaaacaaaa gctaaatgaa 2820
   ttgagagaag cgcaaaagaa gtttgcaacc tatactcaag agcaagttga taaaattttt 2880
   aaacaatgtg ccatagccgc agctaaagaa agaataaact tagctaaatt agcagtagaa 2940
   gaaacaggaa taggtcttgt agaagataaa attataaaaa atcattttgc agcagaatat 3000
   atatacaata aatataaaaa tgaaaaaact tgtggcataa tagaccatga cgattcttta 3060
   ggcataacaa aggttgctga accaattgga attgttgcag ccatagttcc tactactaat 3120
   ccaacttcca cagcaatttt caaatcatta atttctttaa aaacaagaaa cgcaatattc 3180
   ttttcaccac atccacgtgc aaaaaaatct acaattgctg cagcaaaatt aattttagat 3240
   gcagctgtta aagcaggagc acctaaaaat ataataggct ggatagatga gccatcaata 3300
   gaactttctc aagatttgat gagtgaagct gatataatat tagcaacagg aggtccttca 3360
   atggttaaag cggcctattc atctggaaaa cctgcaattg gtgttggagc aggaaataca 3420
   ccagcaataa tagatgagag tgcagatata gatatggcag taagctccat aattttatca 3480
   aagacttatg acaatggagt aatatgcgct tctgaacaat caatattagt tatgaattca 3540
   atatacgaaa aagttaaaga ggaatttgta aaacgaggat catatatact caatcaaaat 3600
   gaaatagcta aaataaaaga aactatgttt aaaaatggag ctattaatgc tgacatagtt 3660
   ggaaaatctg cttatataat tgctaaaatg gcaggaattg aagttcctca aactacaaag 3720
   atacttatag gcgaagtaca atctgttgaa aaaagcgagc tgttctcaca tgaaaaacta 3780
   tcaccagtac ttgcaatgta taaagttaag gattttgatg aagctctaaa aaaggcacaa 3840
   aggctaatag aattaggtgg aagtggacac acgtcatctt tatatataga ttcacaaaac 3900
   aataaggata aagttaaaga atttggatta gcaatgaaaa cttcaaggac atttattaac 3960
   atgccttctt cacagggagc aagcggagat ttatacaatt ttgcgatagc accatcattt 4020
   actcttggat gcggcacttg gggaggaaac tctgtatcgc aaaatgtaga gcctaaacat 4080
   ttattaaata ttaaaagtgt tgctgaaaga agggaaaata tgctttggtt taaagtgcca 4140
   caaaaaatat attttaaata tggatgtctt agatttgcat taaaagaatt aaaagatatg 4200
   aataagaaaa gagcctttat agtaacagat aaagatcttt ttaaacttgg atatgttaat 4260
   aaaataacaa aggtactaga tgagatagat attaaataca gtatatttac agatattaaa 4320
   tctgatccaa ctattgattc agtaaaaaaa ggtgctaaag aaatgcttaa ctttgaacct 4380
   gatactataa tctctattgg tggtggatcg ccaatggatg cagcaaaggt tatgcacttg 4440
   ttatatgaat atccagaagc agaaattgaa aatctagcta taaactttat ggatataaga 4500
   aagagaatat gcaatttccc taaattaggt acaaaggcga tttcagtagc tattcctaca 4560
   actgctggta ccggttcaga ggcaacacct tttgcagtta taactaatga tgaaacagga 4620
   atgaaatacc ctttaacttc ttatgaattg accccaaaca tggcaataat agatactgaa 4680
   ttaatgttaa atatgcctag aaaattaaca gcagcaactg gaatagatgc attagttcat 4740
   gctatagaag catatgtttc ggttatggct acggattata ctgatgaatt agccttaaga 4800
   gcaataaaaa tgatatttaa atatttgcct agagcctata aaaatgggac taacgacatt 4860
   gaagcaagag aaaaaatggc acatgcctct aatattgcgg ggatggcatt tgcaaatgct 4920
   ttcttaggtg tatgccattc aatggctcat aaacttgggg caatgcatca cgttccacat 4980
   ggaattgctt gtgctgtatt aatagaagaa gttattaaat ataacgctac agactgtcca 5040
   acaaagcaaa cagcattccc tcaatataaa tctcctaatg ctaagagaaa atatgctgaa 5100
   attgcagagt atttgaattt aaagggtact agcgataccg aaaaggtaac agccttaata 5160
   gaagctattt caaagttaaa gatagatttg agtattccac aaaatataag tgccgctgga 5220
   ataaataaaa aagattttta taatacgcta gataaaatgt cagagcttgc ttttgatgac 5280
   caatgtacaa cagctaatcc taggtatcca cttataagtg aacttaagga tatctatata 5340
   aaatcatttt aataattctt aaataaacaa tacttaaaac atttgaggag gtcttgtaaa 5400
   catggattgg aagaagatct atgaagacag aacatgcact gcagatgaag cagtaaagag 5460
   cattaagtca ggtgacagag tgctatttgc gcactgtgtt gctgaaccgc cagttcttgt 5520
   agaagcaatg gttgcgaatg cagctgcata caagaatgta acggtttcac acatggttac 5580
   ccttggaaag ggtgaatact caaaaccaga atataaggaa aactttactt ttgaaggttg 5640
   gtttacaagc ccttcaacaa gaggatccat tgcagaagga cacggacagt ttgtccctgt 5700
   attcttccac gaggtaccat ctttaatcag aaaagacatt ttccatgttg atgtattcat 5760
   ggtaatggta tcccctccag atcataacgg attctgctgt gtgggtgtat cttctgacta 5820
   tacgatgcag gctatcaaat cagcaaaaat tgtacttgct gaagtaaatg atcaggtacc 5880
   tgtagtttat ggagatacat ttgttcacgt tagtgaaatc gacaagttcg tagaaacttc 5940
   acatccactt ccagaaatcg gacttcctaa gatcggtgaa gtagaagctg ctattggtaa 6000
   gcactgcgct tccctaatcg aagatggttc cacattacag cttggtatcg gagctattcc 6060
   ggatgctgta ctttcacagc ttaaggacaa gaaacacctt ggtatccact ctgaaatgat 6120
   ttccgacggt gttgtagatc tttacgaagc aggcgttata gactgcagcc aaaagtctat 6180
   cgacaaaggc aaaatggcaa taacattctt aatgggaacg aagagacttt atgatttcgc 6240
   tgcaaacaat ccaaaggttg aattaaagcc ggttgactac ataaatcatc catctgtagt 6300
   tgcacagtgc tccaaaatgg tttgcatcaa tgcttgcttg caagttgatt ttatgggtca 6360
   gattgtatcc gatagtattg ggacaaagca gttctcagga gtaggcggtc aggttgactt 6420
   cgtaagaggt gcatccatgt ctattgacgg aaaaggtaaa gcgatcatcg cgatgccttc 6480
   cgttgcaaag aagaaggatg gaagtatgat ttcgaagatc gttccattca tcgatcacgg 6540
   tgcagctgta actacatcca gaaacgatgc ggactatgtc gtaacggaat atggtattgc 6600
   tgaaatgaag ggtaagtctt tacaggacag agcaagagcg ttaatcaata ttgcacaccc 6660
   tgatttcaaa gatgaattaa aggctgaatt tgaaaagaga ttcaacgcgg cattctaata 6720
   atagcataac cccttggggc ctctaaacgg gtcttgaggg gttttttgct cgag 6774
<210> 6
   <211> 120
   <212> DNA
   <213> Artificial
<220>
   <223> Linker
<400> 6
   ggatccgcgt cccccggggg actagctagc taggaattcg agctcggcgc gcctgcaggt 60
   cgacaagctt gcggccgcat aatgcttaag ccgctcgagc ggccttaatt aaggggcgcc 120